# EUROPEAN PATENT APPLICATION

(11) **EP 4 328 239 A1**
(43) Date of publication of application: **28.02.2024**
(21) Application number: 22192464.0
(22) Date of filing: 26.08.2022
(51) Int. Cl.: C07K 14/47, C07K 14/74, A61K 39/00, A61P 37/02, C07K 14/725

(54) **IMMUNOTHERAPEUTICS BASED ON MAGEA1-DERIVED EPITOPES**

(71) Applicant: Max-Delbrück-Centrum für Molekulare Medizin in der Helmholtz-Gemeinschaft, 13125 Berlin (DE)
(72) Inventor: LORENZ, Felix, 13129 Berlin (DE); Clauß, Julian, 13086 Berlin (DE); EDES, Inan, 13127 Berlin (DE); STAHN, Rainer, 13125 Berlin (DE); SADA JAPP, Alberto, 10439 Berlin (DE); HILGENBERG, Ellen, 10439 Berlin (DE); UCKERT, Wolfgang, 13125 Berlin (DE)
(74) Representative: Moré, Solveig Helga

(57) **Abstract**

The present invention relates to the field of immunotherapy, in particular, adoptive T cell therapy of MAGEA1-associated cancer. TCR alpha chain constructs (TRA) and/or a TCR beta chain constructs (TRB) of TCR constructs specific for an epitope in complex with a human MHC-I, wherein the epitope is an epitope of a MAGEA1 protein, nucleic acids encoding them and host cells expressing them. The invention also provides pharmaceutical compositions or kits based theron. The invention teaches combinations of TCR constructs directed to different epitopes from the same antigen expressed by the same cancer or infectious agent capabel of being presented by different MHC-I molecules, wherein optionally, the antigen is MAGEA1. Preferably, at least one of the TCR constructs used in the combination is a TCR construct of the invention disclosed herein. The pharmaceutical combinations or kits may be used for immunotherapy of a subject, e.g., adoptive T cell therapy or TCR gene therapy, e.g., for use in treatment of a cancer such as lung cancer, colorectal cancer, or multiple myeloma. The invention also provides peptides comprising the epitopes rcognized by the TCR constructs of the invention, which may be for use in vaccination, e.g., peptide vaccination or RNA vaccination.

## Description

The present invention relates to the field of immunotherapy, in particular, adoptive T cell therapy of MAGEA1-associated cancer. TCR alpha chain constructs (TRA) and/or a TCR beta chain constructs (TRB) of TCR constructs specific for an epitope in complex with a human MHC-I, wherein the epitope is an epitope of a MAGEA1 protein, nucleic acids encoding them and host cells expressing them. The invention also provides pharmaceutical compositions or kits based theron. The invention teaches combinations of TCR constructs directed to different epitopes from the same antigen expressed by the same cancer or infectious agent capable of being presented by different MHC-I molecules, wherein optionally, the antigen is MAGEA1. Preferably, at least one of the TCR constructs used in the combination is a TCR construct of the invention disclosed herein. The pharmaceutical combinations or kits may be used for immunotherapy of a subject, e.g., adoptive T cell therapy or TCR gene therapy, e.g., for use in treatment of a cancer such as lung cancer, colorectal cancer, or multiple myeloma. The invention also provides peptides comprising the epitopes recognized by the TCR constructs of the invention, which may be for use in vaccination, e.g., peptide vaccination or RNA vaccination.

Expression of the cancer testis antigen MAGEA1 has been observed in different types of cancer, e.g., lung cancer, colorectal cancer and multiple myeloma, but not in healthy adult tissue, only in immune compromised testis and during fetal development. MAGE-A1 is expressed intracellularly. Peptides (epitopes) thereof presented in the context of a MHC-I (HLA) molecule at a cancer cell's surface are thus potential targets for T cell receptor (TCR)-based immunotherapies.

Current treatments for MAGEA1-positive cancers are not antigen-specific, e.g., chemotherapy, radiotherapy. PD-1/PD-L1 antibodies are a type of immunotherapy blocking checkpoints of T cell inhibition and thus, unleashing tumor-specific T cell responses. Such antibodies have been approved recently but are not tumor antigen-specific and severe side effects have been reported. Antigen-specific treatments potentially have the highest treatment efficacy. MAGEA1-specific TCR-based treatments based on TCR restricted to HLA-A*02:01 are being explored at early clinical level (IMA202-101, clinical phase I, Immatics US, Inc (Tsimberidou et al., 2021); TK8001, clinical phase I, T-knife GmbH (Thistlethwaite *et al.,* 2021)), and harbor the potential of being a highly effective cancer treatment.

A TCR is a heterodimeric cell surface protein of the immunoglobulin super-family which is associated with invariant proteins of the CD3 complex involved in mediating signal transduction. TCRs exist in αβ and γδ forms, which are structurally similar, but have distinct anatomical locations and probably functions. The alpha and beta chains of native heterodimeric aβTCRs are transmembrane proteins, each comprising two extracellular domains, a membrane-proximal constant domain, and a membrane-distal variable domain. Each of the constant and variable domains includes an intra-chain disulfide bond.

The variable region of each TCR chain comprises variable and joining segments, and in the case of the beta chain also a diversity segment. Each variable region comprises three CDRs (Complementarity Determining Regions), highly polymorphic loops, embedded in a framework sequence, one being the hypervariable region named CDR3. There are several types of alpha chain variable (Vα) regions and several types of beta chain variable (Vβ) regions distinguished by their framework, CDR1 and CDR2 sequences, and by a partly defined CDR3 sequence. Unique TRAV or TRBV numbers are given to Vα or Vβs by IMGT nomenclature. TCR specificity for the epitopes recognized is mainly determined by the CDR3 regions (Danska et al., 1990; Garcia and Adams, 2005).

The use of adoptive TCR gene therapy allows activating the patients' own T cells *ex vivo,* equipping the T cells with desired specificities and generation of sufficient numbers of T cells in a short period of time. The TCR may be transduced into all T cells or T-cell subsets such as CD8+ T cells, central memory T cells or T cells with stem cell characteristics, which may ensure better persistence and function upon transfer. TCR-engineered T cells may be infused into patients, e.g., cancer patients that have, e.g., been rendered lymphopenic by chemotherapy or irradiation, inducing homeostatic expansion which greatly enhances engraftment and long term persistence of transferred T cells with higher cure rates.

TCR-based adoptive T cell therapy relies on classical TCR recognition of processed epitopes of antigens presented in the context of MHC molecules. Thus, a T cell expressing a specific TCR specific for an epitope in the context of a specific MHC can only be used for treatment of a patient expressing the respective MHC. Thousands of different MHC alleles exist in the population and each individual carries 6 MHC class I alleles. Some alleles are found to be more frequent in certain populations than others. MAGEA1-specific TCR-based treatments are being explored in early clinical trials and harbor the potential of being a highly effective cancer treatment. However, these efforts are currently limited to HLA-A*02:01 - e.g., IMA202 (clinical phase I, Immatics, Tsimberidou et al., 2021) and TK8001 (clinical phase I, T-knife, Thistlethwaite et al., 2021). Thus, MAGEA1-specific TCRs for other HLA patient groups are also needed to cover a broader population of patients suffering from MAGE-A1+ tumors.

It is critical for successful MAGEA1-specific TCR gene therapy to target relevant immunogenic epitopes to unfold maximal potential of such treatment. In light of the state of the art, the inventors addressed the problem of providing immunogenic epitopes and corresponding TCRs efficiently targeting those epitopes, as well as advantageous combination therapies, in particular for MAGEA1-expressing cancers.

### Summary of the invention

This problem is solved by the claimed subject matter.

In a first embodiment, the invention provides a nucleic acid encoding a TCR alpha chain construct (TRA) and/or a TCR beta chain construct (TRB) of a TCR construct specific for an epitope in complex with a human MHC-I, wherein the epitope is an epitope of a MAGEA1 protein,
a) wherein the epitope has the sequence of SEQ ID NO: 1, the MHC-I is HLA-B*44:03, and the TRA comprises a CDR3 having at least 90% sequence identity to SEQ ID NO: 13 and/or the TRB comprises a CDR3 having at least 90% sequence identity to SEQ ID NO: 18;
b) wherein the epitope has the sequence of SEQ ID NO: 2, the MHC-I is HLA-B*44:03, and the TRA comprises a CDR3 having at least 90% sequence identity to SEQ ID NO: 23 and/or the TRB comprises a CDR3 having at least 90% sequence identity to SEQ ID NO: 28;
c) wherein the epitope has the sequence of SEQ ID NO: 3, the MHC-I is HLA-B*44:03, and the TRA comprises a CDR3 having at least 90% sequence identity to SEQ ID NO: 33 and/or the TRB comprises a CDR3 having at least 90% sequence identity to SEQ ID NO: 38;
d) wherein the epitope has the sequence of SEQ ID NO: 1, the MHC-I is HLA-B*44:03, and the TRA comprises a CDR3 having at least 90% sequence identity to SEQ ID NO: 43 and/or the TRB comprises a CDR3 having at least 90% sequence identity to SEQ ID NO: 48;
e) wherein the epitope has the sequence of SEQ ID NO: 4, the MHC-I is HLA-B*07:02, and the TRA comprises a CDR3 having at least 90% sequence identity to SEQ ID NO: 53 and/or the TRB comprises a CDR3 having at least 90% sequence identity to SEQ ID NO: 58;
f) wherein the epitope has the sequence of SEQ ID NO: 4, the MHC-I is HLA-B*07:02, and the TRA comprises a CDR3 having at least 90% sequence identity to SEQ ID NO: 63 and/or the TRB comprises a CDR3 having at least 90% sequence identity to SEQ ID NO: 68;
g) wherein the epitope has the sequence of SEQ ID NO: 4, the MHC-I is HLA-B*07:02, and the TRA comprises a CDR3 having at least 90% sequence identity to SEQ ID NO: 73 and/or the TRB comprises a CDR3 having at least 90% sequence identity to SEQ ID NO: 78;
h) wherein the epitope has the sequence of SEQ ID NO: 4, the MHC-I is HLA-B*07:02, and the TRA comprises a CDR3 having at least 90% sequence identity to SEQ ID NO: 83 and/or the TRB comprises a CDR3 having at least 90% sequence identity to SEQ ID NO: 88;
i) wherein the epitope has the sequence of SEQ ID NO: 5, the MHC-I is HLA-B*57:01 or HLA-B*57:03, and the TRA comprises a CDR3 having at least 90% sequence identity to SEQ ID NO: 93 and/or the TRB comprises a CDR3 having at least 90% sequence identity to SEQ ID NO: 98;
j) wherein the epitope has the sequence of SEQ ID NO: 6, the MHC-I is HLA-A*02:01, and the TRA comprises a CDR3 having at least 90% sequence identity to SEQ ID NO: 103 and/or the TRB comprises a CDR3 having at least 90% sequence identity to SEQ ID NO: 108;
k) wherein the epitope has the sequence of SEQ ID NO: 7, the MHC-I is HLA-A*03:01, and the TRA comprises a CDR3 having at least 90% sequence identity to SEQ ID NO: 113 and/or the TRB comprises a CDR3 having at least 90% sequence identity to SEQ ID NO: 118;
l) wherein the epitope has the sequence of SEQ ID NO: 7, the MHC-I is HLA-A*03:01, and the TRA comprises a CDR3 having at least 90% sequence identity to SEQ ID NO: 123 and/or the TRB comprises a CDR3 having at least 90% sequence identity to SEQ ID NO: 128;
m) wherein the epitope has the sequence of SEQ ID NO: 8, the MHC-I is HLA-A*26:01, and the TRA comprises a CDR3 having at least 90% sequence identity to SEQ ID NO: 133 and/or the TRB comprises a CDR3 having at least 90% sequence identity to SEQ ID NO: 138; and/or
n) wherein the epitope has the sequence of SEQ ID NO: 9, the MHC-I is HLA-A*29:02, and the TRA comprises a CDR3 having at least 90% sequence identity to SEQ ID NO: 143 and/or the TRB comprises a CDR3 having at least 90% sequence identity to SEQ ID NO: 148; and/or
o) wherein the MHC-I is HLA-A*29:02, and the TRA comprises a CDR3 having at least 90% sequence identity to SEQ ID NO: 173 and/or the TRB comprises a CDR3 having at least 90% sequence identity to SEQ ID NO: 178; and/or
p) wherein the MHC-I is HLA-B*44:03, and the TRA comprises a CDR3 having at least 90% sequence identity to SEQ ID NO: 183 and/or the TRB comprises a CDR3 having at least 90% sequence identity to SEQ ID NO: 188; and/or
q) wherein the epitope has the sequence of SEQ ID NO: 201, the MHC-I is HLA-C*06:02, and the TRA comprises a CDR3 having at least 90% sequence identity to SEQ ID NO: 193 and/or the TRB comprises a CDR3 having at least 90% sequence identity to SEQ ID NO: 198.

In a second embodiment, the invention provides a nucleic acid of embodiment 1, wherein the MHC-I is HLA-B*44:03.

In a third embodiment, the invention provides a nucleic acid of embodiment 2, wherein the epitope has the sequence of SEQ ID NO: 1, and the TRA comprises a CDR3 having at least 90% sequence identity to SEQ ID NO: 13 and/or the TRB comprises a CDR3 having at least 90% sequence identity to SEQ ID NO: 18;
wherein, optionally, the TRA comprises a CDR1 having at least 80% sequence identity to SEQ ID NO: 11, a CDR2 having at least 80% sequence identity to SEQ ID NO: 12 and a CDR3 having at least 90%, preferably, 100% sequence identity to SEQ ID NO: 13, and/or the TRB comprises a CDR1 having at least 80% sequence identity to SEQ ID NO: 16, a CDR2 having at least 80% sequence identity to SEQ ID NO: 17 and a CDR3 having at least 90%, preferably, 100% sequence identity to SEQ ID NO: 18;
wherein, most preferably, the TRA comprises a variable region having at least 90% sequence identity to SEQ ID NO: 14 and/or the TRB comprises a variable region having at least 90% sequence identity to SEQ ID NO: 19.

In a fourth embodiment, the invention provides a nucleic acid of embodiment 2, wherein the epitope has the sequence of SEQ ID NO: 2, and the TRA comprises a CDR3 having at least 90% sequence identity to SEQ ID NO: 23 and/or the TRB comprises a CDR3 having at least 90% sequence identity to SEQ ID NO: 28;
wherein, optionally, the TRA comprises a CDR1 having at least 80% sequence identity to SEQ ID NO: 21, a CDR2 having at least 80% sequence identity to SEQ ID NO: 22 and a CDR3 having at least 90%, preferably, 100% sequence identity to SEQ ID NO: 23, and/or the TRB comprises a CDR1 having at least 80% sequence identity to SEQ ID NO: 26, a CDR2 having at least 80% sequence identity to SEQ ID NO: 27 and a CDR3 having at least 90%, preferably, 100% sequence identity to SEQ ID NO: 28;
wherein, most preferably, the TRA comprises a variable region having at least 90% sequence identity to SEQ ID NO: 24 and/or the TRB comprises a variable region having at least 90% sequence identity to SEQ ID NO: 29.

In a fifth embodiment, the invention provides a nucleic acid of embodiment 2, wherein the epitope has the sequence of SEQ ID NO: 3, and the TRA comprises a CDR3 having at least 90% sequence identity to SEQ ID NO: 33 and/or the TRB comprises a CDR3 having at least 90% sequence identity to SEQ ID NO: 38;
wherein, optionally, the TRA comprises a CDR1 having at least 80% sequence identity to SEQ ID NO: 31, a CDR2 having at least 80% sequence identity to SEQ ID NO: 32 and a CDR3 having at least 90%, preferably, 100% sequence identity to SEQ ID NO: 33, and/or the TRB comprises a CDR1 having at least 80% sequence identity to SEQ ID NO: 36, a CDR2 having at least 80% sequence identity to SEQ ID NO: 37 and a CDR3 having at least 90%, preferably, 100% sequence identity to SEQ ID NO: 38;
wherein, most preferably, the TRA comprises a variable region having at least 90% sequence identity to SEQ ID NO: 34 and/or the TRB comprises a variable region having at least 90% sequence identity to SEQ ID NO: 39.

In a sixth embodiment, the invention provides a nucleic acid of embodiment 2, wherein the epitope has the sequence of SEQ ID NO: 1, the TRA comprises a CDR3 having at least 90% sequence identity to SEQ ID NO: 43 and/or the TRB comprises a CDR3 having at least 90% sequence identity to SEQ ID NO: 48;
wherein, optionally, the TRA comprises a CDR1 having at least 80% sequence identity to SEQ ID NO: 41, a CDR2 having at least 80% sequence identity to SEQ ID NO: 42 and a CDR3 having at least 90%, preferably, 100% sequence identity to SEQ ID NO: 43, and/or the TRB comprises a CDR1 having at least 80% sequence identity to SEQ ID NO: 46, a CDR2 having at least 80% sequence identity to SEQ ID NO: 47 and a CDR3 having at least 90%, preferably, 100% sequence identity to SEQ ID NO: 48;
wherein, most preferably, the TRA comprises a variable region having at least 90% sequence identity to SEQ ID NO: 44 and/or the TRB comprises a variable region having at least 90% sequence identity to SEQ ID NO: 49.

In a seventh embodiment, the invention provides a nucleic acid of embodiment 1, wherein the MHC-I is HLA-B*07:02.

In an eigth embodiment, the invention provides a nucleic acid of embodiment 7, wherein the epitope has the sequence of SEQ ID NO: 4, the MHC-I is HLA-B*07:02, and the TRA comprises a CDR3 having at least 90% sequence identity to SEQ ID NO: 53 and/or the TRB comprises a CDR3 having at least 90% sequence identity to SEQ ID NO: 58;
wherein, optionally, the TRA comprises a CDR1 having at least 80% sequence identity to SEQ ID NO: 51, a CDR2 having at least 80% sequence identity to SEQ ID NO: 52 and a CDR3 having at least 90%, preferably, 100% sequence identity to SEQ ID NO: 53, and/or the TRB comprises a CDR1 having at least 80% sequence identity to SEQ ID NO: 56, a CDR2 having at least 80% sequence identity to SEQ ID NO: 57 and a CDR3 having at least 90%, preferably, 100% sequence identity to SEQ ID NO: 58;
wherein, most preferably, the TRA comprises a variable region having at least 90% sequence identity to SEQ ID NO: 54 and/or the TRB comprises a variable region having at least 90% sequence identity to SEQ ID NO: 59.

In a ninth embodiment, the invention provides a nucleic acid of embodiment 7, wherein the epitope has the sequence of SEQ ID NO: 4, the MHC-I is HLA-B*07:02, and the TRA comprises a CDR3 having at least 90% sequence identity to SEQ ID NO: 63 and/or the TRB comprises a CDR3 having at least 90% sequence identity to SEQ ID NO: 68;
wherein, optionlly, the TRA comprises a CDR1 having at least 80% sequence identity to SEQ ID NO: 61, a CDR2 having at least 80% sequence identity to SEQ ID NO: 62 and a CDR3 having at least 90%, preferably, 100% sequence identity to SEQ ID NO: 63, and/or the TRB comprises a CDR1 having at least 80% sequence identity to SEQ ID NO: 66, a CDR2 having at least 80% sequence identity to SEQ ID NO: 67 and a CDR3 having at least 90%, preferably, 100% sequence identity to SEQ ID NO: 68;
wherein, most preferably, the TRA comprises a variable region having at least 90% sequence identity to SEQ ID NO: 64 and/or the TRB comprises a variable region having at least 90% sequence identity to SEQ ID NO: 69.

In a tenth embodiment, the invention provides a nucleic acid of embodiment 7, wherein the epitope has the sequence of SEQ ID NO: 4, the MHC-I is HLA-B*07:02, and the TRA comprises a CDR3 having at least 90% sequence identity to SEQ ID NO: 73 and/or the TRB comprises a CDR3 having at least 90% sequence identity to SEQ ID NO: 78;
wherein, optionally, the TRA comprises a CDR1 having at least 80% sequence identity to SEQ ID NO: 71, a CDR2 having at least 80% sequence identity to SEQ ID NO: 72 and a CDR3 having at least 90%, preferably, 100% sequence identity to SEQ ID NO: 73, and/or the TRB comprises a CDR1 having at least 80% sequence identity to SEQ ID NO: 76, a CDR2 having at least 80% sequence identity to SEQ ID NO: 77 and a CDR3 having at least 90%, preferably, 100% sequence identity to SEQ ID NO: 78;
wherein, most preferably, the TRA comprises a variable region having at least 90% sequence identity to SEQ ID NO: 74 and/or the TRB comprises a variable region having at least 90% sequence identity to SEQ ID NO: 79.

In an 11^{th} embodiment, the invention provides a nucleic acid of embodiment 7, wherein the epitope has the sequence of SEQ ID NO: 4, the MHC-I is HLA-B*07:02, and the TRA comprises a CDR3 having at least 90% sequence identity to SEQ ID NO: 83 and/or the TRB comprises a CDR3 having at least 90% sequence identity to SEQ ID NO: 88;
wherein, optionally, the TRA comprises a CDR1 having at least 80% sequence identity to SEQ ID NO: 81, a CDR2 having at least 80% sequence identity to SEQ ID NO: 82 and a CDR3 having at least 90%, preferably, 100% sequence identity to SEQ ID NO: 83, and/or the TRB comprises a CDR1 having at least 80% sequence identity to SEQ ID NO: 86, a CDR2 having at least 80% sequence identity to SEQ ID NO: 87 and a CDR3 having at least 90%, preferably, 100% sequence identity to SEQ ID NO: 88;
wherein, most preferably, the TRA comprises a variable region having at least 90% sequence identity to SEQ ID NO: 84 and/or the TRB comprises a variable region having at least 90% sequence identity to SEQ ID NO: 89.

In a 12^{th} embodiment, the invention provides a nucleic acid of embodiment 1, wherein the epitope has the sequence of SEQ ID NO: 5, the MHC-I is HLA-B*57:03, and the TRA comprises a CDR3 having at least 90% sequence identity to SEQ ID NO: 93 and/or the TRB comprises a CDR3 having at least 90% sequence identity to SEQ ID NO: 98;
wherein, optionally, the TRA comprises a CDR1 having at least 80% sequence identity to SEQ ID NO: 91, a CDR2 having at least 80% sequence identity to SEQ ID NO: 92 and a CDR3 having at least 90%, preferably, 100% sequence identity to SEQ ID NO: 93, and/or the TRB comprises a CDR1 having at least 80% sequence identity to SEQ ID NO: 96, a CDR2 having at least 80% sequence identity to SEQ ID NO: 97 and a CDR3 having at least 90%, preferably, 100% sequence identity to SEQ ID NO: 98;
wherein, most preferably, the TRA comprises a variable region having at least 90% sequence identity to SEQ ID NO: 94 and/or the TRB comprises a variable region having at least 90% sequence identity to SEQ ID NO: 99. The MHC-I can also be HLA-B*57:03, as the TCR binds to the same peptide on both HLAs.

In a 13^{th} embodiment, the invention provides a nucleic acid of embodiment 1, wherein the MHC-I is HLA-A*02:01.

In a 14^{th} embodiment, the invention provides a nucleic acid of embodiment 13, wherein the epitope has the sequence of SEQ ID NO: 6, the MHC-I is HLA-A*02:01, and the TRA comprises a CDR3 having at least 90% sequence identity to SEQ ID NO: 103 and/or the TRB comprises a CDR3 having at least 90% sequence identity to SEQ ID NO: 108;
wherein, optionally, the TRA comprises a CDR1 having at least 80% sequence identity to SEQ ID NO: 101, a CDR2 having at least 80% sequence identity to SEQ ID NO: 102 and a CDR3 having at least 90%, preferably, 100% sequence identity to SEQ ID NO: 103, and/or the TRB comprises a CDR1 having at least 80% sequence identity to SEQ ID NO: 106, a CDR2 having at least 80% sequence identity to SEQ ID NO: 107 and a CDR3 having at least 90%, preferably, 100% sequence identity to SEQ ID NO: 108;
wherein, most preferably, the TRA comprises a variable region having at least 90% sequence identity to SEQ ID NO: 104 and/or the TRB comprises a variable region having at least 90% sequence identity to SEQ ID NO: 109.

In a 15^{th} embodiment, the invention provides a nucleic acid of embodiment 1, wherein the epitope has the sequence of SEQ ID NO: 7, the MHC-I is HLA-A*03:01, and the TRA comprises a CDR3 having at least 90% sequence identity to SEQ ID NO: 113 and/or the TRB comprises a CDR3 having at least 90% sequence identity to SEQ ID NO: 118;
wherein, optionally, the TRA comprises a CDR1 having at least 80% sequence identity to SEQ ID NO: 111, a CDR2 having at least 80% sequence identity to SEQ ID NO: 112 and a CDR3 having at least 90%, preferably, 100% sequence identity to SEQ ID NO: 113, and/or the TRB comprises a CDR1 having at least 80% sequence identity to SEQ ID NO: 116, a CDR2 having at least 80% sequence identity to SEQ ID NO: 117 and a CDR3 having at least 90%, preferably, 100% sequence identity to SEQ ID NO: 118;
wherein, most preferably, the TRA comprises a variable region having at least 90% sequence identity to SEQ ID NO: 114 and/or the TRB comprises a variable region having at least 90% sequence identity to SEQ ID NO: 119.

In a 16^{th} embodiment, the invention provides a nucleic acid of embodiment 13, wherein the epitope has the sequence of SEQ ID NO: 7, the MHC-I is HLA-A*03:01, and the TRA comprises a CDR3 having at least 90% sequence identity to SEQ ID NO: 123 and/or the TRB comprises a CDR3 having at least 90% sequence identity to SEQ ID NO: 128;
wherein, optionally, the TRA comprises a CDR1 having at least 80% sequence identity to SEQ ID NO: 121, a CDR2 having at least 80% sequence identity to SEQ ID NO: 122 and a CDR3 having at least 90%, preferably, 100% sequence identity to SEQ ID NO: 123, and/or the TRB comprises a CDR1 having at least 80% sequence identity to SEQ ID NO: 126, a CDR2 having at least 80% sequence identity to SEQ ID NO: 127 and a CDR3 having at least 90%, preferably, 100% sequence identity to SEQ ID NO: 128;
wherein, most preferably, the TRA comprises a variable region having at least 90% sequence identity to SEQ ID NO: 124 and/or the TRB comprises a variable region having at least 90% sequence identity to SEQ ID NO: 129.

In a 17^{th} embodiment, the invention provides a nucleic acid of embodiment 1, wherein the epitope has the sequence of SEQ ID NO: 8, the MHC-I is HLA-A*26:01, and the TRA comprises a CDR3 having at least 90% sequence identity to SEQ ID NO: 133 and/or the TRB comprises a CDR3 having at least 90% sequence identity to SEQ ID NO: 138;
wherein, optionally, the TRA comprises a CDR1 having at least 80% sequence identity to SEQ ID NO: 131, a CDR2 having at least 80% sequence identity to SEQ ID NO: 132 and a CDR3 having at least 90%, preferably, 100% sequence identity to SEQ ID NO: 133, and/or the TRB comprises a CDR1 having at least 80% sequence identity to SEQ ID NO: 136, a CDR2 having at least 80% sequence identity to SEQ ID NO: 137 and a CDR3 having at least 90%, preferably, 100% sequence identity to SEQ ID NO: 138;
wherein, most preferably, the TRA comprises a variable region having at least 90% sequence identity to SEQ ID NO: 134 and/or the TRB comprises a variable region having at least 90% sequence identity to SEQ ID NO: 139.

In an 18^{th} embodiment, the invention provides a nucleic acid of embodiment 1, wherein the epitope has the sequence of SEQ ID NO: 9, the MHC-I is HLA-A*29:02, and the TRA comprises a CDR3 having at least 90% sequence identity to SEQ ID NO: 143 and/or the TRB comprises a CDR3 having at least 90% sequence identity to SEQ ID NO: 148;
wherein, optionally, the TRA comprises a CDR1 having at least 80% sequence identity to SEQ ID NO: 141, a CDR2 having at least 80% sequence identity to SEQ ID NO: 142 and a CDR3 having at least 90%, preferably, 100% sequence identity to SEQ ID NO: 143, and/or the TRB comprises a CDR1 having at least 80% sequence identity to SEQ ID NO: 146, a CDR2 having at least 80% sequence identity to SEQ ID NO: 147 and a CDR3 having at least 90%, preferably, 100% sequence identity to SEQ ID NO: 148;
wherein, most preferably, the TRA comprises a variable region having at least 90% sequence identity to SEQ ID NO: 144 and/or the TRB comprises a variable region having at least 90% sequence identity to SEQ ID NO: 149.

In a 19^{th} embodiment, the invention provides a nucleic acid of embodiment 1, wherein the MHC-I is HLA-A*29:02, and the TRA comprises a CDR3 having at least 90% sequence identity to SEQ ID NO: 173 and/or the TRB comprises a CDR3 having at least 90% sequence identity to SEQ ID NO: 178;
wherein, optionally, the TRA comprises a CDR1 having at least 80% sequence identity to SEQ ID NO: 171, a CDR2 having at least 80% sequence identity to SEQ ID NO: 172 and a CDR3 having at least 90%, preferably, 100% sequence identity to SEQ ID NO: 173, and/or the TRB comprises a CDR1 having at least 80% sequence identity to SEQ ID NO: 176, a CDR2 having at least 80% sequence identity to SEQ ID NO: 177 and a CDR3 having at least 90%, preferably, 100% sequence identity to SEQ ID NO: 178;
wherein, most preferably, the TRA comprises a variable region having at least 90% sequence identity to SEQ ID NO: 174 and/or the TRB comprises a variable region having at least 90% sequence identity to SEQ ID NO: 179.

In a 20^{th} embodiment, the invention provides a nucleic acid of embodiment 2, wherein the MHC-I is HLA-B*44:03, and the TRA comprises a CDR3 having at least 90% sequence identity to SEQ ID NO: 183 and/or the TRB comprises a CDR3 having at least 90% sequence identity to SEQ ID NO: 188;
wherein, optionally, the TRA comprises a CDR1 having at least 80% sequence identity to SEQ ID NO: 181, a CDR2 having at least 80% sequence identity to SEQ ID NO: 182 and a CDR3 having at least 90%, preferably, 100% sequence identity to SEQ ID NO: 183, and/or the TRB comprises a CDR1 having at least 80% sequence identity to SEQ ID NO: 186, a CDR2 having at least 80% sequence identity to SEQ ID NO: 187 and a CDR3 having at least 90%, preferably, 100% sequence identity to SEQ ID NO: 188;
wherein, most preferably, the TRA comprises a variable region having at least 90% sequence identity to SEQ ID NO: 184 and/or the TRB comprises a variable region having at least 90% sequence identity to SEQ ID NO: 189.

In a 21^{st} embodiment, the invention provides a nucleic acid of embodiment 1, wherein the epitope has a sequence of SEQ ID NO: 201, the MHC-I is HLA-C*06:02, and the TRA comprises a CDR3 having at least 90% sequence identity to SEQ ID NO: 193 and/or the TRB comprises a CDR3 having at least 90% sequence identity to SEQ ID NO: 198;
wherein, optionally, the TRA comprises a CDR1 having at least 80% sequence identity to SEQ ID NO: 191, a CDR2 having at least 80% sequence identity to SEQ ID NO: 192 and a CDR3 having at least 90%, preferably, 100% sequence identity to SEQ ID NO: 193, and/or the TRB comprises a CDR1 having at least 80% sequence identity to SEQ ID NO: 196, a CDR2 having at least 80% sequence identity to SEQ ID NO: 197 and a CDR3 having at least 90%, preferably, 100% sequence identity to SEQ ID NO: 198;
wherein, most preferably, the TRA comprises a variable region having at least 90% sequence identity to SEQ ID NO: 194 and/or the TRB comprises a variable region having at least 90% sequence identity to SEQ ID NO: 199.

In a 22^{nd} embodiment, in the nucleic acid of any of embodiments 1-21, if the sequence identity to the recited CDR1 region is not 100%, there is at most one amino acid varying from the recited sequence. Optionally, the sequence identity in the CDR1 region is 100%.

In a 23^{rd} embodiment, in the nucleic acid of any of embodiments 1-22, if the sequence identity to the recited CDR2 region is not 100%, there is at most one amino acid varying from the recited sequence. Optionally, the sequence identity in the CDR2 region is 100%.

In a 24^{th} embodiment, in the nucleic acid of any of embodiments 1-23, if the sequence identity to the recited CDR3 region is not 100%, there is at most one amino acid varying from the recited sequence. Optionally, the sequence identity in the CDR3 region is 100%.

In an 25^{th} embodiment, in the nucleic acid of any of embodiments 1-24, the sequence identity to the recited CDR1 and CDR2 and CDR3 regions is 100%.

In a 26^{th} embodiment, in the nucleic acid of any of embodiments 1-25, the TCR alpha chain construct and/or the TCR beta chain construct further comprise a constant region selected from the group comprising a human constant region, a murine constant region or a chimeric constant region, preferably, both the TCR alpha chain construct and the TCR beta chain construct.

In a 27^{th} embodiment, the nucleic acid of any of embodiments 1-26 encodes at least one TCR alpha and beta chain construct of the TCR construct,
wherein the nucleic acid is selected from the group comprising a viral vector, a transposon, a vector suitable for CRISPR/CAS based recombination or a plasmid suitable for *in vitro* RNA transcription, preferably, a gamma retroviral vector such as MP71.

In a 28th embodiment, the invention provides a protein encoded by of the nucleic acid of any of embodiments 1-27.

In a 29^{th} embodiment, the invention provides a host cell comprising a nucleic acid of any of embodiments 1-27 and/or a protein of embodiment 28, wherein the host cell preferably is a human CD8+ T cell.

In a 30^{th} embodiment, the invention provides a pharmaceutical composition comprising
a) a nucleic acid of any of embodiments 1-27 encoding a TCR construct capable of specifically binding to said epitope in the context of said MHC-I; or
b) a protein of embodiment 28 comprising a TCR construct capable of specifically binding to said epitope in the context of said MHC-I; or
c) a host cell of embodiment 29 expressing a TCR construct capable of specifically binding to said epitope in the context of said MHC-I,
wherein the TCR construct optionally is the TCR construct of embodiment 16 or 15, preferably., the TCR construct of embodiment 16.

In a 31^{st} embodiment, the invention provides a pharmaceutical composition or a kit comprising at least two pharmaceutical compositions comprising
a) at least two nucleic acids, each encoding a TCR construct capable of specifically binding to its respective epitope in the context of the respective MHC-I; or
b) at least two proteins, each comprising a TCR construct capable of specifically binding to its respective epitope in the context of the respective MHC-I; or
c) at least two host cells, each expressing a TCR construct capable of specifically binding to its respective epitope in the context of the respective MHC-I,
wherein the epitopes are peptides from the same antigen expressed by the same cancer or infectious agent, which are capable of being presented by different MHC-I molecules, wherein, optionally, the antigen is MAGEA1,
wherein, preferably, the kit of the pharmaceutical composition is for use in treatment of said cancer or infectious agent.

In a 32^{nd} embodiment, the pharmaceutical composition or kit of embodiment 31 comprises a pharmaceutical composition of embodiment 30.

In a 33^{rd} embodiment, the pharmaceutical composition of any of embodiments 30-31 or the kit comprising the pharmaceutical composition or the kit of any of claims 31-32, comprises
a) at least two nucleic acids of any of embodiments 1-27, each encoding a TCR construct capable of specifically binding to its respective epitope in the context of the respective MHC-I; or
b) at least two proteins of embodiment 28, each comprising a TCR construct capable of specifically binding to its respective epitope in the context of the respective MHC-I; or
c) at least two host cells of embodiment 29, each expressing a TCR construct capable of specifically binding to its respective epitope in the context of the respective MHC-I,
wherein one of said TCR constructs optionally is the TCR construct of embodiment 16 or 15, preferably, the TCR construct of embodiment 16.

In a 34^{th} embodiment, the pharmaceutical composition of any of embodiments 30-31 or the kit comprising the pharmaceutical composition or the kit of any of embodiments 31-32 may be for use in the treatment of a subject expressing said MHC-I and having a cancer expressing MAGEA1 selected from the group comprising, e.g., lung cancer, colorectal cancer, multiple myeloma or any other MAGEA1-expressing cancer,
wherein the treatment preferably is an immunotherapy selected from the group comprising adoptive T cell therapy and TCR gene therapy. Adoptive T cell therapy, wherein the pharmaceutical composition comprises T cells expressing at least one TCR construct of the invention is preferred throughout the invention.

In a 35^{th} embodiment, the invention provides a MAGEA1 peptide comprising at most 25 consecutive amino acids identical to the sequence of amino acids ocurring in MAGEA1 of SEQ ID NO: 10 (wt MAGEA1), comprising an epitope capable of being presented by a human MHC-I, wherein the epitope is selected from the group comprising epitopes
a) having at least 88% sequence identity to any of SEQ ID NO: 1, 2 or 3, wherein the epitope is capable of being presented on HLA-B*44:03,
b) having at least 88% sequence identity to SEQ ID NO: 5, wherein the epitope is capable of being presented on HLA-B*57:01 and HLA-B*57:03 and comprises at most 10 consecutive amino acids identical to the sequence of amino acids ocurring in wt MAGEA1,
c) having at least 88% sequence identity to SEQ ID NO: 7, wherein the epitope is capable of being presented on HLA-A*03:01,
   and
d) having at least 88% sequence identity to SEQ ID NO: 9, wherein the epitope is capable of being presented on HLA-A*29:02,
wherein, preferably, the epitope, when presented by the respective MHC-I, is capable of being specifically recognized by any one of the TCR constructs of embodiment 22.

In a 36^{th} embodiment, the invention provides a MAGEA1 peptide of embodiment 35, comprising at most 25, preferably, at most 20 or at most 15, optionally, at most 11 consecutive amino acids identical to the sequence of amino acids ocurring in wt MAGEA1, comprising an epitope capable of being presented by a human MHC-I, wherein the epitope is selected from the group comprising epitopes
a) of SEQ ID NO: 1, 2 or 3,
b) of SEQ ID NO: 5, wherein the epitope comprises at most 10 consecutive amino acids identical to the sequence of amino acids ocurring in wt MAGEA1,
c) of SEQ ID NO: 7, and
d) of SEQ ID NO: 9.

In a 37^{th} embodiment, the invention provides a MAGEA1 peptide of embodiment 36, comprising at most 20, preferably, at most 15, or at most 11 consecutive amino acids identical to the sequence of amino acids ocurring in wt MAGEA1, comprising an epitope capable of being presented by a human MHC-I, wherein the epitope is selected from the group comprising epitopes
a) having at least 88% sequence identity to any of SEQ ID NO: 1, 2 or 3, wherein the epitope is capable of being presented on HLA-B*44:03,
b) having at least 88% sequence identity to SEQ ID NO: 5, wherein the epitope is capable of being presented on HLA-B*57:01 and HLA-B*57:03 and comprises at most 10 consecutive amino acids identical to the sequence of amino acids ocurring in wt MAGEA1,
c) having at least 88% sequence identity to SEQ ID NO: 7, wherein the epitope is capable of being presented on HLA-A*03:01,
   and
d) having at least 88% sequence identity to SEQ ID NO: 9, wherein the epitope is capable of being presented on HLA-A*29:02.

In a 38^{th} embodiment, the invention provides a MAGEA1 peptide of any of embodiments 35-37, comprising at most 20, preferably, at most 15, or at most 11 consecutive amino acids identical to the sequence of amino acids ocurring in wt MAGEA1, comprising an epitope capable of being presented by a human MHC-I, wherein the epitope is selected from the group comprising epitopes
a) of SEQ ID NO: 1, 2 or 3,
b) of SEQ ID NO: 5, wherein the epitope comprises at most 10 consecutive amino acids identical to the sequence of amino acids ocurring in wt MAGEA1,
c) of SEQ ID NO: 7, and
d) of SEQ ID NO: 9.

In a 39^{th} embodiment, the invention provides a pharmaceutical composition comprising a MAGEA1 peptide of any of embodiments 35-38.

In a 40^{th} embodiment, the invention provides a pharmaceutical composition comprising a MAGEA1 peptide comprising at most 25, optionally, at most 20 or at most 15, preferably, at most 11 consecutive amino acids identical to the sequence of amino acids ocurring in wt MAGEA1, comprising an epitope capable of being presented by a human MHC-I, wherein the epitope is selected from the group comprising epitopes
f) having at least 88%, e.g., 100%, sequence identity to SEQ ID NO: 4, wherein the epitope is capable of being presented on HLA-B*07:02, and
g) having at least 88%, e.e., 100% sequence identity to SEQ ID NO: 8, wherein the epitope is capable of being presented on HLA-A*26:01.

In a 41^{st} embodiment, the pharmaceutical composition of any of embodiments 39 or 40 is for use in vaccinating a subject expressing said MHC-I on which the epitope is capable of being presented.

In a 42^{nd} embodiment, the invention provides a method of treating a subject having a cancer associated with expression of MAGEA1, e.g., lung cancer, colorectal cancer, or multiple myeloma, comprising administering a pharmaceutical composition or a kit of any of embodiments 31-33 or 39-40 to said patient.

In a 43^{rd} embodiment, the invention provides a method of vaccination of a subject, comprising administering the pharmaceutical composition of any of embodiments 37 or 38 to said subject, wherein said subject preferably expresses said MHC-I on which the epitope is capable of being presented. Optionally, said subject has a cancer associated with expression of MAGE-A1, e.g., lung cancer, colorectal cancer, multiple myeloma, or any other MAGEA1-expressing cancer.

### Legends

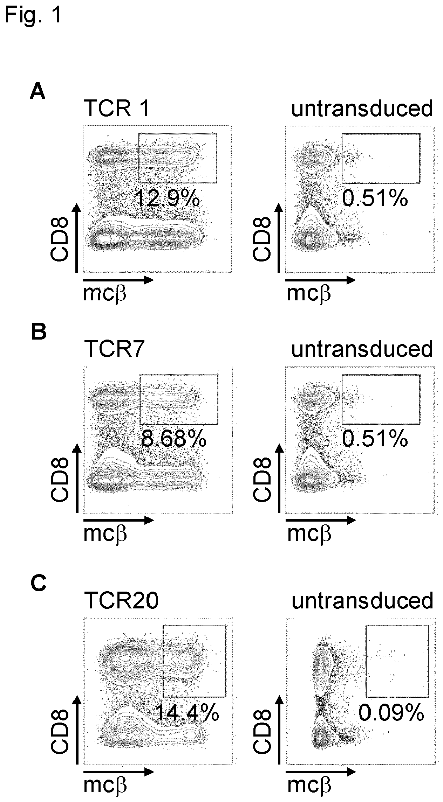

**Figure 1** A-C) Example flow cytometric analysis plots of either TCR-transduced (left) or untransduced (right) human PBMCs.The codon-optimized TCR contains murinized constant regions and can therefore be stained with an anti-mouse constant region beta antibody (mcbeta). CD8+mcbeta+ double-positive populations reflect the percentage of-transduced cytotoxic T cells.
   Shown are representative plots for three different TCRs.

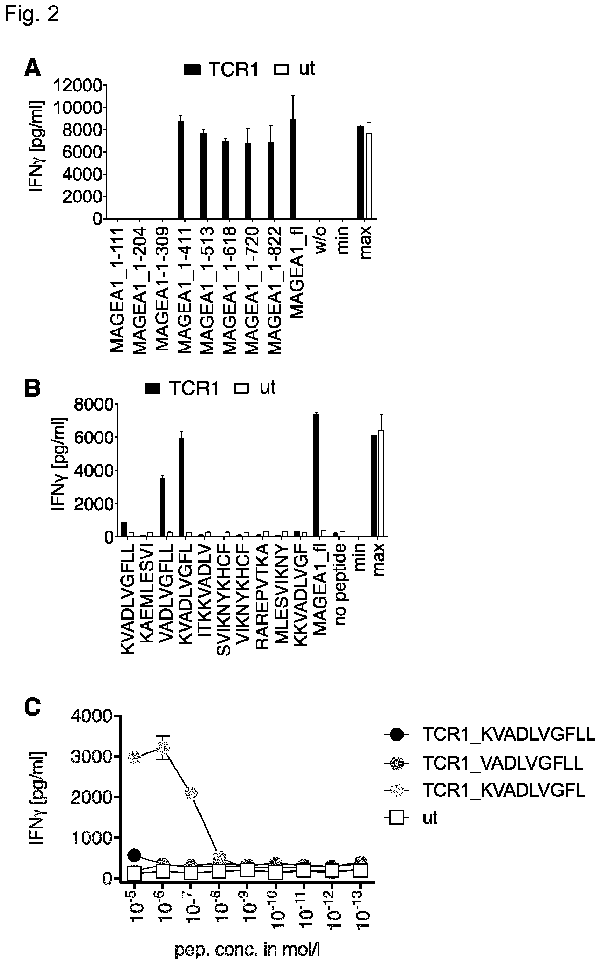

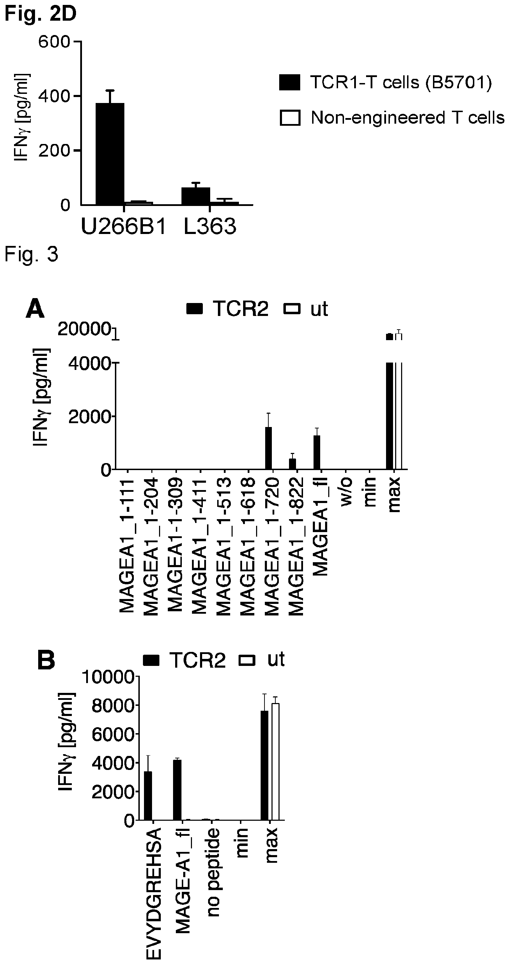

**Figure 2** A) IFNγ production of TCR1-transduced T cells upon stimulation with K562 cells, expressing the respective HLA B*57:03 and different truncated MAGEA1 proteins, or full-length MAGEA1, respectively, was assessed after 20h of co-culture. X-axis indicates the length of each MAGEA1 construct in nucleotides, starting at 1 for the first basepair. MAGEA1_fl: full-length MAGEA1 (930 bp), w/o: contains only K562-HLA B*57:03 and TCR-transduced T cells, min: medium control, max: stimulation with PMA/ lonomycin.
   B) K562-B*57:03 cells were exogenous loaded with 10 different MAGEA1 peptides (10⁻⁶ mol/L) taken from the epitope prediction made by NetMHCpan4.0 and co-cultured with TCR1-transduced T cells. IFNγ release was measured by ELISA 20h later. K562-B*57:03 cells, electroporated with full-length MAGEA1 served as positive control, K562-B*57:03 loaded with no peptide served as negative control. min: medium control; max: stimulation with PMA/ lonomycin.
   C) IFNγ release of TCR1-transduced T cells in response to stimulation with K562-B*57:03, loaded with titrated amounts of MAGEA1 peptides (KVADLVGFLL (SEQ ID NO: 3), VADLVGFLL (SEQ ID NO: 151), KVADLVGFL (SEQ ID NO: 5)). IFNγ release was measured by ELISA 20h later.
   D) Recognition of tumor cell lines U266B1 and L363 by MAGE-A1-specific TCR-T cells. 10,000 CD8+ T cells transduced with TCR1 were co-cultured overnight with 50,000 target tumor cells. The release of the cytotoxic cytokine IFNγ to the supernatant by the TCR-T cells was measured via ELISA. U266B1 and L363 cells were retrovirally transduced with the respective HLA-B*57:01.
**Figure 3** A) IFNγ production of TCR2-transduced T cells upon stimulation with K562 cells, expressing the respective HLA A*26:01 and different truncated MAGEA1 proteins, or full-length MAGEA1, respectively, was assessed after 20h of co-culture. X-axis indicates the length of each MAGEA1 construct in nucleotides, starting at 1 for the first basepair. MAGEA1_fl: full-length MAGEA1 (930 bp), w/o: contains only K562-HLA A*26:01 and TCR-transduced T cells, min: medium control, max: stimulation with PMA/ lonomycin.
   B) K562-A*26:01 cells were exogenous loaded with different MAGEA1 peptide (10⁻⁶mol/L) taken from the epitope prediction made by NetMHCpan4.0 and co-cultured with TCR2-transduced T cells. A total of 12 peptides were tested. Shown is only the stimulating peptide (EVYDGREHSA, SEQ ID NO: 8), which was able to release IFNγ. IFNγ release was measured by ELISA 20h later. K562-A*26:01 cells, electroporated with full-length MAGEA1 served as positive control, K562-A*26:01 loaded with no peptide served as negative control. min: medium control; max: stimulation with PMA/ lonomycin.

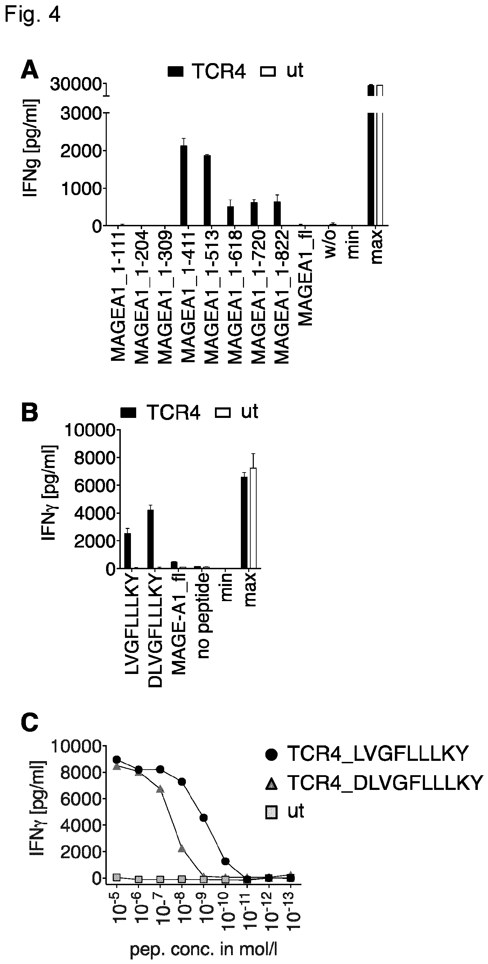

**Figure 4** A) IFNγ production of TCR4-transduced T cells upon stimulation with K562 cells, expressing the respective HLA A*29:02 and different truncated MAGEA1 proteins, or full-length MAGEA1, respectively, was assessed after 20h of co-culture. X-axis indicates the length of each MAGEA1 construct in nucleotides, starting at 1 for the first basepair. MAGEA1_fl: full-length MAGEA1 (930 bp), w/o: contains only K562-HLA A*29:02 and TCR-transduced T cells, min: medium control, max: stimulation with PMA/ lonomycin.
   B) K562-A*29:02 cells were exogenous loaded with different MAGEA1 peptides (10⁻⁶mol/L) taken from the epitope prediction made by NetMHCpan4.0 and co-cultured with TCR4-transduced T cells. A total of 3 peptides were tested. Shown are only the stimulating peptides, which were able to release IFNγ. IFNγ release was measured by ELISA 20h later. K562-A*29:02 cells, electroporated with full-length MAGEA1 served as positive control, K562-A*29:02 loaded with no peptide served as negative control. min: medium control; max: stimulation with PMA/ lonomycin.
   C) IFNγ release of TCR4-transduced T cells in response to stimulation with K562-A*29:02, loaded with titrated amounts of MAGEA1 peptides (LVGFLLLKY (SEQ ID NO: 9), DLVGFLLLKY (SEQ ID NO: 152)). IFNγ release was measured by ELISA 20h later.

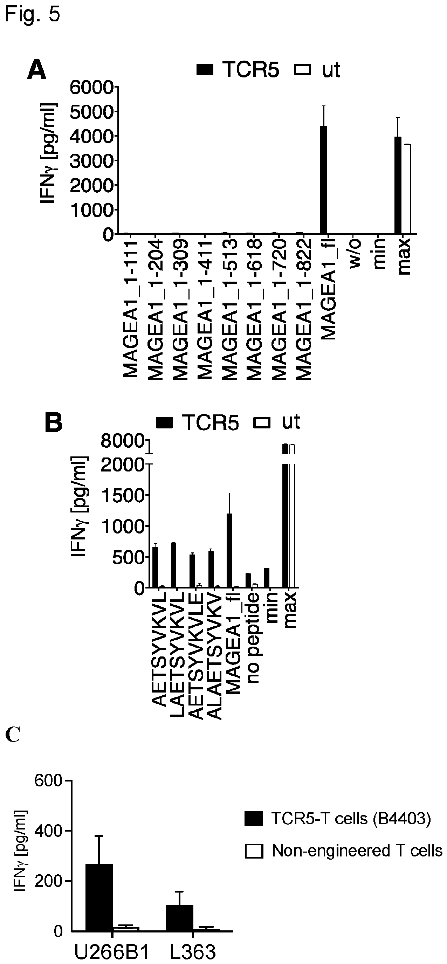

**Figure 5** A) IFNγ production of TCR5-transduced T cells upon stimulation with K562 cells, expressing the respective HLA B*44:03 and different truncated MAGEA1 proteins, or full-length MAGEA1, respectively, was assessed after 20h of co-culture. X-axis indicates the length of each MAGEA1 construct in nucleotides, starting at 1 for the first basepair. MAGEA1_fl: full-length MAGEA1 (930 bp), w/o: contains only K562-HLA B*44:03 and TCR-transduced T cells, min: medium control, max: stimulation with PMA/ lonomycin.
   B) K562-B*44:03 cells were exogenous loaded with different MAGEA1 peptides (10⁻⁶mol/L) taken from the epitope prediction made by NetMHCpan4.0 and co-cultured with TCR5-transduced T cells. A total of 51 peptides were tested. Shown are only the stimulating peptides, which were able to release IFNγ (AETSYVKVL (SEQ ID NO: 153), LAETSYVKVL (SEQ ID NO: 1), AETSYVKVLE (SEQ ID NO: 154), ALAETSYVKV (SEQ ID NO: 155)). IFNγ release was measured by ELISA 20h later. K562-B*44:03 cells, electroporated with full-length MAGEA1 served as positive control, K562-B*44:03 loaded with no peptide served as negative control. min: medium control; max: stimulation with PMA/ lonomycin.
   C) Recognition of tumor cell lines U266B1 and L363 by MAGE-A1-specific TCR-T cells. 10,000 CD8+ T cells transduced with TCR5 were co-cultured overnight with 50,000 target tumor cells. The release of the cytotoxic cytokine IFNγ to the supernatant by the TCR-T cells was measured via ELISA. U266B1 and L363 cells were retrovirally transduced with the respective HLA-B*44:03.

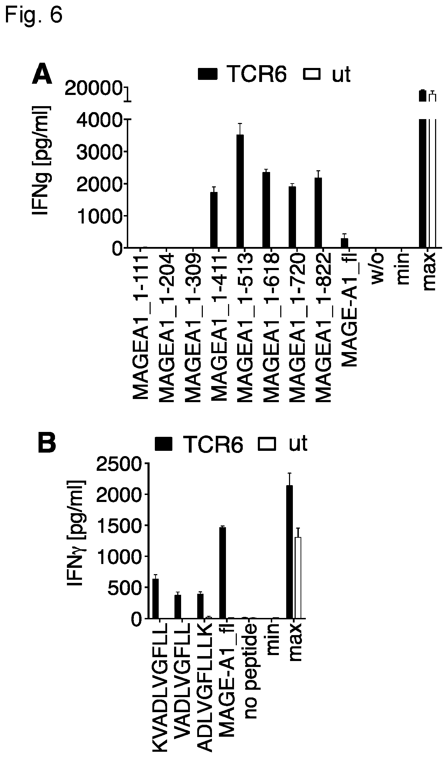

**Figure 6** A) IFNγ production of TCR6-transduced T cells upon stimulation with K562 cells, expressing the respective HLA B*44:03 and different truncated MAGEA1 proteins, or full-length MAGEA1, respectively, was assessed after 20h of co-culture. X-axis indicates the length of each MAGEA1 construct in nucleotides, starting at 1 for the first basepair. MAGEA1_fl: full-length MAGEA1 (930 bp), w/o: contains only K562-HLA B*44:03 and TCR-transduced T cells, min: medium control, max: stimulation with PMA/ lonomycin.
   B) K562-B*44:03 cells were exogenous loaded with different MAGEA1 peptides (10⁻⁶mol/L) taken from the epitope prediction made by NetMHCpan4.0 and co-cultured with TCR6-transduced T cells. A total of 59 peptides were tested. Shown are only the stimulating peptides (KVADLVGFLL (SEQ ID NO: 3), VADLVGFLL (SEQ ID NO: 156), ADLVGFLLLK (SEQ ID NO: 157)), which were able to release IFNγ. IFNγ release was measured by ELISA 20h later. K562-B*44:03 cells, electroporated with full-length MAGEA1 served as positive control, K562- B*44:03 loaded with no peptide served as negative control. min: medium control; max: stimulation with PMA/lonomycin.

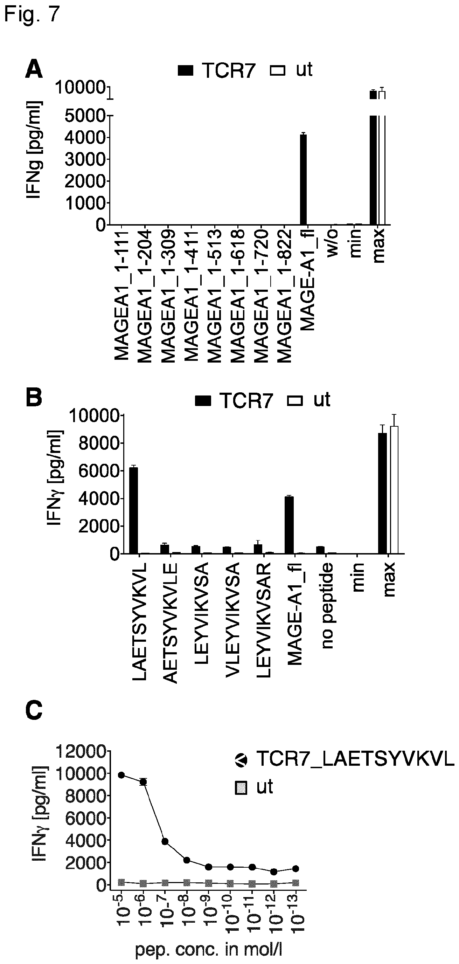

**Figure 7** A) IFNγ production of TCR7-transduced T cells upon stimulation with K562 cells, expressing the respective HLA B*44:03 and different truncated MAGEA1 proteins, or full-length MAGEA1, respectively, was assessed after 20h of co-culture. X-axis indicates the length of each MAGEA1 construct in nucleotides, starting at 1 for the first basepair. MAGEA1_fl: full-length MAGEA1 (930 bp), w/o: contains only K562-HLA B*44:03 and TCR-transduced T cells, min: medium control, max: stimulation with PMA/lonomycin.
   B) K562-B*44:03 cells were exogenous loaded with different MAGEA1 peptides (10⁻⁶mol/L) taken from the epitope prediction made by NetMHCpan4.0 (LAETSYVKVL (SEQ ID NO: 1), AETSYVKVLE (SEQ ID NO: 158), LEYVIKVSA (SEQ ID NO: 159), VLEYVIKVSA (SEQ ID NO: 160), LEYVIKVSAR (SEQ ID NO: 161)) and co-cultured with TCR7-transduced T cells. IFNγ release was measured by ELISA 20h later. K562-B*44:03 cells, electroporated with full-length MAGEA1 served as positive control, K562-B*44:03 loaded with no peptide served as negative control. min: medium control; max: stimulation with PMA/lonomycin.
   C) IFNγ release of TCR7-transduced T cells in response to stimulation with K562- B*44:03, loaded with titrated amounts of the stimulating MAGEA1 peptide (LAETSYVKVL (SEQ ID NO: 1)). IFNγ release was measured by ELISA 20h later.

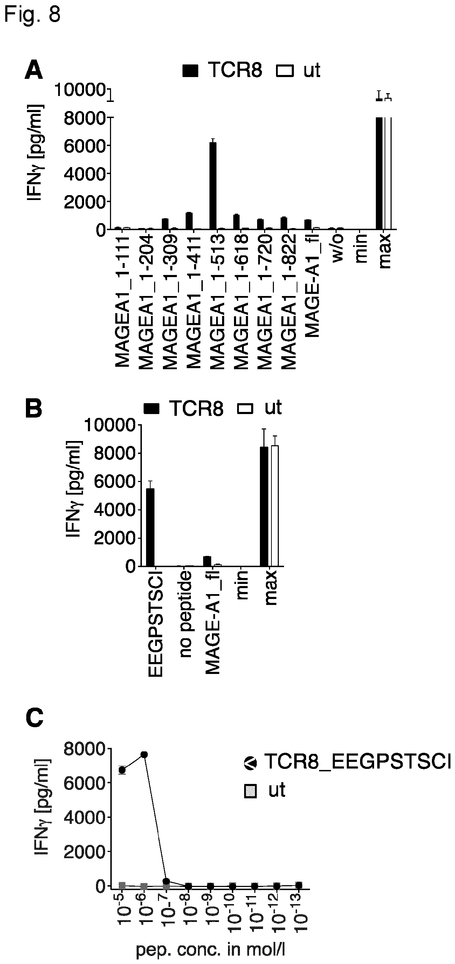

**Figure 8** A) IFNγ production of TCR8-transduced T cells upon stimulation with K562 cells, expressing the respective HLA-B*44:03 and different truncated MAGEA1 proteins, or full-length MAGEA1, respectively, was assessed after 20h of co-culture. X-axis indicates the length of each MAGEA1 construct in nucleotides, starting at 1 for the first basepair. MAGEA1_fl: full-length MAGEA1 (930 bp), w/o: contains only K562-HLA B*44:03 and TCR-transduced T cells, min: medium control, max: stimulation with PMA/lonomycin.
   B) K562-B*44:03 cells were exogenous loaded with different MAGEA1 peptides (10⁻⁶mol/L) taken from the epitope prediction made by NetMHCpan4.0 and co-cultured with TCR8-transduced T cells. A total of 3 peptides were tested. Shown is only the stimulating peptide (EEGPSTSCI, SEQ ID NO: 2), which was able to release IFNγ. IFNγ release was measured by ELISA 20h later. K562-B*44:03 cells, electroporated with full-length MAGEA1 served as positive control, K562-B*44:03 loaded with no peptide served as negative control. min: medium control; max: stimulation with PMA/lonomycin.

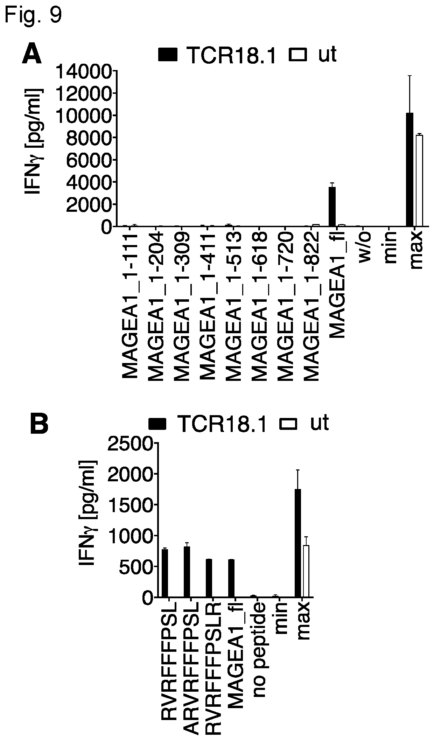

**Figure 9** A) IFNγ production of TCR18.1-transduced T cells upon stimulation with K562 cells, expressing the respective HLA-B*07:02 and different truncated MAGEA1 proteins, or full-length MAGEA1, respectively, was assessed after 20h of co-culture. X-axis indicates the length of each MAGEA1 construct in nucleotides, starting at 1 for the first basepair. MAGEA1_fl: full-length MAGEA1 (930 bp), w/o: contains only K562-HLA-B*07:02 and TCR-transduced T cells, min: medium control, max: stimulation with PMA/lonomycin.
   B) K562-B*07:02 cells were exogenous loaded with different MAGEA1 peptides (10⁻⁶mo!/L) taken from the epitope prediction made by NetMHCpan4.0 and co-cultured with TCR18.1-transduced T cells. A total of 9 peptides were tested. Shown are only the stimulating peptides (RVRFFFPSL (SEQ ID NO: 4), ARVRFFFPSL (SEQ ID NO: 162), RVRFFFPSLR (SEQ ID NO: 163)), which were able to release IFNγ. IFNγ release was measured by ELISA 20h later. K562-B*07:02 cells, electroporated with full-length MAGEA1 served as positive control, K562-B*07:02 loaded with no peptide served as negative control. min: medium control; max: stimulation with PMA/lonomycin.

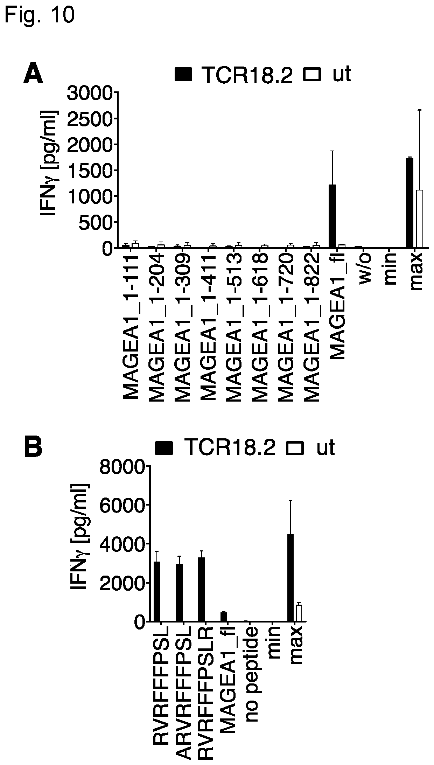

**Figure 10** A) IFNγ production of TCR18.2 -ransduced T cells upon stimulation with K562 cells, expressing the respective HLA-B*07:02 and different truncated MAGEA1 proteins, or full-length MAGEA1, respectively, was assessed after 20h of co-culture. X-axis indicates the length of each MAGEA1 construct in nucleotides, starting at 1 for the first basepair. MAGEA1_fl: full-length MAGEA1 (930 bp), w/o: contains only K562-HLA B*07:02 and TCR-transduced T cells, min: medium control, max: stimulation with PMA/lonomycin.
   B) K562-B*07:02 cells were exogenous loaded with different MAGEA1 peptides (10⁻⁶mol/L) taken from the epitope prediction made by NetMHCpan4.0 and co-cultured with TCR18.2-transduced T cells. A total of 9 peptides were tested. Shown are only the stimulating peptides (RVRFFFPSL (SEQ ID NO: 4), ARVRFFFPSL (SEQ ID NO: 162), RVRFFFPSLR (SEQ ID NO: 163)), which were able to release IFNγ. IFNγ release was measured by ELISA 20h later. K562-B*07:02 cells, electroporated with full-length MAGEA1 served as positive control, K562-B*07:02 loaded with no peptide served as negative control. min: medium control; max: stimulation with PMA/lonomycin.

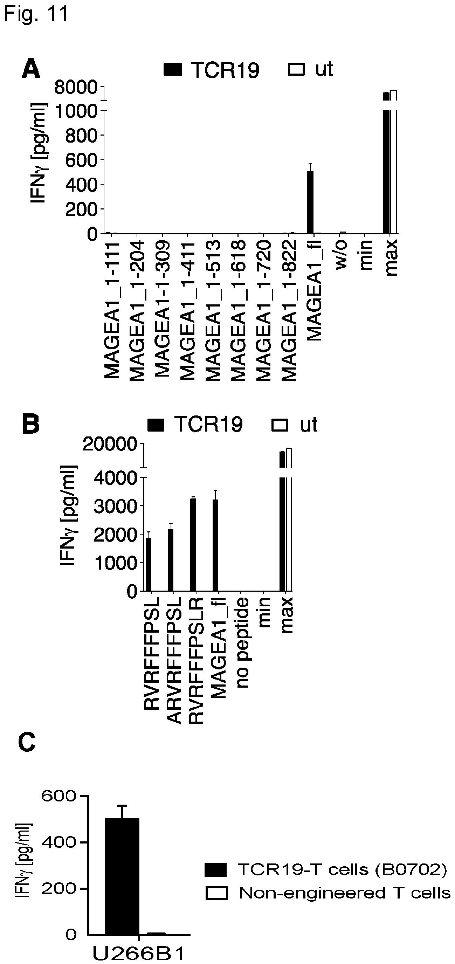

**Figure 11** A) IFNγ production of TCR19-transduced T cells upon stimulation with K562 cells, expressing the respective HLA-B*07:02 and different truncated MAGEA1 proteins, or full-length MAGEA1, respectively, was assessed after 20h of co-culture. X-axis indicates the length of each MAGEA1 construct in nucleotides, starting at 1 for the first basepair. MAGEA1_fl: full-length MAGEA1 (930 bp), w/o: contains only K562-HLA-B*07:02 and TCR-transduced T cells, min: medium control, max: stimulation with PMA/lonomycin.
   B) K562-B*07:02 cells were exogenous loaded with different MAGEA1 peptides (10⁻⁶mol/L) taken from the epitope prediction made by NetMHCpan4.0 and co-cultured with TCR19-transduced T cells. A total of 9 peptides were tested. Shown are only the stimulating peptides (RVRFFFPSL (SEQ ID NO: 4), ARVRFFFPSL (SEQ ID NO: 162), RVRFFFPSLR (SEQ ID NO: 163)), which were able to release IFNγ. IFNγ release was measured by ELISA 20h later. K562-B*07:02 cells, electroporated with full-length MAGEA1 served as positive control, K562-B*07:02 loaded with no peptide served as negative control. min: medium control; max: stimulation with PMA/lonomycin.
   C) Recognition of tumor cell lines U266B1 by MAGE-A1-specific TCR-T cells. 10,000 CD8+ T cells transduced with TCR19 were co-cultured overnight with 50,000 target tumor cells. The release of the cytotoxic cytokine IFNγ to the supernatant by the TCR-T cells was measured via ELISA.

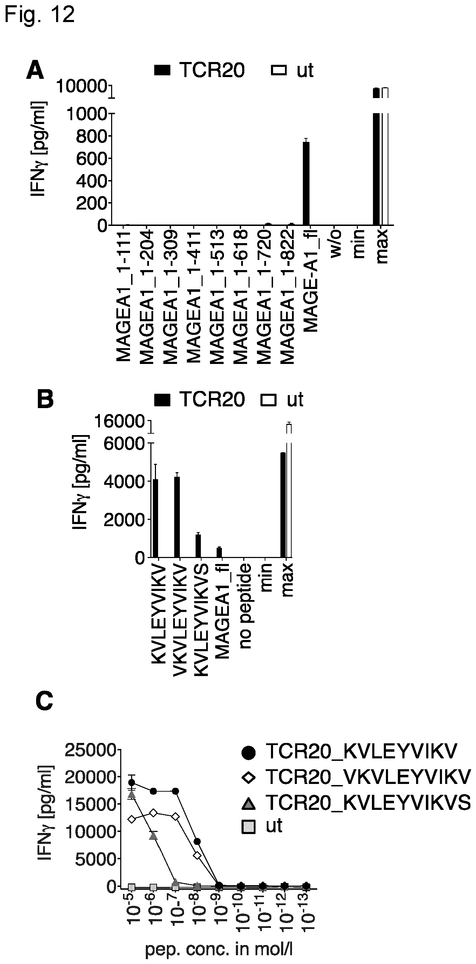

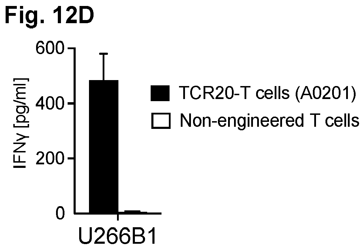

**Figure 12** A) IFNγ production of TCR20-transduced T cells upon stimulation with K562 cells, expressing the respective HLA A*02:01 and different truncated MAGEA1 proteins, or full-length MAGEA1, respectively, was assessed after 20h of co-culture. X-axis indicates the length of each MAGEA1 construct in nucleotides, starting at 1 for the first basepair. MAGEA1_fl: full-length MAGEA1 (930 bp), w/o: contains only K562-HLA A*02:01 and TCR-transduced T cells, min: medium control, max: stimulation with PMA/lonomycin.
   B) K562-A*02:01 cells were exogenous loaded with different MAGEA1 peptides (10⁻⁶mol/L) taken from the epitope prediction made by NetMHCpan4.0 and co-cultured with TCR20-transduced T cells. A total of 7 peptides were tested. Shown are only the stimulating peptides (KVLEYVIKV (SEQ ID NO: 6), VKVLEYVIKV (SEQ ID NO: 164), KVLEYVIKVS (SEQ ID NO: 165)), which were able to release IFNγ. IFNγ release was measured by ELISA 20h later. K562-A*02:01 cells, electroporated with full-length MAGEA1 served as positive control, K562-A*02:01 loaded with no peptide served as negative control. min: medium control; max: stimulation with PMA/lonomycin.
   C) IFNγ release of TCR20-transduced T cells in response to stimulation with K562- A*02:01, loaded with titrated amounts of the stimulating MAGEA1 peptides (KVLEYVIKV (SEQ ID NO: 6), VKVLEYVIKV (SEQ ID NO: 164), KVLEYVIKVS (SEQ ID NO: 165)). IFNγ release was measured by ELISA 20h later.
   D) Recognition of tumor cell lines U266B1 by MAGE-A1-specific TCR-T cells. 10,000 CD8+ T cells transduced with TCR20 were co-cultured overnight with 50,000 target tumor cells. The release of the cytotoxic cytokine IFNγ to the supernatant by the TCR-T cells was measured via ELISA.

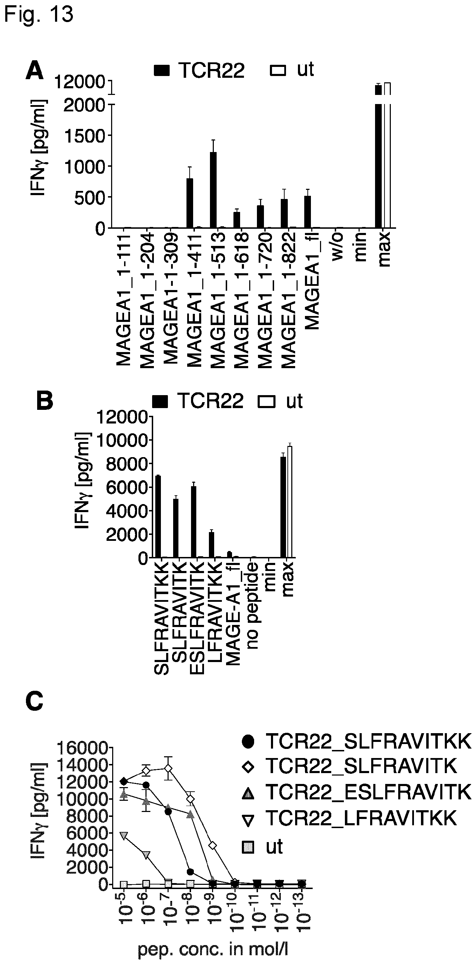

**Figure 13** A) IFNγ production of TCR22-transduced T cells upon stimulation with K562 cells, expressing the respective HLA A*03:01 and different truncated MAGEA1 proteins, or full-length MAGEA1, respectively, was assessed after 20h of co-culture. X-axis indicates the length of each MAGEA1 construct in nucleotides, starting at 1 for the first basepair. MAGEA1_fl: full-length MAGEA1 (930 bp), w/o: contains only K562-HLA A*03:01 and TCR-transduced T cells, min: medium control, max: stimulation with PMA/lonomycin.
   B) K562-A*03:01 cells were exogenous loaded with different MAGEA1 peptides (10⁻⁶mol/L) taken from the epitope prediction made by NetMHCpan4.0 and co-cultured with TCR22-transduced T cells. A total of 10 peptides were tested. Shown are only the stimulating peptides (SLFRAVITKK (SEQ ID NO: 166), SLFRAVITK (SEQ ID NO: 7), ESLFRAVITK (SEQ ID NO: 167), LFRAVITKK (SEQ ID NO: 168)), which were able to release IFNγ. IFNγ release was measured by ELISA 20h later. K562-A*03:01 cells, electroporated with full-length MAGEA1 served as positive control, K562-A*03:01 loaded with no peptide served as negative control. min: medium control; max: stimulation with PMA/lonomycin.
   C) IFNγ release of TCR22-transduced T cells in response to stimulation with K562- A*03:01, loaded with titrated amounts of the stimulating MAGEA1 peptides (SLFRAVITKK (SEQ ID NO: 166), SLFRAVITK (SEQ ID NO: 7), ESLFRAVITK (SEQ ID NO: 167), LFRAVITKK (SEQ ID NO: 168)). IFNγ release was measured by ELISA 20h later.

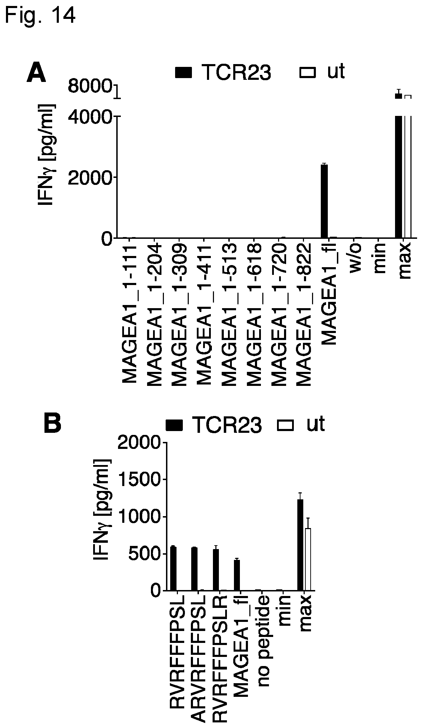

**Figure 14** A) IFNγ production of TCR23-transduced T cells upon stimulation with K562 cells, expressing the respective HLA B*07:02 and different truncated MAGEA1 proteins, or full-length MAGEA1, respectively, was assessed after 20h of co-culture. X-axis indicates the length of each MAGEA1 construct in nucleotides, starting at 1 for the first basepair. MAGEA1_fl: full-length MAGEA1 (930 bp), w/o: contains only K562-HLA B*07:02 and TCR-transduced T cells, min: medium control, max: stimulation with PMA/lonomycin.
   B) K562-B*07:02 cells were exogenous loaded with different MAGEA1 peptides (10⁻⁶mol/L) taken from the epitope prediction made by NetMHCpan4.0 and co-cultured with TCR23-transduced T cells. A total of 9 peptides were tested. Shown are only the stimulating peptides (RVRFFFPSL (SEQ ID NO: 4), ARVRFFFPSL (SEQ ID NO: 162), RVRFFFPSLR (SEQ ID NO: 163)), which were able to release IFNγ. IFNγ release was measured by ELISA 20h later. K562-B*07:02 cells, electroporated with full-length MAGEA1 served as positive control, K562-B*07:02 loaded with no peptide served as negative control. min: medium control; max: stimulation with PMA/lonomycin.

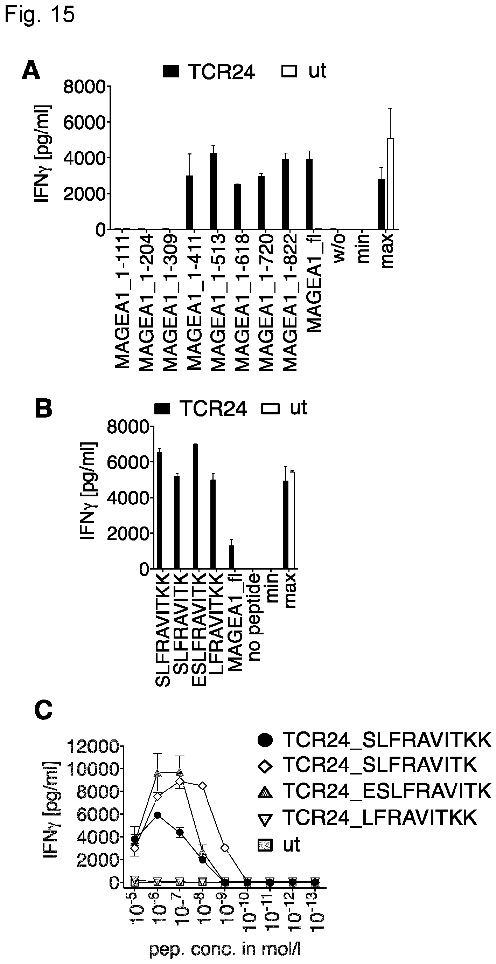

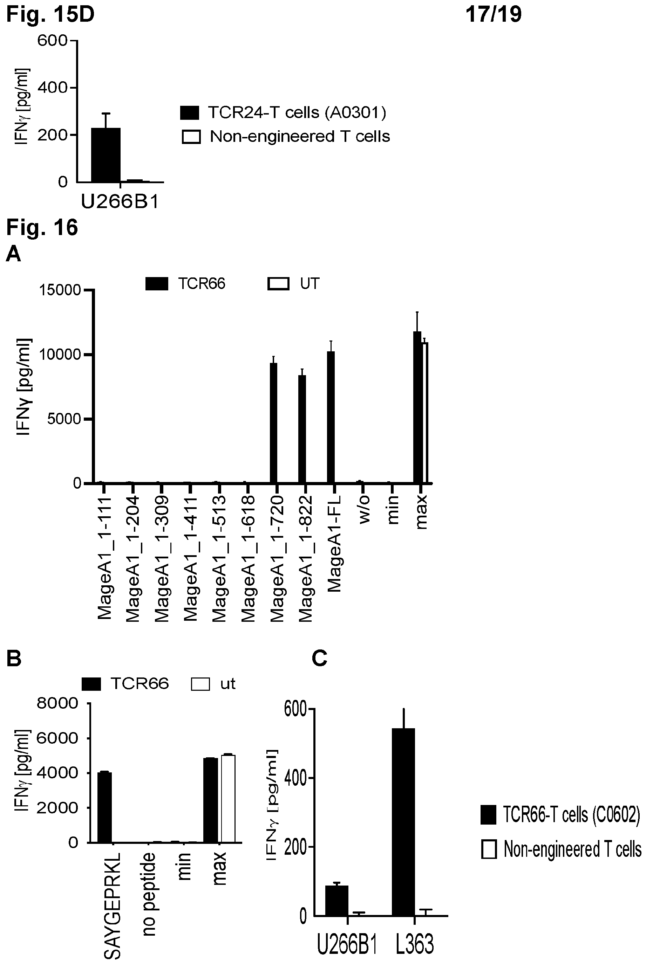

**Figure 15** A) IFNγ production of TCR24-transduced T cells upon stimulation with K562 cells, expressing the respective HLA A*03:01 and different truncated MAGEA1 proteins, or full-length MAGEA1, respectively, was assessed after 20h of co-culture. X-axis indicates the length of each MAGEA1 construct in nucleotides, starting at 1 for the first basepair. MAGEA1_fl: full-length MAGEA1 (930 bp), w/o: contains only K562-HLA A*03:01 and TCR-transduced T cells, min: medium control, max: stimulation with PMA/lonomycin.
   B) K562-A*03:01 cells were exogenous loaded with different MAGEA1 peptides (10⁻⁶mol/L) taken from the epitope prediction made by NetMHCpan4.0 and co-cultured with TCR24-transduced T cells. A total of 10 peptides were tested. Shown are only the stimulating peptides (SLFRAVITKK (SEQ ID NO: 166), SLFRAVITK (SEQ ID NO: 7), ESLFRAVITK (SEQ ID NO: 167), LFRAVITKK (SEQ ID NO: 168)), which were able to release IFNγ. IFNγ release was measured by ELISA 20h later. K562-A*03:01 cells, electroporated with full-length MAGEA1 served as positive control, K562-A*03:01 loaded with no peptide served as negative control. min: medium control; max: stimulation with PMA/lonomycin.
   C) IFNγ release of TCR20-transduced T cells in response to stimulation with K562- A*03:01, loaded with titrated amounts of the stimulating MAGEA1 peptides (SLFRAVITKK (SEQ ID NO: 166), SLFRAVITK (SEQ ID NO: 7), ESLFRAVITK (SEQ ID NO: 167), LFRAVITKK (SEQ ID NO: 168). IFNγ release was measured by ELISA 20h later.
   D) Recognition of tumor cell lines U266B1 by MAGE-A1-specific TCR-T cells. 10,000 CD8+ T cells transduced with TCR24 were co-cultured overnight with 50,000 target tumor cells. The release of the cytotoxic cytokine IFNγ to the supernatant by the TCR-T cells was measured via ELISA.
**Figure 16** A) IFNγ production of TCR66-transduced T cells upon stimulation with K562 cells, expressing the respective HLA C*06:01 and different truncated MAGEA1 proteins, or full-length MAGEA1, respectively, was assessed after 20h of co-culture. X-axis indicates the length of each MAGEA1 construct in nucleotides, starting at 1 for the first basepair. MAGEA1_fl: full-length MAGEA1 (930 bp), w/o: contains only K562-HLA A*03:01 and TCR-transduced T cells, min: medium control, max: stimulation with PMA/lonomycin.
   B) IFNγ release of TCR66-transduced T cells in response to stimulation with K562-C06:02, loaded with titrated amounts of the stimulating MAGEA1 peptides (SAYGEPRKL (SEQ ID NO: 201), IFNγ release was measured by ELISA 20h later.
   C) Recognition of tumor cell lines U266B1 and L363 by MAGE-A1-specific TCR-T cells. 10,000 CD8⁺ T cells transduced with TCR5 were co-cultured overnight with 50,000 target tumor cells. The release of the cytotoxic cytokine IFNγ to the supernatant by the TCR-T cells was measured via ELISA. U266B1 and L363 cells were retrovirally transduced with the respective HLA-B*44:03.

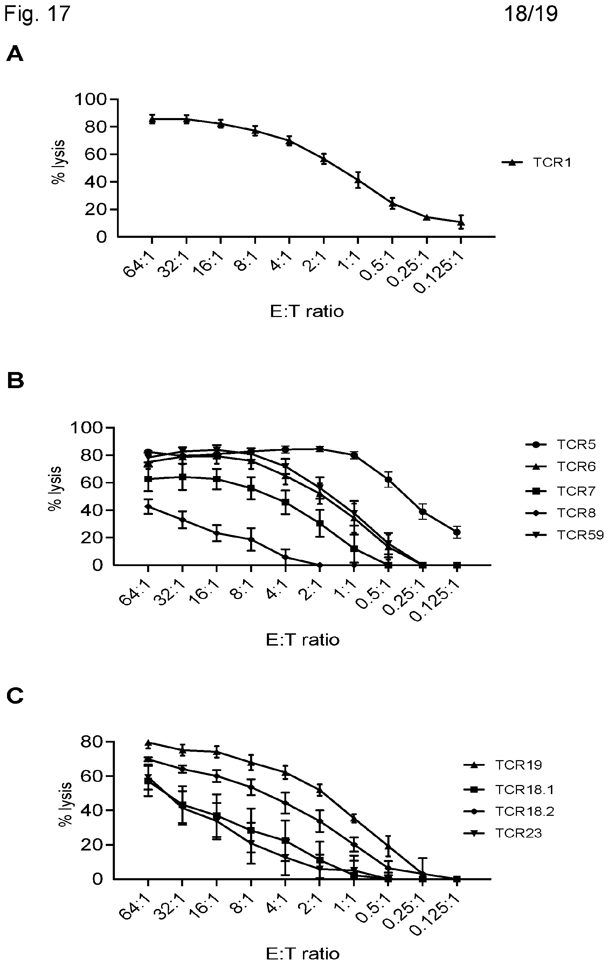

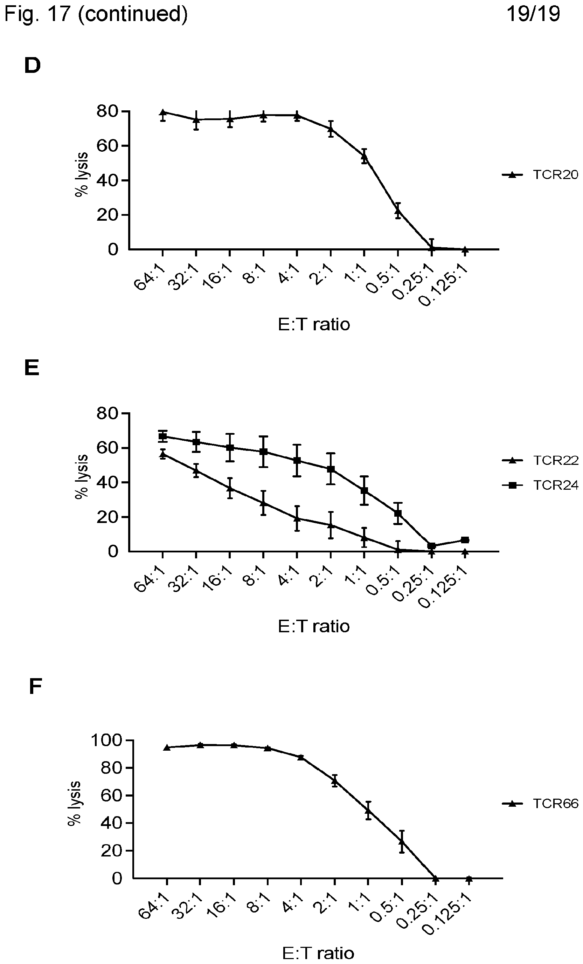

**Figure 17** A) Antigen-specific cytolytic activity of CD8⁺ T cells. 10,000 K562-HLA-B5701-MAGE-A1 tumor cells (target cells, T) expressing luciferase were co-cultured for 18 hours with increasing numbers of T cells (effector, E) up to an effector-to-target (E:T) ratio of 64:1. After 18 hours, live target cells were detected via their luciferase activity. The lysis of target cells was calculated with the following formula: 100-[(luciferase signal T cell co-culture / luciferase signal target cells alone)*100]. Fitted curves are based on a 4PL sigmoidal model, EC50 is determined as concentration of half-maximal lysis. The experiment was performed with four different T cell donors. Symbols and bars show mean ± SEM.
   B) Antigen-specific cytolytic activity of CD8⁺ T cells. 10,000 K562-HLA-B4403-MAGE-A1 tumor cells (target cells, T) expressing luciferase were co-cultured for 18 hours with increasing numbers of T cells (effector, E) up to an effector-to-target (E:T) ratio of 64:1. After 18 hours, live target cells were detected via their luciferase activity. The lysis of target cells was calculated with the following formula: 100-[(luciferase signal T cell co-culture / luciferase signal target cells alone)*100]. Fitted curves are based on a 4PL sigmoidal model, EC50 is determined as concentration of half-maximal lysis. The experiment was performed with four different T cell donors, with the exception of TCR5, where results were obtained from two donors. Symbols and bars show mean ± SEM.
   C) Antigen-specific cytolytic activity of CD8⁺ T cells. 10,000 K562-HLA-B0702-MAGE-A1 tumor cells (target cells, T) expressing luciferase were co-cultured for 18 hours with increasing numbers of T cells (effector, E) up to an effector-to-target (E:T) ratio of 64:1. After 18 hours, live target cells were detected via their luciferase activity. The lysis of target cells was calculated with the following formula: 100-[(luciferase signal T cell co-culture / luciferase signal target cells alone)*100]. Fitted curves are based on a 4PL sigmoidal model, EC50 is determined as concentration of half-maximal lysis. The experiment was performed with four different T cell donors. Symbols and bars show mean ± SEM.
   D) Antigen-specific cytolytic activity of CD8⁺ T cells. 10,000 K562-HLA-A0201-MAGE-A1 tumor cells (target cells, T) expressing luciferase were co-cultured for 18 hours with increasing numbers of T cells (effector, E) up to an effector-to-target (E:T) ratio of 64:1. After 18 hours, live target cells were detected via their luciferase activity. The lysis of target cells was calculated with the following formula: 100-[(luciferase signal T cell co-culture / luciferase signal target cells alone)*100]. Fitted curves are based on a 4PL sigmoidal model, EC50 is determined as concentration of half-maximal lysis. The experiment was performed with four different T cell donors. Symbols and bars show mean ± SEM.
   E) Antigen-specific cytolytic activity of CD8⁺ T cells. 10,000 K562-HLA-A0301-MAGE-A1 tumor cells (target cells, T) expressing luciferase were co-cultured for 18 hours with increasing numbers of T cells (effector, E) up to an effector-to-target (E:T) ratio of 64:1. After 18 hours, live target cells were detected via their luciferase activity. The lysis of target cells was calculated with the following formula: 100-[(luciferase signal T cell co-culture / luciferase signal target cells alone)*100]. Fitted curves are based on a 4PL sigmoidal model, EC50 is determined as concentration of half-maximal lysis. The experiment was performed with four different T cell donors. Symbols and bars show mean ± SEM.
   F) Antigen-specific cytolytic activity of CD8⁺ T cells. 10,000 K562-HLA-C0602-MAGE-A1 tumor cells (target cells, T) expressing luciferase were co-cultured for 18 hours with increasing numbers of T cells (effector, E) up to an effector-to-target (E:T) ratio of 64:1. After 18 hours, live target cells were detected via their luciferase activity. The lysis of target cells was calculated with the following formula: 100-[(luciferase signal T cell co-culture / luciferase signal target cells alone)*100]. Fitted curves are based on a 4PL sigmoidal model, EC50 is determined as concentration of half-maximal lysis. The experiment was performed with four different T cell donors. Symbols and bars show mean ± SEM.

### Detailed description of the invention

Through an innovative method (Lorenz et al., 2017; WO2016146618A1) seventeen MAGEA1-reactive TCRs recognizing endogenously processed and immunogenic MAGEA1 peptides presented in the context of 9 different HLA alleles were identified, among them five TCR recognizing peptides presented in the context of HLA-B*44:03, e.g., TCR 7. As no MAGEA1 epitopes presented on said MHC-I were previously known, the invention, for the first time, enables adoptive T cell therapy or peptide vaccination of patients expressing said MHC-I.

Similarly, the invention provides MAGEA1 epitopes presented on HLA-B*07:02, HLA-A*26:01 and HLA-A*29:02, HLA-A*29:02, HLA-C*06:02 and TCR specifically recognizing MAGEA1 epitopes in the context of said MHCs, and thus renders immunotherapy of patients expressing said MHCs possible.

Epitopes presented on HLA-B*57:01 or HLA-B*57:03, HLA-A*02:01 and HLA-A*03 were already known in the art (EP 1117679, EP 2394655 A2, Celis et al., 1994; Chaux et al., 1999; Corbiere et al., 2004, Pascolo et al., 2001), but the present invention provides novel and advantageous TCR specifically recognizing said epitope/MHC complexes.

Furthermore, the present invention provides an innovative combination therapy. A combination of T cells expressing TCR constructs capable of specifically recognizing epitopes, typically, different epitopes, from the same antigen, e.g., MAGEA1, which are capable of being presented by different MHC-I molecules was found to be more effective than one of T cells expressing only one TCR construct capable of the epitope.

Therefore, the invention also provides such combinations of T cells, or corresponding combinations on the nucleic acid level or protein level, e.g., for use in treatment of MAGEA1 positive cancer, wherein the subject to be treated expresses the MHC-I molecules on which the resepctive epitopes are presented. For example, one or more of said T cell constructs capable of specifically recognizing MAGEA1 epitopes can be a TCR construct of the present invention. Optionally, one of said TCR constructs is a TCR construct recognizing a MAGEA1-peptide in the context of HLA-B*44:03, e.g., TCR7 or a related construct.

### Nucleic acids

In a first embodiment, the invention provides a nucleic acid encoding a TCR alpha chain construct (TRA) and/or a TCR beta chain construct (TRB) of a TCR construct specific for an epitope in complex with a human MHC-I, wherein the epitope is an epitope of a MAGEA1 protein,
a) wherein the epitope has the sequence of SEQ ID NO: 1 (LAETSYVKVL), the MHC-I is HLA-B*44:03, and the TRA comprises a CDR3 having at least 90% sequence identity to SEQ ID NO: 13 and/or the TRB comprises a CDR3 having at least 90% sequence identity to SEQ ID NO: 18;
b) wherein the epitope has the sequence of SEQ ID NO: 2 (EEGPSTSCI), the MHC-I is HLA-B*44:03, and the TRA comprises a CDR3 having at least 90% sequence identity to SEQ ID NO: 23 and/or the TRB comprises a CDR3 having at least 90% sequence identity to SEQ ID NO: 28;
c) wherein the epitope has the sequence of SEQ ID NO: 3 (KVADLVGFLL), the MHC-I is HLA-B*44:03, and the TRA comprises a CDR3 having at least 90% sequence identity to SEQ ID NO: 33 and/or the TRB comprises a CDR3 having at least 90% sequence identity to SEQ ID NO: 38;
d) wherein the epitope has the sequence of SEQ ID NO: 1 (LAETSYVKVL), the MHC-I is HLA-B*44:03, and the TRA comprises a CDR3 having at least 90% sequence identity to SEQ ID NO: 43 and/or the TRB comprises a CDR3 having at least 90% sequence identity to SEQ ID NO: 48;
e) wherein the epitope has the sequence of SEQ ID NO: 4 (RVRFFFPSL), the MHC-I is HLA-B*07:02, and the TRA comprises a CDR3 having at least 90% sequence identity to SEQ ID NO: 53 and/or the TRB comprises a CDR3 having at least 90% sequence identity to SEQ ID NO: 58;
f) wherein the epitope has the sequence of SEQ ID NO: 4 (RVRFFFPSL), the MHC-I is HLA-B*07:02, and the TRA comprises a CDR3 having at least 90% sequence identity to SEQ ID NO: 63 and/or the TRB comprises a CDR3 having at least 90% sequence identity to SEQ ID NO: 68;
g) wherein the epitope has the sequence of SEQ ID NO: 4 (RVRFFFPSL), the MHC-I is HLA-B*07:02, and the TRA comprises a CDR3 having at least 90% sequence identity to SEQ ID NO: 73 and/or the TRB comprises a CDR3 having at least 90% sequence identity to SEQ ID NO: 78;
h) wherein the epitope has the sequence of SEQ ID NO: 4 (RVRFFFPSL), the MHC-I is HLA-B*07:02, and the TRA comprises a CDR3 having at least 90% sequence identity to SEQ ID NO: 83 and/or the TRB comprises a CDR3 having at least 90% sequence identity to SEQ ID NO: 88;
i) wherein the epitope has the sequence of SEQ ID NO: 5 (KVADLVGFL), the MHC-I is HLA-B*57:01 and B*57:03, and the TRA comprises a CDR3 having at least 90% sequence identity to SEQ ID NO: 93 and/or the TRB comprises a CDR3 having at least 90% sequence identity to SEQ ID NO: 98;
j) wherein the epitope has the sequence of SEQ ID NO: 6 (KVLEYVIKV), the MHC-I is HLA-A*02:01, and the TRA comprises a CDR3 having at least 90% sequence identity to SEQ ID NO: 103 and/or the TRB comprises a CDR3 having at least 90% sequence identity to SEQ ID NO: 108;
k) wherein the epitope has the sequence of SEQ ID NO: 7 (SLFRAVITK), the MHC-I is HLA-A*03:01, and the TRA comprises a CDR3 having at least 90% sequence identity to SEQ ID NO: 113 and/or the TRB comprises a CDR3 having at least 90% sequence identity to SEQ ID NO: 118;
l) wherein the epitope has the sequence of SEQ ID NO: 7 (SLFRAVITK), the MHC-I is HLA-A*03:01, and the TRA comprises a CDR3 having at least 90% sequence identity to SEQ ID NO: 123 and/or the TRB comprises a CDR3 having at least 90% sequence identity to SEQ ID NO: 128;
m) wherein the epitope has the sequence of SEQ ID NO: 8 (EVYDGREHSA), the MHC-I is HLA-A*26:01, and the TRA comprises a CDR3 having at least 90% sequence identity to SEQ ID NO: 133 and/or the TRB comprises a CDR3 having at least 90% sequence identity to SEQ ID NO: 138; and/or
n) wherein the epitope has the sequence of SEQ ID NO: 9 (LVGFLLLKY), the MHC-I is HLA-A*29:02, and the TRA comprises a CDR3 having at least 90% sequence identity to SEQ ID NO: 143 and/or the TRB comprises a CDR3 having at least 90% sequence identity to SEQ ID NO: 148; and/or
o) wherein the MHC-I is HLA-A*29:02, and the TRA comprises a CDR3 having at least 90% sequence identity to SEQ ID NO: 173 and/or the TRB comprises a CDR3 having at least 90% sequence identity to SEQ ID NO: 178; and/or
p) wherein the MHC-I is HLA-B*44:03, and the TRA comprises a CDR3 having at least 90% sequence identity to SEQ ID NO: 183 and/or the TRB comprises a CDR3 having at least 90% sequence identity to SEQ ID NO: 188; and/or
q) wherein the epitope has the sequence of SEQ ID NO: 201, the MHC-I is HLA-C*06:02, and the TRA comprises a CDR3 having at least 90% sequence identity to SEQ ID NO: 193 and/or the TRB comprises a CDR3 having at least 90% sequence identity to SEQ ID NO: 198.

In the context of the present invention, "a" is understood to mean "one or more" unless expressly stated otherwise. Accordingly, for example, if the TCR construct of the invention contains both alpha and beta chain constructs, as preferred throughout the invention, it may be encoded by either one or two nucleic acids. The alpha and beta chain constructs together are capable of specifically binding to an epitope from the MAGEA1 protein in complex with a human MHC-I. As intermediate products, the alpha and beta chain constructs are also subject matter of the invention by themselves. The invention also provides a single chain nucleic acid construct, wherein, e.g., TCR alpha and beta chain constructs are separated by a P2A element.

The MHC-I on which the MAGEA1 peptide is presented may be HLA-B*44:03. No TCR against MAGEA1 peptides presented on said HLA have previously been disclosed in the art.

One nucleic acid of the invention encodes a TRA and/or a TRB of a TCR construct specific for an epitope in complex with a human MHC-I, wherein the epitope recognized by the TCR construct has the sequence of SEQ ID NO: 1 and is presented by HLA-B*44:03, and the TRA comprises a CDR3 having at least 90% sequence identity to SEQ ID NO: 13 and/or the TRB comprises a CDR3 having at least 90% sequence identity to SEQ ID NO: 18. Such constructs are designated TCR7-related constructs. SEQ ID NO: 1 is an epitope from MAGEA1 protein which is presently, for the first time, shown to be presented by an MHC-I.

Optionally, the TRA comprises a CDR1 having at least 80% sequence identity to SEQ ID NO: 11, a CDR2 having at least 80% sequence identity to SEQ ID NO: 12 and a CDR3 having at least 90%, preferably, 100% sequence identity to SEQ ID NO: 13. Optionally, the TRB comprises a CDR1 having at least 80% sequence identity to SEQ ID NO: 16, a CDR2 having at least 80% sequence identity to SEQ ID NO: 17 and a CDR3 having at least 90%, preferably, 100% sequence identity to SEQ ID NO: 18.

The TRA may comprise a variable region having at least 90%, optionally, at least 95% or 100% sequence identity to SEQ ID NO: 14. The TRB may comprise a variable region having at least 90%, optionally, at least 95% or 100% sequence identity to SEQ ID NO: 19.

A TCR construct comprising a TRA with a variable region of SEQ ID NO: 14 and a TRB with a variable region of SEQ ID NO: 19 has been shown to have particularly advantageous characteristics herein. It is also designated TCR7.

The variable region of the TRA of said TCR construct may be encoded by a nucleic acid having a sequence of SEQ ID NO: 15, and/or the variable region of the TRB of said TCR construct may be encoded by a nucleic acid having a sequence of SEQ ID NO: 20. Said sequences are codon optimized for expression in human cells, which is preferred. Of course, non-optimized sequences can also be used.

One nucleic acid of the invention encodes a TRA and/or a TRB of a TCR construct specific for an epitope in complex with a human MHC-I, wherein the epitope has the sequence of SEQ ID NO: 2 and is presented by HLA-B*44:03, and the TRA comprises a CDR3 having at least 90% sequence identity to SEQ ID NO: 23 and/or the TRB comprises a CDR3 having at least 90% sequence identity to SEQ ID NO: 28. Such constructs are designated TCR8-related constructs. SEQ ID NO: 2 is an epitope from MAGEA1 protein which is presently, for the first time, shown to be presented by an MHC-I.

Optionally, the TRA comprises a CDR1 having at least 80% sequence identity to SEQ ID NO: 21, a CDR2 having at least 80% sequence identity to SEQ ID NO: 22 and a CDR3 having at least 90%, preferably, 100% sequence identity to SEQ ID NO: 23. Optionally, the TRB comprises a CDR1 having at least 80% sequence identity to SEQ ID NO: 26, a CDR2 having at least 80% sequence identity to SEQ ID NO: 27 and a CDR3 having at least 90%, preferably, 100% sequence identity to SEQ ID NO: 28.

The TRA may comprise a variable region having at least 90%, optionally, at least 95% or 100% sequence identity to SEQ ID NO: 24 and/or the TRB comprises a variable region having at least 90%, optionally, at least 95% or 100% sequence identity to SEQ ID NO: 29.

A TCR construct comprising a TRA with a variable region of SEQ ID NO: 24 and a TRB with a variable region of SEQ ID NO: 29 has been shown to have advantageous characteristics herein. It is also designated TCR8.

The variable region of the TRA of said TCR construct may be encoded by a nucleic acid having a sequence of SEQ ID NO: 25, and/or the variable region of the TRB of said TCR construct may be encoded by a nucleic acid having a sequence of SEQ ID NO: 30. Said sequences are codon optimized for expression in human cells, which is preferred. Of course, non-optimized sequences can also be used.

One nucleic acid of the invention encodes a TRA and/or a TRB of a TCR construct specific for an epitope in complex with a human MHC-I, wherein the epitope has the sequence of SEQ ID NO: 3 and is presented by HLA-B*44:03, and the TRA comprises a CDR3 having at least 90% sequence identity to SEQ ID NO: 33 and/or the TRB comprises a CDR3 having at least 90% sequence identity to SEQ ID NO: 38. Such constructs are designated TCR6-related constructs. SEQ ID NO: 3 is an epitope from MAGEA1 protein which is presently, for the first time, shown to be presented by an MHC-I.

Optionally, the TRA comprises a CDR1 having at least 80% sequence identity to SEQ ID NO: 31, a CDR2 having at least 80% sequence identity to SEQ ID NO: 32 and a CDR3 having at least 90%, preferably, 100% sequence identity to SEQ ID NO: 33. Optionally, the TRB comprises a CDR1 having at least 80% sequence identity to SEQ ID NO: 36, a CDR2 having at least 80% sequence identity to SEQ ID NO: 37 and a CDR3 having at least 90%, preferably, 100% sequence identity to SEQ ID NO: 38.

The TRA may comprise a variable region having at least 90%, optionally, at least 95% or 100% sequence identity to SEQ ID NO: 34. The TRB may comprise a variable region having at least 90%, optionally, at least 95% or 100% sequence identity to SEQ ID NO: 39.

A TCR construct comprising a TRA with a variable region of SEQ ID NO: 34 and a TRB with a variable region of SEQ ID NO: 39 has been shown to have advantageous characteristics herein. It is also designated TCR6.

The variable region of the TRA of said TCR construct may be encoded by a nucleic acid having a sequence of SEQ ID NO: 35, and/or the variable region of the TRB of said TCR construct may be encoded by a nucleic acid having a sequence of SEQ ID NO: 40. Said sequences are codon optimized for expression in human cells, which is preferred. Of course, non-optimized sequences can also be used.

One nucleic acid of the invention encodes a TRA and/or a TRB of a TCR construct specific for an epitope in complex with a human MHC-I, wherein the epitope has the sequence of SEQ ID NO: 1 and the epitope is presented by HLA-B*44:03, and wherein the TRA comprises a CDR3 having at least 90% sequence identity to SEQ ID NO: 43 and/or the TRB comprises a CDR3 having at least 90% sequence identity to SEQ ID NO: 48. Such constructs are designated TCR5-related constructs. Due to their high killing capacity and maximum killing, which, e.g., has been shown to be about 80% or more, TCR5-related constructs are preferred HLA-B*44:03-restricted TCR constructs of the invention.

Optionally, the TRA comprises a CDR1 having at least 80% sequence identity to SEQ ID NO: 41, a CDR2 having at least 80% sequence identity to SEQ ID NO: 42 and a CDR3 having at least 90%, preferably, 100% sequence identity to SEQ ID NO: 43. Optionally, the TRB comprises a CDR1 having at least 80% sequence identity to SEQ ID NO: 46, a CDR2 having at least 80% sequence identity to SEQ ID NO: 47 and a CDR3 having at least 90%, preferably, 100% sequence identity to SEQ ID NO: 48.

The TRA may comprise a variable region having at least 90%, optionally, at least 95% or 100% sequence identity to SEQ ID NO: 44 and/or the TRB comprises a variable region having at least 90%, optionally, at least 95% or 100% sequence identity to SEQ ID NO: 49.

A TCR construct comprising a TRA with a variable region of SEQ ID NO: 44 and a TRB with a variable region of SEQ ID NO: 49 has been show to have advantageous characteristics herein. It is also designated TCR5.

The variable region of the TRA of said TCR construct may be encoded by a nucleic acid having a sequence of SEQ ID NO: 45, and/or the variable region of the TRB of said TCR construct may be encoded by a nucleic acid having a sequence of SEQ ID NO: 50. Said sequences are codon optimized for expression in human cells, which is preferred. Of course, non-optimized sequences can also be used.

The invention also provides TCR constructs capable of recognizing MAGEA1 epitopes presented in the context of HLA-B*07:02.

One nucleic acid of the invention encodes a TRA and/or a TRB of a TCR construct specific for an epitope in complex with a human MHC-I, wherein the epitope has the sequence of SEQ ID NO: 4, the MHC-I is HLA-B*07:02, and the TRA comprises a CDR3 having at least 90% sequence identity to SEQ ID NO: 53 and/or the TRB comprises a CDR3 having at least 90% sequence identity to SEQ ID NO: 58. Such constructs are designated TCR18.1-related constructs. SEQ ID NO: 4 is an epitope from MAGEA1 protein which is presently, for the first time, shown to be presented by HLA-B*07:02. It was previously shown to be presented by Cw7 (C*07:01, C*07:02 (Goodyear et al., 2011).

Optionally, the TRA comprises a CDR1 having at least 80% sequence identity to SEQ ID NO: 51, a CDR2 having at least 80% sequence identity to SEQ ID NO: 52 and a CDR3 having at least 90%, preferably, 100% sequence identity to SEQ ID NO: 53. Optionally, the TRB comprises a CDR1 having at least 80% sequence identity to SEQ ID NO: 56, a CDR2 having at least 80% sequence identity to SEQ ID NO: 57 and a CDR3 having at least 90%, preferably, 100% sequence identity to SEQ ID NO: 58.

The TRA may comprise a variable region having at least 90%, optionally, at least 95% or 100% sequence identity to SEQ ID NO: 54. The TRB may comprise a variable region having at least 90%, optionally, at least 95% or 100% sequence identity to SEQ ID NO: 59. A TCR construct comprising a TRA with a variable region of SEQ ID NO: 54 and a TRB with a variable region of SEQ ID NO: 59 has been shown to have advantageous characteristics herein. It is also designated TCR18.1.

The variable region of the TRA of said TCR construct may be encoded by a nucleic acid having a sequence of SEQ ID NO: 55, and/or the variable region of the TRB of said TCR construct may be encoded by a nucleic acid having a sequence of SEQ ID NO: 60. Said sequences are codon optimized for expression in human cells, which is preferred. Of course, non-optimized sequences can also be used.

One nucleic acid of the invention encodes a TRA and/or a TRB of a TCR construct specific for an epitope in complex with a human MHC-I, wherein the epitope also has the sequence of SEQ ID NO: 4 and the MHC-I is HLA-B*07:02, and the TRA comprises a CDR3 having at least 90% sequence identity to SEQ ID NO: 63 and/or the TRB comprises a CDR3 having at least 90% sequence identity to SEQ ID NO: 68. Such constructs are designated TCR18.2-related constructs.

Optionally, the TRA comprises a CDR1 having at least 80% sequence identity to SEQ ID NO: 61, a CDR2 having at least 80% sequence identity to SEQ ID NO: 62 and a CDR3 having at least 90%, preferably, 100% sequence identity to SEQ ID NO: 63. Optionally, the TRB comprises a CDR1 having at least 80% sequence identity to SEQ ID NO: 66, a CDR2 having at least 80% sequence identity to SEQ ID NO: 67 and a CDR3 having at least 90%, preferably, 100% sequence identity to SEQ ID NO: 68.

The TRA may comprise a variable region having at least 90%, optionally, at least 95% or 100% sequence identity to SEQ ID NO: 64. The TRB may comprise a variable region having at least 90%, optionally, at least 95% or 100% sequence identity to SEQ ID NO: 69.

A TCR construct comprising a TRA with a variable region of SEQ ID NO: 64 and a TRB with a variable region of SEQ ID NO: 69 has been shown to have advantageous characteristics herein. It is also designated TCR18.2.

The variable region of the TRA of said TCR construct may be encoded by a nucleic acid having a sequence of SEQ ID NO: 65, and/or the variable region of the TRB of said TCR construct may be encoded by a nucleic acid having a sequence of SEQ ID NO: 70. Said sequences are codon optimized for expression in human cells, which is preferred. Of course, non-optimized sequences can also be used.

One nucleic acid of the invention encodes a TRA and/or a TRB of a TCR construct specific for an epitope in complex with a human MHC-I, wherein the epitope also has the sequence of SEQ ID NO: 4 and the MHC-I is HLA-B*07:02, and the TRA comprises a CDR3 having at least 90% sequence identity to SEQ ID NO: 73 and/or the TRB comprises a CDR3 having at least 90% sequence identity to SEQ ID NO: 78. Such constructs are designated TCR23-related constructs.

Optionally, the TRA comprises a CDR1 having at least 80% sequence identity to SEQ ID NO: 71, a CDR2 having at least 80% sequence identity to SEQ ID NO: 72 and a CDR3 having at least 90%, preferably, 100% sequence identity to SEQ ID NO: 73. Optionally, the TRB comprises a CDR1 having at least 80% sequence identity to SEQ ID NO: 76, a CDR2 having at least 80% sequence identity to SEQ ID NO: 77 and a CDR3 having at least 90%, preferably, 100% sequence identity to SEQ ID NO: 78.

The TRA may comprise a variable region having at least 90%, optionally, at least 95% or 100% sequence identity to SEQ ID NO: 74. The TRB may comprise a variable region having at least 90%, optionally, at least 95% or 100% sequence identity to SEQ ID NO: 79. A TCR construct comprising a TRA with a variable region of SEQ ID NO: 74 and a TRB with a variable region of SEQ ID NO: 79 has been shown to have advantageous characteristics herein. It is also designated TCR23.

The variable region of the TRA of said TCR construct may be encoded by a nucleic acid having a sequence of SEQ ID NO: 75, and/or the variable region of the TRB of said TCR construct may be encoded by a nucleic acid having a sequence of SEQ ID NO: 80. Said sequences are codon optimized for expression in human cells, which is preferred. Of course, non-optimized sequences can also be used.

One nucleic acid of the invention encodes a TRA and/or a TRB of a TCR construct specific for an epitope in complex with a human MHC-I, wherein the epitope also has the sequence of SEQ ID NO: 4 and the MHC-I also is HLA-B*07:02, and the TRA comprises a CDR3 having at least 90% sequence identity to SEQ ID NO: 83 and/or the TRB comprises a CDR3 having at least 90% sequence identity to SEQ ID NO: 88. Such constructs are designated TCR19-related constructs.

Optionally, the TRA comprises a CDR1 having at least 80% sequence identity to SEQ ID NO: 81, a CDR2 having at least 80% sequence identity to SEQ ID NO: 82 and a CDR3 having at least 90%, preferably, 100% sequence identity to SEQ ID NO: 83. Optionally, the TRB comprises a CDR1 having at least 80% sequence identity to SEQ ID NO: 86, a CDR2 having at least 80% sequence identity to SEQ ID NO: 87 and a CDR3 having at least 90%, preferably, 100% sequence identity to SEQ ID NO: 88.

The TRA may comprise a variable region having at least 90%, optionally, at least 95% or 100% sequence identity to SEQ ID NO: 84. The TRB may comprises a variable region having at least 90%, optionally, at least 95% or 100% sequence identity to SEQ ID NO: 89.

A TCR construct comprising a TRA with a variable region of SEQ ID NO: 84 and a TRB with a variable region of SEQ ID NO: 89 has been shown to have particularly advantageous characteristics herein. It is also designated TCR19.

The variable region of the TRA of said TCR construct may be encoded by a nucleic acid having a sequence of SEQ ID NO: 85, and/or the variable region of the TRB of said TCR construct may be encoded by a nucleic acid having a sequence of SEQ ID NO: 90. Said sequences are codon optimized for expression in human cells, which is preferred. Of course, non-optimized sequences can also be used.

One nucleic acid of the invention encodes a TRA and/or a TRB of a TCR construct specific for an epitope in complex with a human MHC-I, wherein the epitope has the sequence of SEQ ID NO: 5, the MHC-I is HLA-B*57:03, and the TRA comprises a CDR3 having at least 90% sequence identity to SEQ ID NO: 93 and/or the TRB comprises a CDR3 having at least 90% sequence identity to SEQ ID NO: 98. Such constructs are designated TCR1-related constructs. SEQ ID NO: 5 is an epitope from MAGEA1 protein which is presently, for the first time, shown to be presented by an MHC-I. Of note, the inventors found that the TCR1-related constructs of the invention are specific for said epitope both in context of HLA-B*57:03 and HLA-B*57:01. Some experiments underlying this application were done with HLA-B*57:03 and HLA-B*57:01. HLA-B*57:01 is more common than HLA-B*57:03, so it is advantageous that patients having this allele can also be treated with this TCR.

Optionally, the TRA comprises a CDR1 having at least 80% sequence identity to SEQ ID NO: 91, a CDR2 having at least 80% sequence identity to SEQ ID NO: 92 and a CDR3 having at least 90%, preferably, 100% sequence identity to SEQ ID NO: 93. Optionally, the TRB comprises a CDR1 having at least 80% sequence identity to SEQ ID NO: 96, a CDR2 having at least 80% sequence identity to SEQ ID NO: 97 and a CDR3 having at least 90%, preferably, 100% sequence identity to SEQ ID NO: 98.

The TRA may comprise a variable region having at least 90%, optionally, at least 95% or 100% sequence identity to SEQ ID NO: 94. The TRB may comprise a variable region having at least 90%, optionally, at least 95% or 100% sequence identity to SEQ ID NO: 99.

A TCR construct comprising a TRA with a variable region of SEQ ID NO: 94 and a TRB with a variable region of SEQ ID NO: 99 has been shown to have advantageous characteristics herein. It is also designated TCR1.

The variable region of the TRA of said TCR construct may be encoded by a nucleic acid having a sequence of SEQ ID NO: 95, and/or the variable region of the TRB of said TCR construct may be encoded by a nucleic acid having a sequence of SEQ ID NO: 100. Said sequences are codon optimized for expression in human cells, which is preferred. Of course, non-optimized sequences can also be used.

The invention also provides TCR constructs recognizing epitopes of MAGEA1 on HLA-A*02:01, which is a common MHC-I. Other HLA-A2 epitopes of MAGEA1 are already known.

One nucleic acid of the invention encodes a TRA and/or a TRB of a TCR construct specific for an epitope in complex with a human MHC-I, wherein the epitope has the sequence of SEQ ID NO: 6, the MHC-I is HLA-A*02:01, and the TRA comprises a CDR3 having at least 90% sequence identity to SEQ ID NO: 103 and/or the TRB comprises a CDR3 having at least 90% sequence identity to SEQ ID NO: 108. Such constructs are designated TCR20-related constructs.

Optionally, the TRA comprises a CDR1 having at least 80% sequence identity to SEQ ID NO: 101, a CDR2 having at least 80% sequence identity to SEQ ID NO: 102 and a CDR3 having at least 90%, preferably, 100% sequence identity to SEQ ID NO: 103. Optionally, the TRB comprises a CDR1 having at least 80% sequence identity to SEQ ID NO: 106, a CDR2 having at least 80% sequence identity to SEQ ID NO: 107 and a CDR3 having at least 90%, preferably, 100% sequence identity to SEQ ID NO: 108.

The TRA may comprise a variable region having at least 90%, optionally, at least 95% or 100% sequence identity to SEQ ID NO: 104. The TRB may comprise a variable region having at least 90%, optionally, at least 95% or 100% sequence identity to SEQ ID NO: 109.

A TCR construct comprising a TRA with a variable region of SEQ ID NO: 104 and a TRB with a variable region of SEQ ID NO: 109 has been shown to have particularly advantageous characteristics herein. It is also designated TCR20.

The variable region of the TRA of said TCR construct may be encoded by a nucleic acid having a sequence of SEQ ID NO: 105, and/or the variable region of the TRB of said TCR construct may be encoded by a nucleic acid having a sequence of SEQ ID NO: 110. Said sequences are codon optimized for expression in human cells, which is preferred. Of course, non-optimized sequences can also be used.

HLA-A*03:01 is a common MHC-I allele, e.g., in the European or Caucasian population. There thus is a high need in the art for options for treatment of patients expressing said HLA.

One nucleic acid of the invention encodes a TRA and/or a TRB of a TCR construct specific for an epitope in complex with a human MHC-I, wherein the epitope has the sequence of SEQ ID NO: 7, the MHC-I is HLA-A*03:01, and the TRA comprises a CDR3 having at least 90% sequence identity to SEQ ID NO: 113 and/or the TRB comprises a CDR3 having at least 90% sequence identity to SEQ ID NO: 118. Such constructs are designated TCR22-related constructs. SEQ ID NO: 7 is an epitope from MAGEA1 protein which is presently, for the first time, shown to be presented by an MHC-I.

Optionally, the TRA comprises a CDR1 having at least 80% sequence identity to SEQ ID NO: 111, a CDR2 having at least 80% sequence identity to SEQ ID NO: 112 and a CDR3 having at least 90%, preferably, 100% sequence identity to SEQ ID NO: 113. Optionally, the TRB comprises a CDR1 having at least 80% sequence identity to SEQ ID NO: 116, a CDR2 having at least 80% sequence identity to SEQ ID NO: 117 and a CDR3 having at least 90%, preferably, 100% sequence identity to SEQ ID NO: 118.

The TRA may comprise a variable region having at least 90%, optionally, at least 95% or 100% sequence identity to SEQ ID NO: 114. The TRB may comprise a variable region having at least 90%, optionally, at least 95% or 100% sequence identity to SEQ ID NO: 119.

A TCR construct comprising a TRA with a variable region of SEQ ID NO: 114 and a TRB with a variable region of SEQ ID NO: 119 has been shown to have advantageous characteristics herein. It is also designated TCR22.

The variable region of the TRA of said TCR construct may be encoded by a nucleic acid having a sequence of SEQ ID NO: 115, and/or the variable region of the TRB of said TCR construct may be encoded by a nucleic acid having a sequence of SEQ ID NO: 120. Said sequences are codon optimized for expression in human cells, which is preferred. Of course, non-optimized sequences can also be used.

A further nucleic acid of the invention encodes a TRA and/or a TRB of a TCR construct specific for an epitope in complex with a human MHC-I, wherein the epitope also has the sequence of SEQ ID NO: 7, and the MHC-I is HLA-A*03:01. Here, the TRA comprises a CDR3 having at least 90% sequence identity to SEQ ID NO: 123 and/or the TRB comprises a CDR3 having at least 90% sequence identity to SEQ ID NO: 128. Such constructs are designated TCR24-related constructs.

Optionally, the TRA comprises a CDR1 having at least 80% sequence identity to SEQ ID NO: 121, a CDR2 having at least 80% sequence identity to SEQ ID NO: 122 and a CDR3 having at least 90%, preferably, 100% sequence identity to SEQ ID NO: 123. Optionally, the TRB comprises a CDR1 having at least 80% sequence identity to SEQ ID NO: 126, a CDR2 having at least 80% sequence identity to SEQ ID NO: 127 and a CDR3 having at least 90%, preferably, 100% sequence identity to SEQ ID NO: 128. Such constructs have been found to be functionally advantageous, e.g., due to their high killing capacity and high maximum killing, which may be about 70% or more in the experiments described below, and they are therefore preferred TCR constructs of the invention.

The TRA may comprise a variable region having at least 90%, optionally, at least 95% or 100% sequence identity to SEQ ID NO: 124. The TRB may comprise a variable region having at least 90%, optionally, at least 95% or 100% sequence identity to SEQ ID NO: 129.

A TCR construct comprising a TRA with a variable region of SEQ ID NO: 124 and/or a TRB with a variable region of SEQ ID NO: 129 has been shown to have advantageous characteristics herein. It is also designated TCR24.TCR24 is a particularly preferred TCR.

The variable region of the TRA of said TCR construct may be encoded by a nucleic acid having a sequence of SEQ ID NO: 125, and/or the variable region of the TRB of said TCR construct may be encoded by a nucleic acid having a sequence of SEQ ID NO: 130. Said sequences are codon optimized for expression in human cells, which is preferred. Of course, non-optimized sequences can also be used.

The MHC-I on which the MAGEA1 peptide is presented may also be HLA-A*26:01.

One nucleic acid of the invention encodes a TRA and/or a TRB of a TCR construct specific for an epitope in complex with a human MHC-I, wherein the epitope has the sequence of SEQ ID NO: 8, the MHC-I is HLA-A*26:01, and the TRA comprises a CDR3 having at least 90% sequence identity to SEQ ID NO: 133 and/or the TRB comprises a CDR3 having at least 90% sequence identity to SEQ ID NO: 138. Such constructs are designated TCR2-related constructs. An epitope of SEQ ID NO: 8 has previously been described as presented by HLA-A*28 (Chaux et al., 1999).

Optionally, in TCR2 related constructs, the TRA comprises a CDR1 having at least 80% sequence identity to SEQ ID NO: 131, a CDR2 having at least 80% sequence identity to SEQ ID NO: 132 and a CDR3 having at least 90%, preferably, 100% sequence identity to SEQ ID NO: 133. Optionally, the TRB comprises a CDR1 having at least 80% sequence identity to SEQ ID NO: 136, a CDR2 having at least 80% sequence identity to SEQ ID NO: 137 and a CDR3 having at least 90%, preferably, 100% sequence identity to SEQ ID NO: 138.

The TRA may comprise a variable region having at least 90%, optionally, at least 95% or 100% sequence identity to SEQ ID NO: 134. The TRB may comprise a variable region having at least 90%, optionally, at least 95% or 100% sequence identity to SEQ ID NO: 139.

A TCR construct comprising a TRA with a variable region of SEQ ID NO: 134 and a TRB with a variable region of SEQ ID NO: 139 has been shown to have advantageous characteristics herein. It is also designated TCR2.

The variable region of the TRA of said TCR construct may be encoded by a nucleic acid having a sequence of SEQ ID NO: 135, and/or the variable region of the TRB of said TCR construct may be encoded by a nucleic acid having a sequence of SEQ ID NO: 140. Said sequences are codon optimized for expression in human cells, which is preferred. Of course, non-optimized sequences can also be used.

The MHC-I on which the MAGEA1 peptide is presented may also be HLA-A*29:02.

One nucleic acid of the invention encodes a TRA and/or a TRB of a TCR construct specific for an epitope in complex with a human MHC-I, wherein the epitope has the sequence of SEQ ID NO: 9, the MHC-I is HLA-A*29:02, and the TRA comprises a CDR3 having at least 90% sequence identity to SEQ ID NO: 143 and/or the TRB comprises a CDR3 having at least 90% sequence identity to SEQ ID NO: 148. Such constructs are designated TCR4-related constructs. SEQ ID NO: 9 is an epitope from MAGEA1 protein which is presently, for the first time, shown to be presented by an MHC-I.

Optionally, the TRA comprises a CDR1 having at least 80% sequence identity to SEQ ID NO: 141, a CDR2 having at least 80% sequence identity to SEQ ID NO: 142 and a CDR3 having at least 90%, preferably, 100% sequence identity to SEQ ID NO: 143. Optionally, the TRB comprises a CDR1 having at least 80% sequence identity to SEQ ID NO: 146, a CDR2 having at least 80% sequence identity to SEQ ID NO: 147 and a CDR3 having at least 90%, preferably, 100% sequence identity to SEQ ID NO: 148.

The TRA may comprise a variable region having at least 90%, optionally, at least 95% or 100% sequence identity to SEQ ID NO: 144. The TRB may comprise a variable region having at least 90%, optionally, at least 95% or 100% sequence identity to SEQ ID NO: 149.

A TCR construct comprising a TRA with a variable region of SEQ ID NO: 144 and a TRB with a variable region of SEQ ID NO: 149 has been shown to have advantageous characteristics herein. It is also designated TCR2.

The variable region of the TRA of said TCR construct may be encoded by a nucleic acid having a sequence of SEQ ID NO: 145, and/or the variable region of the TRB of said TCR construct may be encoded by a nucleic acid having a sequence of SEQ ID NO: 150. Said sequences are codon optimized for expression in human cells, which is preferred. Of course, non-optimized sequences can also be used.

One nucleic acid of the invention encodes a TRA and/or a TRB of a TCR construct specific for an epitope in complex with a human MHC-I, wherein the MHC-I is HLA-A*29:02, and the TRA comprises a CDR3 having at least 90% sequence identity to SEQ ID NO: 173 and/or the TRB comprises a CDR3 having at least 90% sequence identity to SEQ ID NO: 178. Such constructs are designated TCR56-related constructs.

Optionally, the TRA comprises a CDR1 having at least 80% sequence identity to SEQ ID NO: 171, a CDR2 having at least 80% sequence identity to SEQ ID NO: 172 and a CDR3 having at least 90%, preferably, 100% sequence identity to SEQ ID NO: 173. Optionally, the TRB comprises a CDR1 having at least 80% sequence identity to SEQ ID NO: 176, a CDR2 having at least 80% sequence identity to SEQ ID NO: 177 and a CDR3 having at least 90%, preferably, 100% sequence identity to SEQ ID NO: 178.

The TRA may comprise a variable region having at least 90%, optionally, at least 95% or 100% sequence identity to SEQ ID NO: 174. The TRB may comprise a variable region having at least 90%, optionally, at least 95% or 100% sequence identity to SEQ ID NO: 179.

A TCR construct comprising a TRA with a variable region of SEQ ID NO: 174 and a TRB with a variable region of SEQ ID NO: 179 has been shown to have advantageous characteristics herein. It is also designated TCR56.

The variable region of the TRA of said TCR construct may be encoded by a nucleic acid having a sequence of SEQ ID NO: 175, and/or the variable region of the TRB of said TCR construct may be encoded by a nucleic acid having a sequence of SEQ ID NO: 180. Said sequences are codon optimized for expression in human cells, which is preferred. Of course, non-optimized sequences can also be used.

One nucleic acid of the invention encodes a TRA and/or a TRB of a TCR construct specific for an epitope in complex with a human MHC-I, wherein the MHC-I is HLA-B*44:03, and the TRA comprises a CDR3 having at least 90% sequence identity to SEQ ID NO: 183 and/or the TRB comprises a CDR3 having at least 90% sequence identity to SEQ ID NO: 188. Such constructs are designated TCR59-related constructs.

Optionally, the TRA comprises a CDR1 having at least 80% sequence identity to SEQ ID NO: 181, a CDR2 having at least 80% sequence identity to SEQ ID NO: 182 and a CDR3 having at least 90%, preferably, 100% sequence identity to SEQ ID NO: 183. Optionally, the TRB comprises a CDR1 having at least 80% sequence identity to SEQ ID NO: 186, a CDR2 having at least 80% sequence identity to SEQ ID NO: 187 and a CDR3 having at least 90%, preferably, 100% sequence identity to SEQ ID NO: 188.

The TRA may comprise a variable region having at least 90%, optionally, at least 95% or 100% sequence identity to SEQ ID NO: 184. The TRB may comprise a variable region having at least 90%, optionally, at least 95% or 100% sequence identity to SEQ ID NO: 189.

A TCR construct comprising a TRA with a variable region of SEQ ID NO: 184 and a TRB with a variable region of SEQ ID NO: 189 has been shown to have advantageous characteristics herein. It is also designated TCR59.

The variable region of the TRA of said TCR construct may be encoded by a nucleic acid having a sequence of SEQ ID NO: 185, and/or the variable region of the TRB of said TCR construct may be encoded by a nucleic acid having a sequence of SEQ ID NO: 190. Said sequences are codon optimized for expression in human cells, which is preferred. Of course, non-optimized sequences can also be used.

One nucleic acid of the invention encodes a TRA and/or a TRB of a TCR construct specific for an epitope in complex with a human MHC-I, wherein the epitope has the sequence of SEQ ID NO: 201, the MHC-I is HLA-C*06:02, and the TRA comprises a CDR3 having at least 90% sequence identity to SEQ ID NO: 193 and/or the TRB comprises a CDR3 having at least 90% sequence identity to SEQ ID NO: 198. Such constructs are designated TCR66-related constructs.

Optionally, the TRA comprises a CDR1 having at least 80% sequence identity to SEQ ID NO: 191, a CDR2 having at least 80% sequence identity to SEQ ID NO: 192 and a CDR3 having at least 90%, preferably, 100% sequence identity to SEQ ID NO: 193. Optionally, the TRB comprises a CDR1 having at least 80% sequence identity to SEQ ID NO: 196, a CDR2 having at least 80% sequence identity to SEQ ID NO: 197 and a CDR3 having at least 90%, preferably, 100% sequence identity to SEQ ID NO: 198. Such TCR constructs are preferred due to their excellent killing capacity. in particular, they can have a particularly high killing capacity of more than 90%, and up to 100%.

The TRA may comprise a variable region having at least 90%, optionally, at least 95% or 100% sequence identity to SEQ ID NO: 194. The TRB may comprise a variable region having at least 90%, optionally, at least 95% or 100% sequence identity to SEQ ID NO: 199.

A TCR construct comprising a TRA with a variable region of SEQ ID NO: 194 and a TRB with a variable region of SEQ ID NO: 199 has been shown to have advantageous characteristics herein. It is also designated TCR66.

The variable region of the TRA of said TCR construct may be encoded by a nucleic acid having a sequence of SEQ ID NO: 195, and/or the variable region of the TRB of said TCR construct may be encoded by a nucleic acid having a sequence of SEQ ID NO: 200. Said sequences are codon optimized for expression in human cells, which is preferred. Of course, non-optimized sequences can also be used.

In any of the TRA and/or TRB constructs of the invention, independently, the CDR1, CDR2 and CDR3 may have at least 80%, at least 90% or 100% sequece identity to the recited sequences, and the CDR3 may have at least 90% or 100% sequece identity to the recited sequences. Preferably, the CDR3 has 100% sequence identity to the recited defined CDR3. Typically, if the sequence is not identical, there is at most one amino acid exchange, deletion or insertion, typically, an amino acid exchange. The exchange may be a conserved substitution, i.e., one amino acid of a particular type (e.g., polar, apolar, acidic, basic, aromatic) is exchanged against another amino acid of the same type. Methods for affinity maturation are known in the art and further described below.

Optionally, the sequence identity to the recited CDR1 and CDR2 and CDR3 regions is 100% in the TRA or TRB, preferably, in both TRA and TRB of the TCR constructs of the invention.

A TCR alpha and/or beta chain construct of the invention may comprise all characteristics or domains corresponding to its native counterpart, i.e., a TCR alpha or beta chain, but this is not essential. Preferably, the TCR alpha and/or beta chain construct comprises at least a variable region, or a variable and a constant region, e.g., the variable and/or constant region having at least 60%, at least 70%, at least 80%, at least 90% or at least 95% sequence identity to a human variable or constant TCR region.

The TCR alpha chain constructs and/or the TCR beta chain constructs of the invention preferably comprise a constant region. For adoptive TCR therapy, it is preferred that the TCR construct comprises full length TCR alpha and beta chains comprising variable and constant regions, including transmembrane regions. The constant region may be a human constant region, a murine constant region or a chimeric constant region. The TCR construct may be of essentially or exclusively human origin to minimize immunogenicity. To prevent pairing with endogenous TCR chains, the constructs of the invention however preferably contain one or more, e.g., 1-5, 1-10 or 1-20, amino acid exchanges, insertions or deletions in comparison to a human sequence, e.g., providing an additional cysteine to enable formation of an additional disulfide bond (Sommermeyer and Uckert, 2010). To this end, the constant region of the TCR alpha and beta chain construct may also be a murine constant region.

Alternatively, expression of endogenous TCR chains may also be suppressed, e.g., by expression of suitable miRNA.

The construct may also be a chimeric antigen receptor, or part of it, wherein, e.g. a human TCR variable region may be linked to a different immunoglobulin constant domain, e.g. an IgG constant domain, or to an antibody domain capable of specifically binding to an antigen such as CD19.

Single chain constructs (scTCR) are encompassed as well as heterodimeric TCR constructs. A scTCR can comprise a variable region of a first TCR chain construct (e.g., an alpha chain) and an entire (full-length) second TCR chain (e.g., a beta chain), or vice versa. Furthermore, the scTCR can optionally comprise one or more linkers which join the two or more polypeptides together. The linker can be, for instance, a peptide which joins together two single chains. Also provided is such a scTCR of the invention, which is fused to a cytokine, e.g., a human cytokine, such as IL-2, IL-7 or IL-15.

To enable specific recognition of the epitope in the context of the MHC, in particular, for therapeutic purposes, the nucleic acid of the invention encodes one TCR alpha and one beta chain construct of a TCR construct of the invention, which together recognize the respective epitope. e.g., a TCR construct specific for the epitope of SEQ ID NO: 1, as described herein.

Typically, the nucleic acid sequences provided by the invention are codon-optimized for expression in human cells.

In the context of the invention, the nucleic acid can either be DNA or RNA. Preferably, it is DNA. The nucleic acid may, e.g., be a viral vector, a transposon, a vector suitable for CRISPR/CAS based recombination or a plasmid suitable for *in vitro* RNA transcription. The nucleic acid of the invention preferably is a vector. Suitable vectors include those designed for propagation and expansion, or for expression or both, such as plasmids and viruses. The vector may be an expression vector suitable for expression in a host cell selected from the group comprising a human T cell or a human T cell precursor, preferably, a human T cell such as CD8+ T cell. Said CD8+ cell may be a central-memory T cell, effector-memory T cell, stem cell-like T cell or effector T cell, or a mixture of these. The vector may be a viral vector, e.g. a retroviral, in particular gamma-retroviral or lentiviral vector. Examples of suitable expression vectors include the retroviral vector MP71. The expression vector comprises regulatory sequences, such as transcription and translation initiation and termination codons, which are specific to the type of host cell (e.g., bacterium, fungus, plant, animal or human) into which the vector is to be introduced and in which the expression of the nucleic acid of the invention shall be performed, typically, in the context of the invention, human CD8+ T cells. Furthermore, the vector of the invention may include one or more marker genes, which allow for selection of transduced or transfected hosts. The expression vector can comprise a native or, preferably, a heterologous promoter operably linked to the nucleotide sequence encoding the TCR construct of the invention, or to a nucleotide sequence which is complementary to or which hybridizes to the nucleotide sequence encoding the constructs of the invention. The selection of promoters includes, e.g., strong, weak, inducible, tissue-specific and developmental-specific promoters. The promoter can be a non-viral promoter or a viral promoter. Preferably, it is a heterologous promoter, i.e., a promoter not naturally linked to a TCR in human T cells, such as long terminal repeat promoter, which is suitable for expression in human T cells. The inventive recombinant expression vectors can be designed for either transient expression, for stable expression, or for both. Also, the recombinant expression vectors can be made for constitutive expression or for inducible expression.

### Proteins

The present invention also provides a protein, i.e., an alpha or beta chain construct, or, preferably, a TCR construct comprising both alpha and beta chain constructs capable of specifically binding an epitope from a MAGEA1-protein in the context of the respective MHC-I, as described herein, e.g., a TCR construct specific for the epitope of SEQ ID NO: 1, as described herein. TCR constructs specific for the epitope of SEQ ID NO: 7 are particularly advantageous, as this epitope can be presented on the common HLA allele HLA-A*03:01. The protein is encoded by a nucleic acid of the invention.

The terms "capable of specifically binding", "capable of (specifically) recognizing" or "recognizing" or "specific for" a given antigen, as used herein, are synonymous, and mean that the TCR construct can specifically bind to and immunologically recognize said epitope, preferably with high affinity.

The term does not necessarily mean that the TCR construct is not capable of recognizing other proteins, too. In particular, the TCR construct may further recognize slightly longer peptides comprising the sequences defined herein, e.g., with one N-terminal and/or C-terminal additional amino acid, which is typically also present in the wt MAGEA1 sequence of SEQ ID NO: 10. They may also recognize slightly shorter pepides, e.g., lacking a C-terminal or an N-terminal amino acid, or peptides shifted by one position in the MAGEA1 sequence. For example, TCR 1-related constructs specifically recognize KVADLVGFL (SEQ ID NO: 5), but further recognize VADLVGFLL (SEQ ID NO: 151).

For example, a TCR may be considered to "be capable of specifically binding" to a peptide from a MAGEA1 protein, if T cells expressing the TCR secrete at least about 200 pg/mL or more (e.g. 250 pg/mL or more, 300 pg/mL or more, 400 pg/mL or more, 500 pg/mL or more, 600 pg/mL or more, 700 pg/mL or more, 1000 pg/mL or more, 2,000 pg/mL or more, 2,500 pg/mL or more, 5,000 pg/mL or more) of interferon γ (IFN-y) upon co-culture with target cells pulsed with a low concentration of the respective epitope, e.g., about 10⁻¹¹ mol/L, 10⁻¹⁰ mol/L, 10⁻⁹ mol/L, 10⁻⁸ mol/L, 10⁻⁷ mol/L, 10⁻⁶ mol/L, 10⁻⁵ mol/L, but not without epitope or with an unrelated control peptide epitope. Preferably, this is tested with 10.000 TCR+CD8+ T cells and 20.000-50.000, preferably, 50.000 target cells expressing an appropriate HLA, e.g., with an assay as described below in the examples. Alternatively, or additionally, a TCR may be considered to have "antigenic specificity" for an epitope if T cells expressing the TCR secrete at least twice as much IFN-γ as the untransduced background level of IFN-γ upon co-culture with target cells pulsed with a low concentration of the appropriate peptide. Alternatively, target cells may also express MAGEA1 instead of being pulsed with the epitope in peptide form. Of course, target cells express an appropriate HLA. Such "specificity" as described above can - for example - be analyzed with an ELISA.

A high affinity is correlated with a high peptide sensitivity, as described above for the TCR of the invention, e.g., with a half maximum IFN-γ release with a peptide concentration of 10⁻⁶ mol/L or, preferably, 10⁻⁷ mol/L, 10⁻⁸ mol/L or less. Alternatively, affinity can be analyzed by methods well known to the skilled person, e.g. by BiaCore.

Based on the defined CDR3 and variable region sequences provided by the invention, it is possible to carry out affinity maturation of the TCR sequences (Chervin et al., 2008; Robbins et al., 2008). Non-synonymous nucleotide substitutions, which lead to amino acid exchanges in the CDR3 sequence, may lead to enhanced affinity of the TCR to target antigen. Furthermore, TCR sequence changes in other parts of the variable TRA and TRB regions may change affinity of the TCR to the peptide-MHC complex. This may increase overall affinity of the TCR to the peptide-MHC, but harbors the risk of unspecific recognition and increased cross-reactivity (Linette et al., 2013). It is preferred that TCRs varying from the specific sequences provided retain exclusive specificity for the target antigen provided, i.e., that they are not cross-reactive, most importantly, that they do not have cross-reactivity for human self-peptides. Potential cross-reactivity of TCR can be tested as known in the art, e.g., against known self-peptides loaded on cells with the correct MHC allele (Morgan et al., 2013). Accordingly, it is preferred that adoptive transfer of T cells expressing the TCR construct of the invention has no or significant negative effects on healthy tissue.

The TCR construct according to the invention can also be provided in the form of a multimeric complex, comprising at least two scTCR molecules, wherein said scTCR molecules are each fused to at least one biotin moiety, and wherein said scTCRs are interconnected by biotin-strepavidin interaction to allow the formation of said multimeric complex. Also provided are multimeric complexes of a higher order, comprising more than two, e.g., four, scTCR of the invention.

The TCR construct of the invention can be modified to comprise a detectable label, such as, for instance, a radioisotope, a fluorophore (e.g., fluorescein isothiocyanate (FITC), phycoerythrin (PE)), a tag, such as a HIS-tag, an enzyme (e.g., alkaline phosphatase, horseradish peroxidase), or particles (e.g., gold particles or magnetic particles).

### Host cells

The invention also provides a host cell comprising a nucleic acid and/or protein of the invention. The host cell can be a eukaryotic cell, e.g., plant, animal, fungi, or algae, or can be a prokaryotic cell, e.g., bacteria or protozoa. The host cell can be a cultured cell or a primary cell, i.e., isolated directly from an organism, e.g., a human. The host cell can be an adherent cell or a suspended cell, i.e., a cell that grows in suspension. For purposes of producing a recombinant TCR, polypeptide, or protein, the host cell is preferably a mammalian cell. Most preferably, the host cell is a human cell. While the host cell can be of any cell type, can originate from any type of tissue, and can be of any developmental stage, the host cell preferably is a peripheral blood leukocyte (PBL) or a peripheral blood mononuclear cell (PBMC). More preferably, the host cell is a T cell or T cell precursor, in particular, a human T cell, which may be isolated from PBMC. The T cell can be any T cell, such as a cultured T cell, e.g. a primary T cell, or a T cell from a cultured T cell line, or a T cell obtained from a mammal, preferably, it is a T cell or T cell precursor from a human patient. The T cell can be obtained from numerous sources, such as blood, bone marrow, lymph node, the thymus, or other tissues or fluids. T cells can also be enriched for or purified. It can be, e.g., a tumor infiltrating cell (TIL), effector cell, central effector cell, memory T cell, naive T cell, and the like, preferably a central-memory T cell. Alternatively, the host cell may be another immune effector cell, e.g., an NK cell or a macrophage.

Preferably, in particular, in the context of therepy of a human, the host cell is human, e.g., the T cell is a human T cell. The T cell preferably is a CD8+ cell (e.g., cytotoxic T cell). Preferably, the T cell is a T cell isolated from a human subject, e.g., a human cancer patient, in particular, the patient who is to be treated. Alternatively, the T cell may be from a third-party donor who may be related or unrelated to the patient. Such T cells may be genetically engineered, e.g., in multiple ways. For example, by knock out of the endogenous TCR and/or HLA. T-cells may also be generated from autologous or third-party donor stem cells, wherein, optionally, the stem cells are not human embryonic stem cells.

Preferably, the host cell is a human T cell, preferably, a CD8+ T cell comprising a nucleic acid of the invention which is an expression vector, wherein the nucleic acid encoding the TRA and/or TRB is operably linked to a heterologous promotor, wherein the host cell expresses a TCR construct of the invention. The TCR construct may, e.g., recognize a peptide epitope in the context of HLA-B*44:03. Said nucleic acid may, e.g., encode a TCR5 related TCR construct. The host cell may also be a human CD8+ T cell comprising a nucleic acid of the invention which is an expression vector, wherein the nucleic acid encoding the TRA and/or TRB is operably linked to a heterologous promotor, wherein the host cell expresses a TCR construct of the invention that recognizes the peptide epitope of SEQ ID NO: 7 in the context of HLA-A*03:01, preferably, a TCR24 related constructs.

The invention also provides a host cell expressing two or more different TCR constructs, e.g., two TCR constructs of the invention. Preferably, in this context, the TCR constructs are single chain TCR constructs to avoid mispairing of TCR chains. Said two single chain TCR constructs may be encoded on a single expression vector.

### Pharmaceutical compositions

The invention also provides a pharmaceutical composition comprising
a) a nucleic acid of the invention encoding a TCR construct capable of specifically binding to its respective epitope in the context of the respective MHC-I; or
b) a protein of the invention comprising a TCR construct capable of specifically binding to its respective epitope in the context of the respective MHC-I (i.e., a TCR construct of the invention); or
c) a host cell of the invention expressing a TCR construct capable of specifically binding to its respective epitope in the context of the respective MHC-I, wherein the hostcell preferably is a human T cell, e.g., a CD8+ T cells.

Optionally, the TCR construct is the TCR construct specific for an epitope in the context of HLA-B*44:03, e.g., a TCR construct specific for the epitope of SEQ ID NO: 1.

The TCR construct may alternatively be the TCR construct specific for the epitope of SEQ ID NO: 7 in the context of HLA-A*03:01, e.g., a TCR24 related TCR construct.

The pharmaceutical compositon preferably comprises a host cell of the invention.

The "respective" epitope is the epitope to which the TCR construct can specifically bind in the context of the "respective" MHC-I. The "respective" MHC-I" is the MHC-I to which, in combination with said epitope, the TCR construct binds. For example, for TCR7 related constructs, the "respective" epitope has SEQ ID NO: 1 and the "respective" MHC-I is HLA-B*44:03. For example, for TCR20 related constructs, the "respective" epitope has SEQ ID NO: 6 and the "respective" MHC-I is HLA-A*02:01. For example, for TCR24 related constructs, the "respective" epitope has SEQ ID NO: 7 and the "respective" MHC-I is HLA-A*03:01.

In another embodiment, the invention provides a pharmaceutical composition or a kit comprising at least two pharmaceutical compositions comprising
a) at least two nucleic acids, each encoding a TCR construct capable of specifically binding to its respective epitope in the context of the respective MHC-I; or
b) at least two proteins, each comprising a TCR construct capable of specifically binding to its respective epitope in the context of the respective MHC-I; or
c) at least two host cells, each expressing a TCR construct capable of specifically binding to its respective epitope in the context of the respective MHC-I,
wherein the epitopes are peptides from the same antigen, which are capable of being presented by different MHC-I molecules (a "combination kit or pharmaceutical compositon"). The antigen is preferably specifically expressed by a cancer or infectious agent. Optionally, the antigen is MAGEA1. Preferably, the pharmaceutical composition or a kit comprises at least two host cells.

Advantageously, the kit or the pharmaceutical composition is for use in treatment of said cancer or infectious agent, preferably, for use if treatment of a MAGEA1-associated cancer.

Preferably said combination pharmaceutical composition or kit comprises at least one, optionally, two pharmaceutical composition(s) of the invention comprising a nucleic acid of the invention, a protein of the invention or a host cell of the invention.

Preferably, the combination kit seperately comprises two host cells, such as human CD8+ T cells, each expressing a TCR construct capable of specifically binding to its respective epitope in the context of the respective MHC-I. For example, the first TCR construct in the first host cell may recognize its epitope in the context of HLA-A*03:01, and the epitope may have a sequence of SEQ ID NO: 7. The TCR construct may be a TCR24 related TCR construct, e.g., TCR24. Optionally, in combination with this first TCR, or in combination with a different TCR, the second TCR construct in the second host cell may recognize its epitope in the context of HLA-B*44:03, and the epitope may have a sequence of SEQ ID NO: 1. The TCR construct may, e.g., be a TCR5 related TCR construct, such as TCR5.

A combination kit of the invention may further comprise a different host cell, such as human CD8+ T cell, expressing a TCR construct capable of specifically binding to its respective epitope in the context of the respective MHC-I which differs from the MHC-I in the context of which the first TCR construct of the first host cell recognized its respective epitope. For example, if the first TCR construct in the first host cell recognizes its epitope in the context of HLA-A*03:01, the second TCR construct may recognize an epitope in the context of HLA-B*44:03, HLA-A*02:01, HLA-B*07:02, HLA-B*57:03, HLA-A*03:01, HLA-A*26:01, HLA-C*06:02 or HLA-A*29:02. Optionally, the second TCR construct is a TCR18.1-related, TCR18.2-related, TCR23-related, TCR19-related, TCR1-related, TCR20-related, TCR22-related, TCR5-related, TCR2-related, TCR5-related, TCR6-related, TCR7-related, TCR8-related, TCR56-related, TCR59-related, TCR66-related or TCR4-related TCR construct. However, of course, the second TCR construct may also be a different TCR construct, as long as it also recognized an epitope from the same antigen, e.g., MAGEA1, in the context of a different MHC-I.

The inventors have surprisingly found that it is advantageous to use a combination of at least two TCR constructs expressed by T cells for adoptive T cell therapy, in particular, two or more T cells each expressing a TCR construct capable of specifically binding to its respective epitope in the context of the respective MHC-I.

Without intending to be bound by the theory, it is believed that attacking the cancer based on recognition of different epitopes from the same antigen by different T cells can help to sustain the attack and avoid immue evasion, e.g., through down-regulation of expression of MHC-I groups (e.g. HLA-A's or HLA-B's) or individual HLA alleles (e.g. HLA-A*02:01 or HLA-B*44:03).

It is also possible to target a cancer or infectious agent with three or more TCR constructs, in particular, three or more host cells each comprising a nucleic acid encoding a TCR construct capable of specifically binding to its respective epitope in the context of the respective MHC-I, wherein the epitopes are peptides from the same antigen expressed by the same cancer in the context of different MHC-I.

The kit or pharmaceutical composition is for use in treatment of the cancer (or infectious agent) from which the antigen is derived. In particular, the pharmaceutical composition or the kit comprising the pharmaceutical composition of the invention, wherein the epitopes are MAGEA1-peptides, may be for use in the treatment of a subject having a cancer expressing MAGEA1 selected from the group comprising lung cancer, colorectal cancer, and multiple myeloma. The treated subject advantageously also expresses an MHC-I which, together with the epitope, can be recognized by the TCR construct. If a combination kit or pharmaceutical composition is used, the treated subject advantageously expresses both MHC-I which, together with the respective epitopes, can be recognized by the two TCR constructs.

Adoptive T cell therapy (option c) is preferred throughout the present application, wherein the host cell is a T cell, preferably, a human CD8+ T cell. Said host cell typically comprises a nucleic acid encoding the TCR construct under the control of a heterologous promotor.

However, gene therapy with a nucleic acid of the invention (option a) is also possible, wherein, e.g., a lentiviral vector may be used.

A TCR construct of the invention in protein form (option b) may also be used in therapy, e.g., for targeting a toxin to which it is linked to a cancer, or for targeting to a cancer a bacterial minicell, which may comprise a therapeutic agent such as a toxin. A TCR construct of the invention in protein form may also be used for targeting a diagnostic agent to a cancer. The composition may thus also be a diagnostic composition.

The T cells each expressing its respective TCR construct may be contained in a kit, wherein, separately, each T cell is stored, e.g., in a pharmaceutically acceptable buffer. The components of a kit of the invention may be formulated for simultaneous administration or for administration in any sequence. The components may also be for repeated administration. Tran *et al.* (Tran et al., 2014) describe a possible regimen of administration. Alternatively, they may be mixed before administration and administered together. Alternatively, the T cells each expressing its respective TCR construct may be contained in a single pharmaceutical composition. The same applies for nucleic acids or proteins of the invention.

The pharmaceutical compositions or kits of the invention typically are for intravenous administration. They may further comprise pharmaceutically acceptable carriers such as buffers, e.g., physiological saline or PBS. They may further comprise excipients, such as stabilizers such as SPGA, DMSO, carbohydrates (e.g. sorbitol, mannitol, starch, sucrose, glucose, dextran), proteins such as albumin or casein, or protein containing agents such as bovine serum or skimmed milk.

T cells are typically administered to a patient in a concentration of 1 × 10⁵ - 1 × 10⁹ cells per kg. About 1 × 10⁶ - 2 × 10¹¹ cells may be administered to a patient as a single dose. These parameters may be adapted by the medical practioner, depending, e.g., on the patients age, sex, body weight and medical condition. Protocols disclosed, e.g., in CS Hinrichs et al., J. Clin. Onc. 2017 (Hinrichs et al., 2017) may be adapted to use the host cells of the present invention.

The pharmaceutical composition of the invention or a kit comprising a pharmaceutical composition or a kit of the invention may be for use in the treatment of a patient expressing an MHC in the context of which the respective TCR recognizes the respective epitope, e.g., as disclosed herein. Average HLA (MHC-I) distribution in different populations can be found, e.g., under http://allelefrequencies.net/. It should be tested before treatment if a patient expresses the respective MHC-I.

The subject typically is a patient, e.g., having a cancer, usually, a mammalian patient. The subject may be a mouse, but, preferably, the subject is a human subject.

Pharmaceutical compositions and kits of the present invention may be used in combination with other agents, in particular, with other anti-cancer agents. For example, other anti-cancer agents may be TCR-engineered T cells expressing TCRs specific for other antigens expressed in the MAGEA1-positive tumor (e.g. MAGE-A3, NY-ESO, o. a.), checkpoint inhibitors or other immunotherapies as well as antibodies, small molecule inhibitors or other types of reagents.

One preferred medicinal use of the invention is immune therapy, preferably adoptive T cell therapy. The product and methods of the invention are particularly useful in the context of adoptive T cell therapy. The administration of the compounds of the invention can for example involve the administration, e.g., infusion of T cells of the invention into said patient. Preferably, such T cells are autologous T cells of the patient which were *in vitro* transduced with a nucleic acid of the present invention.

Alternatively, the patient may also be administered a nucleic acid of the invention, in particularly, an expression vector, for *in vivo* transduction of T cells.

Protein TCR constructs of the invention may also, e.g., be used for diagnostic purposes to find out if a subject expresses the respective protein, and, in particular, if the epitope recognized by the TCR construct in the context of the MHC-I is presented. To this end, such constructs are preferably labelled to facilitate detection. Preferably, a patient presenting such an epitope on the respective MHCI is treated by an adoptive T cell therapy of the invention.

The invention also relates to a method of preparing a host cell of the invention, comprising introducing an expression vector encoding a TCR construct of the invention into a suitable host cell, preferably, a human CD8+ T cell isolated from a patient.

Also disclosed is a method of treating a subject having a cancer associated with expression of MAGEA1, e.g., lung cancer, colorectal cancer, or multiple myeloma, comprising administering an effective amount of a pharmaceutical composition or a kit of the invention to said subject.

### Peptides

In one embodiment, the invention also provides specific peptides, which may be for use in peptide vaccination. These peptides are based on the epitopes which can be recognized by the TCR constructs of the invention.

The invention provides a MAGEA1 peptide comprising at most 25 consecutive amino acids identical to the sequence of amino acids ocurring in MAGEA1 of SEQ ID NO: 10 (wt MAGEA1), comprising an epitope capable of being presented by a human MHC-I, wherein the epitope is selected from the group comprising epitopes
a) having at least 88% sequence identity to any of SEQ ID NO: 1, 2 or 3, wherein the epitope is capable of being presented on HLA-B*44:03,
b) having at least 88% sequence identity to SEQ ID NO: 5, wherein the epitope is capable of being presented on HLA-B*57:03 and comprises at most 10 consecutive amino acids identical to the sequence of amino acids ocurring in wt MAGEA1,
c) having at least 88% sequence identity to SEQ ID NO: 7, wherein the epitope is capable of being presented on HLA-A*03:01,
   and
d) having at least 88% sequence identity to SEQ ID NO: 9, wherein the epitope is capable of being presented on HLA-A*29:02,
wherein, preferably, the epitope, when presented by the respective MHC-I, is capable of being specifically recognized by any one of the TCR constructs of the invention, in particular, a TCR construct of the invention disclosed to recognize the epitope having the sequence on which the peptide is based.

The invention also provides a MAGEA1 peptide as defined herein, comprising at most 25, preferably, at most 20 or at most 15, optionally, at most 11 consecutive amino acids identical to the sequence of amino acids ocurring in wt MAGEA1, comprising an epitope capable of being presented by a human MHC-I, wherein the epitope is selected from the group comprising epitopes
a) of SEQ ID NO: 1, 2 or 3,
b) of SEQ ID NO: 5, wherein the epitope comprises at most 10 consecutive amino acids identical to the sequence of amino acids ocurring in wt MAGEA1,
c) of SEQ ID NO: 7, and
d) of SEQ ID NO: 9.

Optionally, the peptide consists of a peptide of SEQ ID NO: 1, 2, 3, 5, 7, or 9.

The invention further provides a MAGEA1 peptide as defined herein, comprising at most 20, preferably, at most 15, or at most 11 consecutive amino acids identical to the sequence of amino acids ocurring in wt MAGEA1, comprising an epitope capable of being presented by a human MHC-I, wherein the epitope is selected from the group comprising epitopes
a) having at least 88% sequence identity to any of SEQ ID NO: 1, 2 or 3, wherein the epitope is capable of being presented on HLA-B*44:03,
b) having at least 88% sequence identity to SEQ ID NO: 5, wherein the epitope is capable of being presented on HLA-B*57:03 and comprises at most 10 consecutive amino acids identical to the sequence of amino acids ocurring in wt MAGEA1,
c) having at least 88% sequence identity to SEQ ID NO: 7, wherein the epitope is capable of being presented on HLA-A*03:01,
   and
d) having at least 88% sequence identity to SEQ ID NO: 9, wherein the epitope is capable of being presented on HLA-A*29:02.

The invention also provides a MAGEA1 peptide as defined herein, comprising at most 20, preferably, at most 15, or at most 11 consecutive amino acids identical to the sequence of amino acids ocurring in wt MAGEA1, comprising an epitope capable of being presented by a human MHC-I, wherein the epitope is selected from the group comprising epitopes
a) of SEQ ID NO: 1, 2 or 3,
b) of SEQ ID NO: 5, wherein the epitope comprises at most 10 consecutive amino acids identical to the sequence of amino acids ocurring in wt MAGEA1,
c) of SEQ ID NO: 7, and
d) of SEQ ID NO: 9.

A pharmaceutical composition comprising a MAGEA1 peptide as defined above is also provided by the present invention. Preferably, said pharmaceutical composition is for use in vaccinating a subject expressing said MHC-I on which the epitope is capable of being presented.

Additonally, the invention provides a pharmaceutical composition comprising a MAGEA1 peptide comprising at most 25, optionally, at most 20 or at most 15, preferably, at most 11 consecutive amino acids identical to the sequence of amino acids ocurring in wt MAGEA1, comprising an epitope capable of being presented by a human MHC-I, wherein the epitope is selected from the group comprising epitopes
f) having at least 88%, e.g., 100%, sequence identity to SEQ ID NO: 4, wherein the epitope is capable of being presented on HLA-B*07:02, and
g) having at least 88%, e.e., 100% sequence identity to SEQ ID NO: 8, wherein the epitope is capable of being presented on HLA-A*26:01,
wherein, preferably, said pharmaceutical composition is for use in vaccinating a subject expressing said MHC-I on which the epitope is capable of being presented.

Also disclosed is a method of vaccination of a subject, comprising administering the pharmaceutical composition of the invention to said subject, wherein said subject preferably expresses said MHC-I on which the epitope is capable of being presented. Optionally, said subject has a cancer associated with expression of MAGE-A1, e.g., lung cancer, colorectal cancer, or multiple myeloma.

The present invention is further illustrated in the following examples with reference to the accompanying figures and sequences, nevertheless, without being limited thereto. For the purposes of the present invention, all references as cited herein are incorporated by reference in their entirety.

### Examples

### 1) Generation of MAGEA1-specific TCR constructs and transgenic T cells

Through an innovative method (Lorenz et al., 2017; WO2016146618A1) 17 MAGEA1-reactive TCRs recognizing endogenously processed and immunogenic MAGEA1 peptides presented in the context of 9 different HLA alleles were identified.

In brief, electroporation of full-length MAGEA1 ivtRNA into dendritic cells (DCs) enabled the endogenous translation and processing of the antigen, resembling the natural situation. MAGEA1 peptides that have been cleaved by the proteasome, transported to the endoplasmatic reticulum (ER) via TAP and chopped by ERAP proteases were loaded onto their preferred MHC-I complex and shuttled to the surface of the DCs.

Antigen-specific T cells were generated by isolating naïve CD8+ T cells from healthy donors and stimulating them with antigen-expressing mature DCs. This led to proliferation of T cells recognizing immunogenic peptide-MHC (pMHC) complexes at the surface of DCs in a completely unbiased manner as certain MHC alleles were not preferentially selected for T cell stimulation. After 4 weeks of cultivation with an intermittent re-stimulation, outgrown T cells were collected.

The generated T cells were screened for antigen-MHC specificity. For this, a K562-based cell library, consisting of single MHC-I allele-expressing cells (K562-HLA) was used. Six K562-HLA cells were chosen to match each of the six HLA alleles of the original T cell donor. Those K562-HLA cells were transfected to express full-length MAGEA1. Stimulated T cells and the different K562-HLA target cells were co-cultured to analyze which antigen-HLA combination is recognized by the T cells. As read-out, IFNγ secretion was detected using ELISA, and CD137 expression on T cells was assessed using flow cytometry. T cells responding to a specific antigen-HLA combination were FACS-sorted depending on CD137 expression.

Next, RNA was extracted from sorted T cells and a TCR alpha- and TCR beta-specific RACE-PCR was performed. PCR products were used for next generation sequencing to identify individual TCR chains and their frequency within the sample.

TCR chains that were found with a frequency of at least 5% were cloned into the γ-retrovira! expression vector MP71 (Engels et al., 2003; Leisegang et al., 2008; Sommermeyer and Uckert, 2010). Combinations of MP71 vectors carrying different TCR alpha and beta chains were used to transduce primary human T cells from PBMCs, which were then stimulated with K562-HLA cells with or without MAGEA1. T cells transduced with the correct TCR alpha and beta chain combination produced IFNγ upon recognition of K562 cells carrying the correct HLA allele and expressing MAGEA1.

To this point, knowledge of the exact MAGEA1 epitope is not required. The method used herein takes 1) endogenous processing, 2) preferred HLA alleles and 3) immunogenicity (recognition by TCRs) into account. These factors are key for successful identification of peptides that serve as high quality epitopes for TCR targeting.

Newly identified TCR alpha and beta chains were linked with a P2A genetic element in a TCR beta-P2A-TCR alpha configuration and cloned into the MP71 vector. Linkage via the P2A element facilitates stochiometric expression of both chains from one plasmid and thus, increases transduction rates. Furthermore, constant TCR alpha and constant TCR beta regions were replaced by their murine counterparts, which additionally enhances pairing and surface expression. The whole TCR construct was subsequently codon-optimized.

Figure 1A-C shows the transduction rate of human PBMCs with the optimized TCR construct compared to untransduced PBMCs. Transduction rates in this example were 8.68% - 14.4%, respectively.

### 2) Identification of epitopes

Primary human T cells isolated from PBMC were transduced with the respective TCR and used to identify immunogenic MAGEA1 epitopes. To map the exact epitope, truncated versions of the full-length MAGEA1 gene were designed which differ by 102 nt +/- 6 nt. Truncated constructs were then cloned into the pcDNA3.1(-) expression vector and used to transfect K562-HLA target cells. These cells were used for stimulation of the TCR-transduced T cells. The recognition allowed for an identification of a region from which the epitope is derived.

For example, TCR1-transduced T cells were able to recognize K562-HLA-B*57:03 cells, expressing MAGEA1_1-411, but not MAGEA1_1-309. This indicates that the epitope must be expressed from the gene region spanning from base pair 282-411 assuming that the first 10-mer peptide from MAGEA1_1-411 is expressed from the gene region 282-312. This corresponds to amino acid (aa) region 94-137 (Figure 2A).

Candidate peptides from the identified protein region were determined using the epitope prediction algorithm netMHCpan4.0 (http://www.cbs.dtu.dk/services/NetMHCpan/) for the respective MHC-I, e.g., for TCR1, HLA-B*57:03. Predicted peptides were generated and, loaded on K562 cells transfected with the respective MHC-I, used for stimulation of TCR-transduced T cells. The peptides capable of stimulating most IFNγ production are generally considered the cognate epitope.

Figure 2B exemplarily shows a stimulation of TCR1-transduced T cells with MAGEA1 peptides predicted by NetMHCpan4.0 in the context of HLA-B*57:03. Predicted peptides from protein region 94-137 were used for further analysis. For TCR1, a set of 10 MAGEA1 peptides was loaded on K562-HLA-B*57:03 transgenic cells. These cells were used to stimulate TCR1-transduced T cells. Figure 2B shows that TCR1-transduced T cells released the highest amounts of IFNγ in response to the 9-mer peptide KVADLVGFL (SEQ ID NO: 5) indicating that this peptide serves as the cognate epitope for TCR1. KVADLVGFL was ranked #19 by the current state-of-the-art epitope prediction algorithm netMHCpan4.0, indicating that a number of additional fectors other than binding affinity influence presentation and immunogenicity of the antigenic peptide.

In analogy to Figure 2 Figures 3-14 show A) mapping of the MAGEA1 gene region expressing the peptide recognized by the TCRs, B) epitope mapping using epitopes predicted in the respective gene region by netMHCpan4.0 (if existing), using peptide-pulsed K562 cells expressing the HLA allele recognized by each TCR, wherein, for most TCR except TCR1 and TCR7, only stimulation with peptides evoking a significant response is shown in the graph, and DC) a peptide titration using peptide-pulsed K562 cells expressing the HLA allele recognized by each TCR (for TCR4, TCR7, TCR8, TCR20, TCR22 and TCR24). K562 cells transgenic for the HLA allele restricting the tested TCR were pulsed with titrated amounts of different peptides that were recognized by TCR-transduced PBMCs in the epitope mapping assay (BD). The peptide recognized by TCR-transduced PBMCs at concentrations lower than the other tested peptides was defined as cognate MAGEA1 epitope of the respective TCR.

It is noteworthy that the epitopes recognized, e.g., by TCR1, TCR2, TCR7 and TCR8 are ranked at position 19 or lower in the netMHCpan4.0 prediction file. This clearly indicates that epitope prediction algorithms are not accurate tools to determine immunogenic epitopes, while the epitopes provided in the present invention are functional immunogenic epitopes presented in a physiological setting.

The peptide set presented herein has unique features, which are key for successful immunotherapy of MAGEA1-positive tumors. Namely, 1) the peptides are endogenously processed, 2) the preferred immunodominant HLA is known and 3) the peptides are immunogenic as they are able to induce a TCR-triggered T cell response. Many of the peptides were not predicted accurately by an epitope prediction algorithm (netMHCpan4.0). Epitope prediction algorithms enable prediction of binding affinity of a given peptide to an MHC allele. However, processing, presentation and immunogenicity cannot be assess accurately using currently available software. Studies have shown that >95% of predicted epitopes are not found at the cell surface of tumor cells (Bassani-Sternberg et al., 2016) (Abelin et al., 2017). In addition, it remains unclear which HLA alleles are best suited to present peptides of a given antigen. Finally, surface expression of epitopes does not imply that a pMHC complex is immunogenic and thus, recognized by a TCR.

It is critical for successful immunotherapy to precisely define which of the huge number of potential peptides of MAGEA1 are epitopes that can be targeted by immunotherapy.

TCR constructs generated in the context of the invention are characterized in the tables below.

**Table 1: TCR summary with core epitopes recognized by the TCR constructs**

| TCR | MHC-I | Epitope | SEQ ID NO of epitope: |
|---|---|---|---|
| 7 | B*44:03 | LAETSYVKVL | 1 |
| 8 | B*44:03 | EEGPSTSCI | 2 |
| 6 | B*44:03 | KVADLVGFLL | 3 |
| 5 | B*44:03 | LAETSYVKVL | 1 |
| 18.1 | B*07:02 | RVRFFFPSL | 4 |
| 18.2 | B*07:02 | RVRFFFPSL | 4 |
| 23 | B*07:02 | RVRFFFPSL | 4 |
| 19 | B*07:02 | RVRFFFPSL | 4 |
| 1 | B*57:03 | KVADLVGFL | 5 |
| 20 | A*02:01 | KVLEYVIKV | 6 |
| 22 | A*03:01 | SLFRAVITK | 7 |
| 24 | A*03:01 | SLFRAVITK | 7 |
| 2 | A*26:01 | EVYDGREHSA | 8 |
| 4 | A*29:02 | LVGFLLLKY | 9 |
| 66 | C*06:02 | SAYGEPRKL | 201 |

The inventors have shown that some TCR constucts further specifically recognize peptides one amino acid longer or shorter than the optimal epitopes, or peptides shifted by one position in the wt MAGEA1 sequence. For example, TCR1-related constructs specifically recognize KVADLVGFL (SEQ ID NO: 5), but further recognize VADLVGFLL (SEQ ID NO: 151). For example, TCR4-related constructs specifically recognize LVGFLLLKY (SEQ ID NO: 9), but further recognize DLVGFLLLKY (SEQ ID NO: 152). For example, TCR6-related constructs specifically recognize KVADLVGFLL (SEQ ID NO: 3), but further recognize VADLVGFLL (SEQ ID NO: 151) or ADLVGFLLLK (SEQ ID NO: 157). For example, TCR18.1, TCR18.2, TCR19 and TCR23-related constructs specifically recognize RVRFFFPSL (SEQ ID NO: 4), but further recognize ARVRFFFPSL (SEQ ID NO: 162) or RVRFFFPSLR (SEQ ID NO: 163). For example, TCR20-related constructs specifically recognize KVLEYVIKV (SEQ ID NO: 6), but further recognize VKVLEYVIKV (SEQ ID NO: 164), or KVLEYVIKVS (SEQ ID NO: 165). For example, TCR22 or TCR24-related constructs specifically recognize SLFRAVITK (SEQ ID NO: 7), but further recognize SLFRAVITKK (SEQ ID NO: 166), ESLFRAVITK (SEQ ID NO: 167) or LFRAVITKK (SEQ ID NO: 168). Of course, the recognition is in combination with (or in complex with, which is used exchangeably herein) the respective MHC-I.

**Table 2: CDR sequences of preferred TCR constructs of the invention. Numbers in parentheses: SEQ ID NO:**

| **TCR** | | **CDR-1 aa seq** | **CDR-2 aa seq** | **CDR-3 aa seq** |
|---|---|---|---|---|
| TCR 7 | TRA | SIFNT (11) | LYKAGEL (12) | CAALYGNKLVF (13) |
| | TRB | SGHRS (16) | YFSETQ (17) | CASSSMGANVLTF (18) |
| TCR 8 | TRA | TSGFNG (21) | NVLDGL (22) | CAPSSNTGKLIF (23) |
| | TRB | SGHNS (26) | FNNNVP (27) | CASSLPQETQYF (28) |
| TCR 6 | TRA | DRGSQS (31) | IYSNGD (32) | CAVSSNTGKLIF (33) |
| | TRB | SGHNS (36) | FNNNVP (37) | CASKWGQGAGDTQYF (38) |
| TCR 5 | TRA | SIFNT (41) | LYKAGEL (42) | CAGLRDNYGQNFVF (43) |
| | TRB | LNHDA (46) | SQIVND (47) | CASRVGGFEGYTF (48) |
| TCR 18.1 | TRA | TTLSN (51) | LVKSGEV (52) | CANRDNYGQNFVF (53) |
| | TRB | MNHEY (56) | SMNVEV (57) | CASSRTSGISYEQYF (58) |
| TCR 18.2 | TRA | TTLSN (61) | LVKSGEV (62) | CANRDNYGQNFVF (63) |
| | TRB | MNHEY (66) | SMNVEV (67) | CASTRTGTLPEAFF (68) |
| TCR 23 | TRA | TRDTTYY (71) | RNSFDEQN (72) | CALSVTYTGNQFYF (73) |
| | TRB | SGDLS (76) | YYNGEE (77) | CASSVQGQGTDTQYF (78) |
| TCR 19 | TRA | TSENNYY (81) | QEAYKQQN (82) | CAFISGYSTLTF (83) |
| | TRB | MNHEY (86) | SMNVEV (87) | CASRTSHPQTYEQYF (88) |
| TCR 1 | TRA | ATGYPS (91) | ATKADDK (92) | CALSGGGSYIPTF (93) |
| | TRB | SGHAT (96) | FQNNGV (97) | CASSSPGQLNTEAFF (98) |
| TCR 20 | TRA | DRGSQS (101) | IYSNGD (102) | CAVNAPRPGYSTLTF (103) |
| | TRB | MDHEN (106) | SYDVKM (107) | CASSLSMNTEAFF (108) |
| TCR 22 | TRA | TSINN (111) | IRSNERE (112) | CATDEGGAQKLVF (113) |
| | TRB | MDHEN (116) | SYDVKM (117) | CATVGRGEDRGEQFF (118) |
| TCR 24 | TRA | VSPFSN (121) | MTFSENT (122) | CVVSNNAGNMLTF (123) |
| | TRB | LNHNV (126) | YYDKDF (127) | CATSRESLGRDEQFF (128) |
| TCR 2 | TRA | TSDPSYG (131) | QGSYDQQN (132) | CASREGSARQLTF (133) |
| | TRB | MDHEN (136) | SYDVKM (137) | CASDSADTQYF (138) |
| TCR 4 | TRA | SSVPPY (141) | YTSAATLV (142) | CAVSNSGNTPLVF (143) |
| | TRB | PRHDT (146) | FYEKMQ (147) | CASSSTTGDGYTF (148) |
| TCR56 | TRA | TSESDYY (171) | QEAYKQQN (172) | AYCRGSNFGNEKLT (173) |
| | TRB | MNHEY (176) | SMNVEV (177) | ASSLGTTNTGELF (178) |
| TCR59 | TRA | TSINN (181) | IRSNERE (182) | ATDPTGTASKLT (183) |
| | TRB | SEHNR (186) | FQNEAQ (187) | ASSSGPYNEQF (188) |
| TCR66 | TRA | TRDTTYY (191) | RNSFDEQN (192) | ALSEFRDDKII (193) |
| | TRB | GTSNPN (196) | SVGIG (197) | AWSVASNTEAF (198) |

**Table 3 Variable segments of preferred TCR constructs of the invention**

| SEQ ID Nos for CDR3 cf. Table 2. | | | | | | |
|---|---|---|---|---|---|---|
| TCR | TRAV | CDR3 | TRAJ | TRBV | CDR3 | TRBJ |
| TCR1 | TRAV9-2^{∗}01 | CALSGG GSYIPTF | TRAJ6^{∗}01 | TRBV11-2^{∗}01 | CASSSPGQ LNTEAFF | TRBJ1-1^{∗}01 |
| TCR2 | TRAV14/D V4^{∗}02 | CASREG SARQLT F | TRAJ22^{∗}01 | TRBV28^{∗} 01 | CASDSADT QYF | TRBJ2-3^{∗}01 |
| TCR4 | TRAV8-4^{∗}01 | CAVSNS GNTPLV F | TRAJ29^{∗}01 | TRBV13^{∗} 01 | CASSSTTG DGYTF | TRBJ1-2^{∗}01 |
| TCR5 | TRAV35^{∗}0 1 | CAGLRD NYGQNF VF | TRAJ26^{∗}01 | TRBV19^{∗} 01 | CASRVGGF EGYTF | TRBJ1-2^{∗}01 |
| TCR6 | TRAV12-2^{∗}01 | CAVSSN TGKLIF | TRAJ27^{∗}02 | TRBV12-3^{∗}01 | CASKWGQ GAGDTQY F | TRBJ2-3^{∗}01 |
| TCR7 | TRAV35^{∗}0 1 | CAALYG NKLVF | TRAJ47^{∗}01 | TRBV5-1^{∗}01 | CASSSMGA NVLTF | TRBJ2-6^{∗}01 |
| TCR8 | TRAV1-2^{∗}01 | CAPSSNT GKLIF | TRAJ37^{∗}02 | TRBV12-3^{∗}01 | CASSLPQE TQYF | TRBJ2-5^{∗}01 |
| TCR18. 1 | TRAV25^{∗}0 1 | CANRDN YGQNFV F | TRAJ26^{∗}01 | TRBV27^{∗} 01 | CASSRTSGI SYEQYF | TRBJ2-7^{∗}01 |
| TCR18. 2 | TRAV25^{∗}0 1 | CANRDN YGQNFV F | TRAJ26^{∗}01 | TRBV27^{∗} 01 | CASTRTGT LPEAFF | TRBJ1-1^{∗}01 |
| TCR19 | TRAV38-1^{∗}01 | CAFISGY STLTF | TRAJ11^{∗}01 | TRBV27^{∗} 01 | CASRTSHP QTYEQYF | TRBJ2-7^{∗}01 |
| TCR20 | TRAV12-2^{∗}01 | CAVNAP RPGYST LTF | TRAJ11^{∗}01 | TRBV28^{∗} 01 | CASSLSMN TEAFF | TRBJ1-1^{∗}01 |
| TCR22 | TRAV17^{∗}0 1 | CATDEG GAQKLV F | TRAJ54^{∗}01 | TRBV28^{∗} 01 | CATVGRGE DRGEQFF | TRBJ2-1^{∗}01 |
| TCR23 | TRAV19^{∗}0 1 | CALSVT YTGNQF YF | TRAJ49^{∗}01 | TRBV9^{∗}0 1 | CASSVQGQ GTDTQYF | TRBJ2-3^{∗}01 |
| TCR24 | TRAV10^{∗}0 1 | CVVSNN AGNMLT F | TRAJ39^{∗}01 | TRBV15^{∗} 01 | CATSRESL GRDEQFF | TRBJ2-1^{∗}01 |
| TCR56 | TRAV38-1^{∗}04 | AYCRGSN FGNEKLT | TRAJ48^{∗}01 | TRBV27^{∗} 01 | ASSLGTTNT GELF | TRBJ2-2^{∗}01 |
| TCR59 | TRAV17^{∗}0 1 F | ATDPTGT ASKLT | TRAJ44^{∗}01 | TRBV7-9^{∗}03 | ASSSGPYNE QF | TRBJ2-1^{∗}01 |
| TCR66 | TRAV19^{∗}0 1 | ALSEFRD DKII | TRAJ30^{∗}01 | TRBV30^{∗} 05 | AWSVASNT EAF | TRBJ1-1^{∗}01 |

### Functional characterization

The generated TCR-engineered T cells were functionally characterized using *in vitro* assays.

Functional TCRs were selected for recognition of immunodominant pMHC combinations without prior knowledge of epitope specificity. Briefly: To map the exact epitope sequence recognized by the different TCRs, 3'-truncated minigenes of the MAGEA1 gene were designed, which differ in their length by 102 nt (+3/-9 nt). 3'-truncated minigenes were subsequently cloned into the expression plasmid pcDNA3.1(-). 20 µg of each plasmid was transfected into K562 cells, also expressing the respective corresponding MHC-I alleles (A*02:01, A*03:01; A*26:01, A*29:01, B*07:02, B*44:03, B*57:03, C*06:02), 48 hours prior to a co-culture assay with the TCR-engineered T cells. Effector cell to target cell ratio was 2:1, meaning 5×10⁴ TCR-engineered T cells were co-cultured with 2.5 × 10⁴ antigen-presenting K562 cells. The ability of TCR-transduced T cells to recognize their target cells was assessed 24h later by an IFNγ ELISA. Stimulation with phorbol myristate acetate (PMA) and ionomycin served as positive control.

After identification of the gene region where the putative epitope is encoded, NetMHCpan 4.0 server (http://www.cbs.dtu.dk/services/NetMHCpan/) was applied to predict possible epitopes for each TCR in combination with its respective MHC-I restriction. Predicted peptides were then ordered from JPT Peptide Technologies, Berlin. For testing, peptides were loaded onto K562 target cells and a co-culture assay was performed in combination with TCR-engineered T cells. The ability of TCR-transduced T cells to recognize their target epitope was assessed 24h later by an IFNγ ELISA. Endogenously processed MAGEA1 and stimulation with phorbol myristate acetate (PMA) and ionomycin served as positive control.

The peptide sensitivity of the isolated TCRs was analyzed in peptide titration experiments. To this end, peptides were loaded in titrating amounts onto K562 target cells, ranging from 10⁻⁵ mol/L to 10⁻¹³ mol/L. A co-culture assay was performed in combination with TCR-engineered T cells. Effector cell to target cell ratio was 2:1, meaning 5×10⁴ TCR-engineered T cells were co-cultured with 2.5 × 10⁴ peptide loaded K562 cells. The ability of TCR-transduced T cells to recognize their target epitope was assessed 24h later by an IFNγ ELISA.

According to the described isolation and characterization procedure and the identification of the epitopes recognized by TCR-engineered T cells, we have isolated in total seventeen MAGEA1-specific TCRs, and the relevant, immunogenic peptides (epitopes) recognized by these TCRs. Some of the epitopes wer already known in the art by other MHC molecules, other, in particular, peptides of SEQ ID NO: 1, 2, 3, 5, 6, 7 and 9 have been newly identified herein to be presented by MHC molecules.

### Combinations of TCR

For therapeutic approaches, two different TCRs, which target the same tumor-antigen, but are restricted to distinct MHC-I molecules, may be combined. In this way, tumor immunevasion due to downregulation of a specific MHC-I, may be prevented and the anti-tumor effect of adoptive T cell therapy may be enhanced.

The enhancing effect of a combinatorial TCR therapy is tested in an *in vitro* experiment as follows: First, two TCRs with distinct MHC-I restrictions are chosen, e.g. TCR7, restricted to MHC-B*44:03 and TCR20, restricted to MHC-A*02:01. Both recognize an endogenously processed MAGEA1 epitope. TCR-engineered T cells are generated with these two TCRs, as done already before. Second, tumor cell lines are chosen that express MAGEA1 and the respective MHC-I molecules, B*44:03 and A*02:01.

TCR-engineered T cells, expressing TCR7 and TCR20, are mixed in equal amounts, e.g. 2.5×10⁴ + 2.5×10⁴, and co-cultured together with the tumor cell lines. Effector to target cell ratio is 2:1. For comparison, co-cultures are set-up with solely TCR7- or TCR20-engineered T cells and the respective tumor cell lines. Upon antigen recognition on the tumor cell lines, the TCR-engineered T cells secrete IFNγ, which can be detected by an IFNγ ELISA, e.g., 24h later.

### Cytotoxicity assay

To further assess the functional characteristics of the TCR constructs, the 14 best-performing TCRs restricted to six different HLA alleles (A*02:01, A*03:01, B*07:02, B*44:03, B*57:01, C*06:02) were analyzed. Together, these HLAs cover more than 80% of the patient population carrying MAGEA1-positive (MAGEA1+) tumors.

An in vitro luciferase-based kill assay was established to enable lead TCR identification across different target epitopes and HLAs. All TCRs used in the analysis recognized endogenously processed and presented immunodominant epitopes. Following this, tumor cell line recognition using naturally MAGEA1+ U266B1 cells was executed for the best performing candidates on each HLA. Most TCR, and all of the six lead TCRs showed strong killing along with excellent tumor cell line recognition.

To identify the best-performing TCRs, a two-step approach was taken, using a killing assay and a tumor cell recognition assay. Table 4 shows the cell lines used in this study:

**Table 4**

| **Name** | **Origin** | **Assay** | **HLA allele (expression in rpkm1)** | **MAGE-A1 status (expression in rpkm¹)** | **Reference** |
|---|---|---|---|---|---|
| K562 | DSMZ | Kill assay | No endogenous HLA | Transduced with full-length MAGE-A1 and luciferase | Lorenz et al. 2017 and WO2016146618A1 |
| U266B1 | ATCC | Tumor cell line recognition | **A*02:01** / **A*03:01** (44) **B*07:02** / B*40:01 (133) C*03:04 / C*07:02 (133) | Naturally positive for MAGE-A1 (178.7) | TIB-196 |
| L363 | DSMZ | Tumor cell line recognition | **A*02:01** / A*31:01 (23) **B*07:02** / B*40:01 (46) C*03:04 / C*07:02 (60) | Naturally positive for MAGE-A1 (55.3) | ACC 49 |

| | | | | | |
|---|---|---|---|---|---|
| ¹rpkm (reads per kilobase of transcript per million mapped reads) based on TRON Cell Line Portal - http://celllines.tron-mainz.de | | | | | |

### Kill assay

For the kill assay, K562 cells expressing the respective single HLAs as well as firefly luciferase were engineered to express full-length MAGEA1. These cells were then co-cultured with T cells expressing the different TCRs to determine their cytolytic activity. As K562-HLA-MAGEA1 cells express full-length MAGEA1, killing capacity of TCR-T cells targeting MAGEA1 via the same HLA can be studied and compared even if TCRs recognize different epitopes of MAGEA1. Using the kill assay, the candidate TCRs that have the highest killing were determined capacity as determined by the EC50 kill value.

Briefly, in the killing assay, T cells or untransduced T cells were cultured at different effector-to-target (E:T) ratios with K562-HLA-MAGEA1 cells. After an overnight co-culture, the live K562-HLA-MAGEA1 tumor cells were detected via their luciferase signal. Specific killing was determined as activity of TCRT cells compared to non-engineered T cells. For the panel of 14 MAGEA1-specific TCR-T cells, we observed robust cytolytic activity. TCR-T cells were able to efficiently kill the different K562-HLAMAGEA1 target cells (engineered to express the respective HLA, full-length MAGEA1 and firefly luciferase) at low effector to target (E:T) ratios (Fig. 17). Note: Killing capacity may not be compared quantitatively between TCRs targeting different HLAs as expression of HLA and antigen may vary between different K562-HLA-MAGE-A1 cell lines. However, EC50 values can serve as a rough estimate to compare killing capacity of TCRs targeting MAGE-A1 via different HLAs. The EC50 killing value as well as maximum killing capacity are shown in Table 5.

**Table 5 Antigen-specific cytolytic activity of CD8+ T cells depicted as tabular overview for each HLA**

| **TCR** | **HLA** | **EC50 killing capacity** | **Maximum killing** |
|---|---|---|---|
| **TCR20** | A*02:01 | 0.8 | 80% |
| **TCR22** | A*03:01 | n.a. | 60% |
| **TCR24** | A*03:01 | 1.3 | 70% |
| **TCR18.1** | B*07:02 | n.a. | 55% |
| **TCR18.2** | B*07:02 | 20.0 | 70% |
| **TCR19** | B*07:02 | 1.3 | 80% |
| **TCR23** | B*07:02 | n.a. | 55% |
| **TCR5** | B*44:03 | 0.4 | 80% |
| **TCR6** | B*44:03 | 1.4 | 80% |
| **TCR7** | B*44:03 | n.a. | 60% |
| **TCR8** | B*44:03 | n.a. | 40% |
| **TCR59** | B*44:03 | 1.1 | 80% |
| **TCR1** | B*57:01 | 1.7 | 85% |
| **TCR66** | C*06:02 | 1.1 | 100% |

| | | | |
|---|---|---|---|
| EC50 killing capacity: effector:target cell (E:T) ratio at which 50% of target cells were killed by TCR-T cells; maximum killing: Maximum percentage of targets killed by TCR-T cells at an E:T ratio of 64:1; n.a., not available because no sigmoidal curve could be determined and thus, EC50 could not be calculated | | | |

### Tumor recognition of cell lines

Recognition of tumor cells that endogenously process and present epitopes is crucial for efficacious TCR-T cell therapy in patients. Here, we analyzed the ability of the MAGEA1-specific lead TCR-T cells to recognize the tumor line U266B1. U266B1 cells naturally express MAGE-A1 as well as HLA-A*02:01, A*03:01 and B*07:02, which makes the U266B1 cell line an ideal target. HLAs B*44:03, B*57:01 and C*06:02 are not expressed by U266B1. For these HLAs, we transduced the MAGE-A1 expressing cell lines U266B1 and L363 with the respective HLAs. As beta-microglobulin expression is the rate-limiting factor for surface HLA expression, overexpression of transgenic HLAs was not assumed.

T cells carrying TCR20 (A*02:01), TCR24 (A*03:01), TCR19 (B*07:02), TCR5 (B*44:03), TCR1 (B*57:01) and TCR66 (C*06:02) specifically released cytotoxic cytokine IFNγ when co-cultured with MAGEA1+ and HLA+ tumor cells. We observed varying levels of release of the cytotoxic cytokine IFNγ for the different TCRs, which may be due to T cells from different donors as well as different HLA expression levels on the tumor cell lines (Table 4). Taken together, these results show that T cells carrying one or more of the TCR or the invention, e.g., one or more of the 6 lead TCRs, which recognize immunodominant MAGE-A1 epitopes over different HLAs, may be candidates for the effective targeting of MAGE-A1+ tumors in patients.

### References

Abelin, J.G., Keskin, D.B., Sarkizova, S., Hartigan, C.R., Zhang, W., Sidney, J., Stevens, J., Lane, W., Zhang, G.L., Eisenhaure, T.M., et al. (2017). Mass Spectrometry Profiling of HLA-Associated Peptidomes in Mono-allelic Cells Enables More Accurate Epitope Prediction. Immunity 46, 315-326.
Bassani-Sternberg, M., Braunlein, E., Klar, R., Engleitner, T., Sinitcyn, P., Audehm, S., Straub, M., Weber, J., Slotta-Huspenina, J., Specht, K., et al. (2016). Direct identification of clinically relevant neoepitopes presented on native human melanoma tissue by mass spectrometry. Nature communications 7, 13404.
Celis, E., Fikes, J., Wentworth, P., Sidney, J., Southwood, S., Maewal, A., Del Guercio, M.F., Sette, A., and Livingston, B. (1994). Identification of potential CTL epitopes of tumor-associated antigen MAGE-1 for five common HLA-A alleles. Molecular immunology 31, 1423-1430.
Chaux, P., Luiten, R., Demotte, N., Vantomme, V., Stroobant, V., Traversari, C., Russo, V., Schultz, E., Cornelis, G.R., Boon, T., and van der Bruggen, P. (1999). Identification of five MAGE-A1 epitopes recognized by cytolytic T lymphocytes obtained by in vitro stimulation with dendritic cells transduced with MAGE-A1. Journal of immunology 163, 2928-2936.
Chervin, A.S., Aggen, D.H., Raseman, J.M., and Kranz, D.M. (2008). Engineering higher affinity T cell receptors using a T cell display system. Journal of immunological methods 339, 175-184. Corbiere, V., Nicolay, H., Russo, V., Stroobant, V., Brichard, V., Boon, T., and van der Bruggen, P. (2004). Identification of a MAGE-1 peptide recognized by cytolytic T lymphocytes on HLA-B*5701 tumors. Tissue antigens 63, 453-457.
Danska, J.S., Livingstone, A.M., Paragas, V., Ishihara, T., and Fathman, C.G. (1990). The presumptive CDR3 regions of both T cell receptor alpha and beta chains determine T cell specificity for myoglobin peptides. The Journal of experimental medicine 172, 27-33.
Engels, B., Cam, H., Schuler, T., Indraccolo, S., Gladow, M., Baum, C., Blankenstein, T., and Uckert, W. (2003). Retroviral vectors for high-level transgene expression in T lymphocytes. Human gene therapy 14, 1155-1168.
Garcia, K.C., and Adams, E.J. (2005). How the T cell receptor sees antigen--a structural view. Cell 122, 333-336.
Goodyear, O.C., Pearce, H., Pratt, G., and Moss, P. (2011). Dominant responses with conservation of T-cell receptor usage in the CD8+ T-cell recognition of a cancer testis antigen peptide presented through HLA-Cw7 in patients with multiple myeloma. Cancer immunology, immunotherapy : CII 60, 1751-1761.
Hinrichs, C.S., Doran, S.L., Stevanovic, S., Adhikary, S., Mojadidi, M., Kwong, M.L., Faquin, W.C., Feldman, S., Somerville, R., Sherry, R.M., et al. (2017). A phase I/II clinical trial of E6 T-cell receptor gene therapy for human papillomavirus (HPV)-associated epithelial cancers. Journal of Clinical Oncology 35, 3009-3009.
Leisegang, M., Engels, B., Meyerhuber, P., Kieback, E., Sommermeyer, D., Xue, S.A., Reuss, S., Stauss, H., and Uckert, W. (2008). Enhanced functionality of T cell receptor-redirected T cells is defined by the transgene cassette. Journal of molecular medicine 86, 573-583.
Linette, G.P., Stadtmauer, E.A., Maus, M.V., Rapoport, A.P., Levine, B.L., Emery, L., Litzky, L., Bagg, A., Carreno, B.M., Cimino, P.J., et al. (2013). Cardiovascular toxicity and titin crossreactivity of affinity-enhanced T cells in myeloma and melanoma. Blood 122, 863-871.
Lorenz, F.K.M., Ellinger, C., Kieback, E., Wilde, S., Lietz, M., Schendel, D.J., and Uckert, W. (2017). Unbiased Identification of T-Cell Receptors Targeting Immunodominant Peptide-MHC Complexes for T-Cell Receptor Immunotherapy. Human gene therapy 28, 1158-1168.
Morgan, R.A., Chinnasamy, N., Abate-Daga, D., Gros, A., Robbins, P.F., Zheng, Z., Dudley, M.E., Feldman, S.A., Yang, J.C., Sherry, R.M., et al. (2013). Cancer regression and neurological toxicity following anti-MAGE-A3 TCR gene therapy. Journal of immunotherapy 36, 133-151.
Pascolo, S., Schirle, M., Guckel, B., Dumrese, T., Stumm, S., Kayser, S., Moris, A., Wallwiener, D., Rammensee, H.G., and Stevanovic, S. (2001). A MAGE-A1 HLA-A A*0201 epitope identified by mass spectrometry. Cancer research 61, 4072-4077.
Robbins, P.F., Li, Y.F., El-Gamil, M., Zhao, Y., Wargo, J.A., Zheng, Z., Xu, H., Morgan, R.A., Feldman, S.A., Johnson, L.A., et al. (2008). Single and dual amino acid substitutions in TCR CDRs can enhance antigen-specific T cell functions. Journal of immunology 180, 6116-6131. Sommermeyer, D., and Uckert, W. (2010). Minimal amino acid exchange in human TCR constant regions fosters improved function of TCR gene-modified T cells. Journal of immunology 184, 6223-6231.
Thistlethwaite F., Busse, A., Calvo, E., et al., (2021). A First-in-Human, Phase 1/2 Clinical Trial of TK-8001, a MAGE-A1 Directed T cell Receptor in Patients with Advanced-stage Solid Tumors (The "IMAG1NE!-Trial. J Immunother Cancer 9, (Suppl2), A499.
Tran, E., Turcotte, S., Gros, A., Robbins, P.F., Lu, Y.C., Dudley, M.E., Wunderlich, J.R., Somerville, R.P., Hogan, K., Hinrichs, C.S., et al. (2014). Cancer immunotherapy based on mutation-specific CD4+ T cells in a patient with epithelial cancer. Science 344, 641-645. Tsimberidou, A. Von Morris, K., Vo, H.H., et al. (2021). T-cell receptor-based therapy: an innovative therapeutic approach for solid tumors. J Hematol Oncol. 14:102-124.

## Claims

1. A nucleic acid encoding a TCR alpha chain construct (TRA) and/or a TCR beta chain construct (TRB) of a TCR construct specific for an epitope in complex with a human MHC-I, wherein the epitope is an epitope of a MAGEA1 protein,
a) wherein the epitope has the sequence of SEQ ID NO: 7, the MHC-I is HLA-A*03:01, and the TRA comprises a CDR3 having at least 90% sequence identity to SEQ ID NO: 123 and/or the TRB comprises a CDR3 having at least 90% sequence identity to SEQ ID NO: 128;
b) wherein the epitope has the sequence of SEQ ID NO: 7, the MHC-I is HLA-A*03:01, and the TRA comprises a CDR3 having at least 90% sequence identity to SEQ ID NO: 113 and/or the TRB comprises a CDR3 having at least 90% sequence identity to SEQ ID NO: 118;
c) wherein the epitope has the sequence of SEQ ID NO: 1, the MHC-I is HLA-B*44:03, and the TRA comprises a CDR3 having at least 90% sequence identity to SEQ ID NO: 13 and/or the TRB comprises a CDR3 having at least 90% sequence identity to SEQ ID NO: 18;
d) wherein the epitope has the sequence of SEQ ID NO: 2, the MHC-I is HLA-B*44:03, and the TRA comprises a CDR3 having at least 90% sequence identity to SEQ ID NO: 23 and/or the TRB comprises a CDR3 having at least 90% sequence identity to SEQ ID NO: 28;
e) wherein the epitope has the sequence of SEQ ID NO: 3, the MHC-I is HLA-B*44:03, and the TRA comprises a CDR3 having at least 90% sequence identity to SEQ ID NO: 33 and/or the TRB comprises a CDR3 having at least 90% sequence identity to SEQ ID NO: 38;
f) wherein the epitope has the sequence of SEQ ID NO: 1, the MHC-I is HLA-B*44:03, and the TRA comprises a CDR3 having at least 90% sequence identity to SEQ ID NO: 43 an/or d the TRB comprises a CDR3 having at least 90% sequence identity to SEQ ID NO: 48;
g) wherein the epitope has the sequence of SEQ ID NO: 4, the MHC-I is HLA-B*07:02, and the TRA comprises a CDR3 having at least 90% sequence identity to SEQ ID NO: 53 and/or the TRB comprises a CDR3 having at least 90% sequence identity to SEQ ID NO: 58;
h) wherein the epitope has the sequence of SEQ ID NO: 4, the MHC-I is HLA-B*07:02, and the TRA comprises a CDR3 having at least 90% sequence identity to SEQ ID NO: 63 and/or the TRB comprises a CDR3 having at least 90% sequence identity to SEQ ID NO: 68;
i) wherein the epitope has the sequence of SEQ ID NO: 4, the MHC-I is HLA-B*07:02, and the TRA comprises a CDR3 having at least 90% sequence identity to SEQ ID NO: 73 and/or the TRB comprises a CDR3 having at least 90% sequence identity to SEQ ID NO: 78;
j) wherein the epitope has the sequence of SEQ ID NO: 4, the MHC-I is HLA-B*07:02, and the TRA comprises a CDR3 having at least 90% sequence identity to SEQ ID NO: 83 and/or the TRB comprises a CDR3 having at least 90% sequence identity to SEQ ID NO: 88;
k) wherein the epitope has the sequence of SEQ ID NO: 5, the MHC-I is HLA-B*57:03 or HLA-B*57:03, and the TRA comprises a CDR3 having at least 90% sequence identity to SEQ ID NO: 93 and/or the TRB comprises a CDR3 having at least 90% sequence identity to SEQ ID NO: 98;
l) wherein the epitope has the sequence of SEQ ID NO: 6, the MHC-I is HLA-A*02:01, and the TRA comprises a CDR3 having at least 90% sequence identity to SEQ ID NO: 103 and/or the TRB comprises a CDR3 having at least 90% sequence identity to SEQ ID NO: 108;
m) wherein the epitope has the sequence of SEQ ID NO: 8, the MHC-I is HLA-A*26:01, and the TRA comprises a CDR3 having at least 90% sequence identity to SEQ ID NO: 133 and/or the TRB comprises a CDR3 having at least 90% sequence identity to SEQ ID NO: 138; and/or
n) wherein the epitope has the sequence of SEQ ID NO: 9, the MHC-I is HLA-A*29:02, and the TRA comprises a CDR3 having at least 90% sequence identity to SEQ ID NO: 143 and/or the TRB comprises a CDR3 having at least 90% sequence identity to SEQ ID NO: 148; and/or
o) wherein the MHC-I is HLA-A*29:02, and the TRA comprises a CDR3 having at least 90% sequence identity to SEQ ID NO: 173 and/or the TRB comprises a CDR3 having at least 90% sequence identity to SEQ ID NO: 178; and/or
p) wherein the MHC-I is HLA-B*44:03, and the TRA comprises a CDR3 having at least 90% sequence identity to SEQ ID NO: 183 and/or the TRB comprises a CDR3 having at least 90% sequence identity to SEQ ID NO: 188; and/or
q) wherein, the epitope has the sequence of SEQ ID NO: 201, the MHC-I is HLA-C*06:02, and the TRA comprises a CDR3 having at least 90% sequence identity to SEQ ID NO: 193 and/or the TRB comprises a CDR3 having at least 90% sequence identity to SEQ ID NO: 198.

2. A nucleic acid of claim 1, wherein the MHC-I is HLA-A*03:01 and the epitope has the sequence of SEQ ID NO: 7.

3. A nucleic acid of claim 2, wherein the TRA comprises a CDR3 having at least 90% sequence identity to SEQ ID NO: 123 and/or the TRB comprises a CDR3 having at least 90% sequence identity to SEQ ID NO: 128;
wherein, optionally, the TRA comprises a CDR1 having at least 80% sequence identity to SEQ ID NO: 121, a CDR2 having at least 80% sequence identity to SEQ ID NO: 122 and a CDR3 having at least 90%, preferably, 100% sequence identity to SEQ ID NO: 123, and/or the TRB comprises a CDR1 having at least 80% sequence identity to SEQ ID NO: 126, a CDR2 having at least 80% sequence identity to SEQ ID NO: 127 and a CDR3 having at least 90%, preferably, 100% sequence identity to SEQ ID NO: 128.

4. A nucleic acid of claim 2, wherein the TRA comprises a CDR3 having at least 90% sequence identity to SEQ ID NO: 113 and/or the TRB comprises a CDR3 having at least 90% sequence identity to SEQ ID NO: 118;
wherein, optionally, the TRA comprises a CDR1 having at least 80% sequence identity to SEQ ID NO: 111, a CDR2 having at least 80% sequence identity to SEQ ID NO: 112 and a CDR3 having at least 90%, preferably, 100% sequence identity to SEQ ID NO: 113, and/or the TRB comprises a CDR1 having at least 80% sequence identity to SEQ ID NO: 116, a CDR2 having at least 80% sequence identity to SEQ ID NO: 117 and a CDR3 having at least 90%, preferably, 100% sequence identity to SEQ ID NO: 118.

5. A nucleic acid of claim 1, wherein the MHC-I is HLA-B*07:02 and the epitope has the sequence of SEQ ID NO: 4, and, optionally, the TRA comprises a CDR3 having at least 90% sequence identity to SEQ ID NO: 83 and/or the TRB comprises a CDR3 having at least 90% sequence identity to SEQ ID NO: 88;
wherein, preferably, the TRA comprises a CDR1 having at least 80% sequence identity to SEQ ID NO: 81, a CDR2 having at least 80% sequence identity to SEQ ID NO: 82 and a CDR3 having at least 90%, such as 100% sequence identity to SEQ ID NO: 83, and/or the TRB comprises a CDR1 having at least 80% sequence identity to SEQ ID NO: 86, a CDR2 having at least 80% sequence identity to SEQ ID NO: 87 and a CDR3 having at least 90%, such as 100% sequence identity to SEQ ID NO: 88.

6. .A nucleic acid of claim 1, wherein the epitope has the sequence of SEQ ID NO: 201, the MHC-I is HLA-C*06:02 and the TRA comprises a CDR3 having at least 90% sequence identity to SEQ ID NO: 193 and/or the TRB comprises a CDR3 having at least 90% sequence identity to SEQ ID NO: 198;
wherein, optionally, the TRA comprises a CDR1 having at least 80% sequence identity to SEQ ID NO: 191, a CDR2 having at least 80% sequence identity to SEQ ID NO: 192 and a CDR3 having at least 90%, preferably, 100% sequence identity to SEQ ID NO: 193, and/or the TRB comprises a CDR1 having at least 80% sequence identity to SEQ ID NO: 196, a CDR2 having at least 80% sequence identity to SEQ ID NO: 197 and a CDR3 having at least 90%, preferably, 100% sequence identity to SEQ ID NO: 198.

7. A nucleic acid of claim 1, wherein the MHC-I is HLA-B*44:03, wherein, optionally, the epitope has the sequence of SEQ ID NO: 1, and the TRA comprises a CDR3 having at least 90% sequence identity to SEQ ID NO: 43 and the TRB comprises a CDR3 having at least 90% sequence identity to SEQ ID NO: 48;
wherein, preferably, the TRA comprises a CDR1 having at least 80% sequence identity to SEQ ID NO: 41, a CDR2 having at least 80% sequence identity to SEQ ID NO: 42 and a CDR3 having at least 90%, such as 100% sequence identity to SEQ ID NO: 43, and/or the TRB comprises a CDR1 having at least 80% sequence identity to SEQ ID NO: 46, a CDR2 having at least 80% sequence identity to SEQ ID NO: 47 and a CDR3 having at least 90%, such as 100% sequence identity to SEQ ID NO: 48.

8. A nucleic acid of claim 1, wherein the epitope has the sequence of SEQ ID NO: 6, the MHC-I is HLA-A*02:01, and the TRA comprises a CDR3 having at least 90% sequence identity to SEQ ID NO: 103 and/or the TRB comprises a CDR3 having at least 90% sequence identity to SEQ ID NO: 108;
wherein, optionally, the TRA comprises a CDR1 having at least 80% sequence identity to SEQ ID NO: 101, a CDR2 having at least 80% sequence identity to SEQ ID NO: 102 and a CDR3 having at least 90%, preferably, 100% sequence identity to SEQ ID NO: 103, and/or the TRB comprises a CDR1 having at least 80% sequence identity to SEQ ID NO: 106, a CDR2 having at least 80% sequence identity to SEQ ID NO: 107 and a CDR3 having at least 90%, preferably, 100% sequence identity to SEQ ID NO: 108.

9. A nucleic acid of claim 1, wherein the epitope has the sequence of SEQ ID NO: 5, the MHC-I is HLA-B*57:01 or HLA-B*57:03, and the TRA comprises a CDR3 having at least 90% sequence identity to SEQ ID NO: 93 and/or the TRB comprises a CDR3 having at least 90% sequence identity to SEQ ID NO: 98;
wherein, optionally, the TRA comprises a CDR1 having at least 80% sequence identity to SEQ ID NO: 91, a CDR2 having at least 80% sequence identity to SEQ ID NO: 92 and a CDR3 having at least 90%, preferably, 100% sequence identity to SEQ ID NO: 93, and/or the TRB comprises a CDR1 having at least 80% sequence identity to SEQ ID NO: 96, a CDR2 having at least 80% sequence identity to SEQ ID NO: 97 and a CDR3 having at least 90%, preferably, 100% sequence identity to SEQ ID NO: 98.

10. The nucleic acid of any of the preceding claims, wherein the sequence identity to the recited CDR1 and CDR2 and CDR3 regions is 100%.

11. The nucleic acid of any of the preceding claims, wherein the TCR alpha chain construct and/or the TCR beta chain construct further comprise a constant region selected from the group comprising a human constant region, a murine constant region or a chimeric constant region.

12. The nucleic acid of any of the preceding claims, encoding at least one TCR alpha and beta chain construct of the TCR construct,
wherein the nucleic acid is selected from the group comprising a viral vector, a transposon, a vector suitable for CRISPR/CAS based recombination or a plasmid suitable for *in vitro* RNA transcription.

13. A TCR construct encoded by the nucleic acid of any of the preceding claims.

14. A host cell comprising a nucleic acid of any of claims 1-12 and/or a protein of claim 13, wherein the host cell preferably is a human T cell.

15. A pharmaceutical composition comprising
a) a nucleic acid of any of claims 1-12 encoding a TCR construct capable of specifically binding to said epitope in the context of said MHC-I; or
b) a protein of claim 13 comprising a TCR construct capable of specifically binding to said epitope in the context of said MHC-I; or
c) a host cell of claim 14 expressing a TCR construct capable of specifically binding to said epitope in the context of said MHC-I.

16. A pharmaceutical composition or a kit comprising at least two pharmaceutical compositions comprising
a) at least two nucleic acids, each encoding a TCR construct capable of specifically binding to its respective epitope in the context of the respective MHC-I; or
b) at least two proteins, each comprising a TCR construct capable of specifically binding to its respective epitope in the context of the respective MHC-I; or
c) at least two host cells, each expressing a TCR construct capable of specifically binding to its respective epitope in the context of the respective MHC-I,
wherein the epitopes are peptides from the same antigen expressed by the same cancer or infectious agent, which are capable of being presented by different MHC-I molecules, wherein, optionally, the antigen is MAGEA1,
wherein, preferably, the kit or the pharmaceutical composition is for use in treatment of said cancer or infectious agent.

17. A pharmaceutical composition of any of claims 15 or 16 or a kit comprising the pharmaceutical composition or the kit of any of claims 15 or 16, comprising
a) at least one, preferably, two nucleic acids of any of claims 1-12, each encoding a TCR construct capable of specifically binding to its respective epitope in the context of the respective MHC-I; or
b) at least one, preferably, two proteins of claim 13, each comprising a TCR construct capable of specifically binding to its respective epitope in the context of the respective MHC-I; or
c) at leastone, preferably, two host cells of claim 14, each expressing a TCR construct capable of specifically binding to its respective epitope in the context of the respective MHC-I.

18. The pharmaceutical composition of any of claims 15-17 or the kit comprising the pharmaceutical composition or the kit of any of claims 15-17 for use in the treatment of a subject expressing said MHC-I and having a cancer expressing MAGEA1 selected from the group comprising lung cancer, colorectal cancer, and multiple myeloma.
wherein the treatment preferably is an immunotherapy selected from the group comprising adoptive T cell therapy and TCR gene therapy.

19. A MAGEA1 peptide comprising at most 11 consecutive amino acids identical to the sequence of amino acids ocurring in MAGEA1 of SEQ ID NO: 10, comprising an epitope capable of being presented by a human MHC-I, wherein the epitope is selected from the group comprising epitopes
a) having at least 88% sequence identity to SEQ ID NO: 7, wherein the epitope is capable of being presented on HLA-A*03:01,
b) having at least 88% sequence identity to any of SEQ ID NO: 1, 2 or 3, wherein the epitope is capable of being presented on HLA-B*44:03,
c) having at least 88% sequence identity to SEQ ID NO: 5, wherein the epitope is capable of being presented on HLA-B*57:01 and HLA-B*57:03 and comprises at most 10 consecutive amino acids identical to the sequence of amino acids ocurring in MAGEA1 of SEQ ID NO: 10,
and
d) having at least 88% sequence identity to SEQ ID NO: 9, wherein the epitope is capable of being presented on HLA-A*29:02,
wherein, preferably, the epitope, when presented by the respective MHC-I, is capable of being specifically recognized by one of the TCR construct proteins of claim 13.

20. A pharmaceutical composition comprising a MAGEA1 peptide of claim 19, or a MAGEA1 peptide comprising at most 11 consecutive amino acids identical to the sequence of amino acids ocurring in MAGEA1 of SEQ ID NO: 10, comprising an epitope capable of being presented by a human MHC-I, wherein the epitope is selected from the group comprising epitopes
a) having at least 88% sequence identity to SEQ ID NO: 4, wherein the epitope is capable of being presented on HLA-B*07:02, and
b) having at least 88% sequence identity to SEQ ID NO: 8, wherein the epitope is capable of being presented on HLA-A*26:01,
wherein the pharmaceutical composition is for use in vaccinating a subject expressing said MHC-I on which the epitope is capable of being presented.
